# EUROPEAN PATENT APPLICATION

(11) **EP 4 843 568 A1**
(43) Date of publication of application: **25.11.2026**
(21) Application number: 25741581.0
(22) Date of filing: 16.01.2025
(51) Int. Cl.: A61K 9/44, A61K 9/28, A61K 31/198, A61K 31/137, A61K 47/38, A61K 47/32

(54) **OSMOTIC PUMP TABLET, METHOD FOR PREPARING SAME, AND USE THEREOF**

(30) Priority: 16.01.2024 CN 202410065638
(71) Applicant: Shanghai WD Pharmaceutical Co., Ltd, Shanghai 201203 (CN)
(72) Inventor: DONG, Liangchang, Shanghai 201203 (CN); FAN, Ming, Shanghai 201203 (CN); MA, Wenbo, Shanghai 201203 (CN); SHI, Jingmin, Shanghai 201203 (CN); ZHANG, Danyong, Shanghai 201203 (CN); WU, Gang, Shanghai 201203 (CN)
(74) Representative: IK-IP LTD
(86) International application number: PCT/CN2025/072755
(87) International publication number: WO 2025/153020

(57) **Abstract**

Provided are an osmotic pump tablet, a method for preparing same, and use thereof. The osmotic pump tablet comprises a tablet core and a coating film enclosing the tablet core. The coating film is provided with a drug release hole. The tablet core comprises a drug-containing layer. The drug-containing layer comprises an active pharmaceutical ingredient, a hydrophilic polymer, and a surfactant, the hydrophilic polymer comprises hydroxypropyl cellulose, and the surfactant comprises poloxamer. Alternatively, the drug-containing layer comprises an active pharmaceutical ingredient and a hydrophilic polymer, but does not comprise a surfactant, and the hydrophilic polymer comprises povidone K29/32. Alternatively, the osmotic pump tablet comprises a drug-containing quick-release outer coating, the drug-containing quick-release outer coating comprises an active pharmaceutical ingredient and a pharmaceutically acceptable excipient, and the pharmaceutically acceptable excipient is a binder.

## Description

The present application claims the priority of Chinese application CN2024100656387 filed on January 16, 2024. The contents of the above patent applications are incorporated herein by reference in its entirety.

### TECHNICAL FIELD

The present disclosure relates to an osmotic pump tablet, method for preparing same and use thereof.

### BACKGROUND

A great number of active pharmaceutical ingredient(s) (APIs), including levodopa (LD), carbidopa (CD), baclofen, acyclovir, valacyclovir, ganciclovir, metformin, gabapentin, etc., have an absorption window limited at the upper gastrointestinal tract. The incorporation of these APIs into conventional extended-release dosages will not only result in compromised bioavailability, but also lead to failure of achieving prolonged therapeutic coverage. Therefore, a number of technologies have been disclosed in the prior art to extend the retention time in the stomach. These technologies include: expansion (USP 4735804, 5002772 and 6685962), swelling (USP 4434153, 5750585, 5972389, 6120803, 6660300 B1, US 2007/0196396A1 and USP 9439851), floating (USP 4167558, 5232704 and 6261601), raft-forming (USP 4140760 and 5068109), sinking (USP 4193985 and 4900557) and muco-adhesion (USP 6207197 and US2005/030552), etc. The technologies described above have very limited success, especially when oral dosage forms using these technologies are administered at fasting state. Therefore, there is a need for a novel controlled-release system or drug controlled-release system that could provide prolonged exposure to these active pharmaceutical ingredient(s) with their absorption window limited at the upper gastrointestinal tract. The oral retention device and these drugs are combined to form a drug-device composition to form an oral retention drug delivery system, which can provide long-term exposure to these drugs with their absorption window limited to the upper gastrointestinal tract.

In addition, for local administration of oral mucosa, liquid or semi-solid preparations, such as commonly used sprays, gargles, ointments, stay in the oral cavity for a short time and cannot achieve the effect of continuous administration. Therefore, there is a need for a drug delivery system that could provide long-term exposure of pharmaceutical ingredients, and is suitable for drugs with absorption windows in the upper gastrointestinal tract or requiring local administration in the oral cavity. The drug delivery device is loaded with drugs and fixed in the oral cavity after being combined with a fixator to form a drug controlled-release system that provides long-term exposure of drugs with their absorption window limited to the upper gastrointestinal tract.

Regarding oral drug delivery devices, US10668274 and CN1997421A disclose an oral drug containing container based on an electrically controlled drug release mechanism. The drug-containing container and the electric control system they claimed are difficult to implement. CN105873631A discloses an oral drug delivery device that requires an electronic or mechanical pump as external power for drug delivery. In addition, CN1925823A discloses a dental bracket for attaching fluoride pills to teeth to improve the treatment or prevention of dental caries, and this patent application does not involve drug absorption in the upper gastrointestinal tract. Furthermore, CN109908461A discloses an oral drug delivery device applied to a false tooth, tooth braces or an oral implant, which is suitable for administration of oral mucosa and does not involve drug absorption in the upper gastrointestinal tract. The above disclosed patent applications may require external power, or rely on a false tooth, tooth braces, etc., for realization, or do not involve drug absorption at the upper gastrointestinal tract.

CN114191307A and CN212973560U disclose an oral retention device for retaining the medicinal tablets in the oral cavity, providing long-term exposure of drugs with absorption windows limited to the upper gastrointestinal tract to obtain long-term stable blood drug concentrations. The oral retention device has taken into account that the medicinal tablets are inserted from back to front, close to the throat and are blocked by the oral tissues such as the buccal fat pad tip, so that the medicinal tablets are not easy to fall off when wearing the device. At the same time, the tooth-matching component in the oral retention device can fit with the patient's teeth according to personalized design, making it stable to wear. However, for patients with too short clinical crowns, small buccal space, or special oral conditions, when the mouth movement is large, it may cause poor retention of the oral retention device, and there is a risk of lifting or falling off.

The above oral drug delivery devices do not take into account the firmness of wearing under special oral conditions, and that complex structures or large volume of device may result in poor wearing comfort and safety for patients, and may also have a significant impact on facial appearance when wearing.

Osmotic pump tablets are an ideal oral controlled-release preparation for active pharmaceutical ingredient(s) whose absorption window is limited to the upper gastrointestinal tract. Early osmotic pump tablets were single-layer tablet core osmotic pump tablets, generally composed of a water-soluble drug tablet core, a coating membrane coating, and a drug-release orifice. When in an aqueous environment, water enters the tablet core through the semipermeable controlled-release membrane, hydrating the tablet core to generate a drug solution, resulting in a difference in internal and external osmotic pressure. Driven by the osmotic pressure difference, water continuously enters the tablet core through the semipermeable coating membrane, driving the drug solution to be released from the drug-release orifice. Single layer tablet core osmotic pump tablets can be used for controlled release preparations of large-dose water-soluble active pharmaceutical ingredient(s). However, for poorly water-soluble drugs, the osmotic pressure generated by the drug itself is low. Although adding an osmotic agent can provide osmotic pressure, the solid drug powders tend to settle downwards, making it difficult to obtain ideal large-dose poorly water-soluble drug osmotic pump tablets through single-layer tablet core osmotic pump tablets. Drug release is not constant and there are many residues in the end.

Compared with single-layer tablet core osmotic pump tablets, bi-layer tablet core osmotic pump tablets consist of a drug-containing layer and a push layer, which are capable of providing zero order constant rate drug release with complete drug release (drug release greater than 90%) for both water-soluble and water insoluble drugs. Bi-layer tablet core osmotic pump tablets are currently the most mature and suitable dosage form for industrial production of osmotic pump tablets made from drugs, especially poorly water-soluble drugs. However, the drug release is limited by the drug loading rate and the permeability of the controlled-release membrane, and the drug loading and drug release are often not high. Conventional bi-layer tablet core osmotic pump tablets cannot maintain the interface between the drug-containing layer and the push layer due to their own structural limitations when pushing drugs, especially large-dose drugs, resulting in the push layer passing through the drug-release orifice before the drug-containing layer in the middle and late stages of drug release, changing the release rate and causing a large amount of drug residue.

There is also a high-dose osmotic pump tablet, it is a great challenge to the physical and chemical properties of the controlled-release membrane to achieve high-flux drug release. On the one hand, the membrane needs to have high mechanical strength to resist the resistance caused by the high drug loading of mud passing through the drug-release orifice, thereby avoiding membrane rupture during the drug release process. On the other hand, the membrane needs to have good medium permeability, so that water can quickly pass through the controlled-release membrane to enter the tablet core, and the drug can be quickly released after the drug-containing layer is hydrated. Currently, there are no high-dose osmotic pump tablets suitable for active pharmaceutical ingredient(s) whose absorption window is limited to the upper gastrointestinal tract, and this problem needs to be solved urgently.

### DETAILED DESCRIPTION OF THE INVENTION

In order to overcome the defect that the current extended release drugs cannot provide a prolonged and a steady plasma profiles of active pharmaceutical ingredient(s); and to avoid the potential precipitation of cellulose acetate during the coating process of the controlled-rate membrane so as to solve the defects of uneven membrane coating and drug release, a controlled-release system of active pharmaceutical ingredient(s) and a preparation method thereof are provided.

Therefore, one aspect of the present disclosure relates to an extended release dosage form with an absorption window at the upper gastrointestinal tract, wherein the extended release dosage form comprises an extended release platform and a retention enabling platform;

The extended release platform (ERP) is a pharmaceutical composition comprising a tablet core and a coating membrane, wherein the tablet core comprises a drug-containing layer, and the coating membrane comprises cellulose acetate and copovidone, wherein the weight of the cellulose acetate is 50-70% of the weight of the coating membrane, and the weight of the copovidone is 30-50% of the weight of the coating membrane;

The function of the retention enabling platform is to maintain the extended release platform in the oral cavity. At least one end of the retention enabling platform is connected with a cover, so that the extended release platform can maintain in the retention enabling platform.

In order to solve the technical problem of the defect of low bioavailability due to the short residence time of the active pharmaceutical ingredient(s) in the stomach, another aspect of the present disclosure provides a new oral retention device that retains a medicinal tablet in the oral cavity without easily dropping out. Specifically, the present disclosure provides an oral retention device, with a medicinal tablet being inserted in a way from back to front such that, because the back side of the device is close to the throat, and due to blocking by the oral tissues, such as the buccal fat pad tip and the pterygomandibular folds, the medicinal tablet will not easily drop out from the device in the oral cavity, which will not cause suffocation due to accidental swallowing.

The oral retention device is similar to the retention enabling platform described above, but does not contain a cover, which comprises a tooth-matching component and a drug-loaded component, the tooth-matching component being connected to the drug-loaded component, wherein the tooth-matching component is used to bridge a tooth in the oral cavity and matches with the tooth, and the drug-loaded component can hold at least one medicinal tablet and is used to retain the medicinal tablet in the oral cavity.

In some embodiments, the medicinal tablet in the oral retention device may be the retention enabling platform or pharmaceutical composition or osmotic pump tablet of the present disclosure.

In some embodiments, the copovidone is prepared by the following method comprising: polymerizing vinylpyrrolidone and vinyl acetate, and the weight of the vinylpyrrolidone and the vinyl acetate is in the ratio of 40: 60-80: 20. Preferably, the weight of the vinylpyrrolidone and the vinyl acetate is in the ratio of 50: 50-70: 30. More preferably, the weight ratio of the vinylpyrrolidone and the vinyl acetate is in the ratio of 60: 40.

In some embodiments, the retention enabling platform comprises a personalized retention enabling module and a drug fastened module, the drug fastened module can fasten the extended release platform, and the personalized retention enabling module can maintain the drug fastened module in the oral cavity. More preferably, the drug fastened module is one or more reservoirs. In one embodiment, the reservoir is in a basket structure. In another embodiment, the shape of the cross section of the reservoir is polygon, circular closed ring or circular open ring, or a combination thereof.

In some embodiments, at least one end of the reservoir is connected with a cover, so that the extended release platform can be maintained in the reservoir; more preferably, the cover is a strip.

In some embodiments, the retention enabling module can be fitted to any one or more teeth in the oral cavity. Preferably the mandibular permanent teeth. More preferably the mandibular molar. Most preferably the mandibular second molar and its anterior and posterior molar.

The retention enabling module can be customized to fit and wrap, clamp or insert the entire maxillary teeth or the entire mandibular permanent teeth; preferably wrap, clamp or insert the mandibular permanent tooth; more preferably wrap the mandibular molar; most preferably wrap, clamp or insert the second mandibular molar and its adjacent parts of the first molar and the second bicuspid.

When the retention enabling platform is an oral retention device, the personalized retention enabling module can be a tooth-matching component, and the drug fastened module is equivalent to drug-loaded component.

In some embodiments, the tooth-matching component and the drug-loaded component are connected on respective sides.

In some embodiments, the tooth-matching component can match with any one or more teeth in the oral cavity.

In some embodiments, the tooth-matching component has a length equal to the length of 2-5 teeth.

In some embodiments, the teeth are mandibular permanent teeth. Preferably, they are mandibular molars. More preferably, they are any combination of the following: (i) the first molar and the second molar; (ii) the first molar, the second molar and the second premolar; (iii) the first molar, the second molar and the third molar; or (iv) the first molar, the second molar, the third molar and the second premolar.

In some embodiments, the tooth-matching component is wrapped, embedded, fitted, or inserted into the teeth that matches with the tooth-matching component.

In some embodiments, the drug-loaded component has a reticular structure or a nonreticular structure.

In some embodiments, the drug-loaded component has cross section in the shape of a circular, elliptical, polygonal, or special-shaped closed ring or open ring structure.

In some embodiments, the drug-loaded component comprises at least one ring body and at least one retainer, or the drug-loaded component is constituted by at least one retainer; wherein the ring body has an opening for insertion of a medicinal tablet, and the retainer has a structure for limiting the medicinal tablet in the drug-loaded component.

In some embodiments, the ring body is an open ring body or a closed ring body.

In some embodiments, the ring body is a circular, elliptical, polygonal, or special-shaped closed ring body or open ring body.

In some embodiments, the ring body is provided as one ring body that is located on the molar side in the horizontal direction formed by the molars and the incisors.

In some embodiments, the retainer is of a circular arc hollow or solid shape.

In some embodiments, the retainer is formed by connecting a closed ring body and a semicircular part perpendicular to the closed ring body.

In some embodiments, the retainer abuts with the ring body, or the retainer and the ring body are arranged at an interval. Preferably, the retainer and the ring body are abutted by means of integral molding or are connected together by a connecting structure.

In some embodiments, the retainer is provided as one retainer.

In some embodiments, the retainer is located on the incisor side in the horizontal direction formed by the molars and the incisors.

In some embodiments, the opening faces the molars in a horizontal direction formed by the molars and the incisors, and is used to enable the medicinal tablet to be inserted from the molars toward the incisors in the horizontal direction; or the opening is provided in a direction perpendicular to the horizontal direction such that the medicinal tablet is inserted down from the above in the direction perpendicular to the horizontal direction; or the opening faces the buccal side in a direction perpendicular to the horizontal direction such that the medicinal tablet is inserted from the buccal side to the lingual side in the direction perpendicular to the horizontal direction.

The extended release platform of the present disclosure is an osmotic pump delivery system including LD and CD. The osmotic pump delivery system can be a single-layer elementary osmotic pump or a bi-layer push-pull system. Osmotic pump delivery system could provide constant release of LD/CD in the oral cavity, which is in sharp contrast with matrix extended release system. The matrix extended release system is susceptible to the conditions in oral cavity such as pH, saliva availability and voluntary or involuntary rubbing of the hydrated matrix tablets by tongue.

The retention enabling platform (REP) of the present disclosure is a personalized ERP retainer that enables to fasten the ERP in oral cavity. Therefore, LD/CD can be released near the throat and readily swallowed to the stomach. The REP is a kind of REP with security features, which can prevent the accidental blockage of the extended release system. In the present disclosure, the "personalized" refers to the preparation of the retention enabling platform or retention enabling module that enables to fasten ERP in the oral cavity and fit to the shape of one or more teeth or the whole maxillary teeth or the whole mandibular teeth of the patient according to the shape of the teeth of the patient.

Another aspect of the present disclosure relates to the preparation method of the extended release dosage form, and the preparation method of the retention enabling platform or the oral retention device comprises 3D printing, injection molding or impression molding. By combining oral scanning, CAD/CAM design and preparation method, REP can be accurately and rapidly prepared according to the image of individual oral scanning.

Herein, the working principle of 3D printing is basically the same as that of ordinary printer, except that the printing materials are different. The printing materials of ordinary printers are ink and paper, while 3D printers are equipped with different "printing materials" such as metal, ceramics, plastic, sand, etc. After the printer is connected with the computer, the "printing materials" can be stacked layer upon layer through computer control, thereby making the blueprint on the computer into a real object in the end. 3D printing refers to the technical principles of ordinary printers, in which the process of layering is very similar to inkjet printing. This printing technology is called 3D stereo printing technology. There are many different technologies for 3D printing, which differ in that modules are created by using available materials and different layers. Common materials for 3D printing are nylon glass fiber, polylactic acid, ABS resin, durable nylon material, gypsum material, aluminum material, metal titanium, titanium alloy, stainless steel, silver plating, gold plating, cobalt chromium alloy, cobalt chromium molybdenum alloy or rubber material. 3D printing is advantageous in that the automated operation can be implemented, the production speed is fast, the design blueprint in the computer can be directly and accurately converted into a physical model, and it is also suitable for small-scale custom manufacturing.

Herein, 3D printing comprises the following steps:
(1) in preferable design software 3Shape Dental System, adding a saved drug-loaded component plan, assembling the plan with a tooth-matching component plan to form an integrated oral retention device plan, and exporting a 3D printable file;
(2) importing the 3D printable file into a 3D printer, and printing the oral retention device; wherein the 3D printing preferably uses a laser sintering process;
   preferably, before the step (1), the following steps are included: (0) in preferable software SolidWorks, designing the drug-loaded component plan according to the data of the size of the medicinal tablet, and saving the plan; and/or in the design software 3Shape Dental System, designing the tooth-matching component plan according to the data of the size of teeth of the subject;
   more preferably, the data of the size of the medicinal tablet and/or the data of the tooth of the subject are obtained by means of scanning with a scanner, and the scanner is preferably 3Shape TRIOS^{®} scanner or Medit i500 scanner.

In some embodiments, a "3Shape TRIOS^{®}" intraoral scanner was used to scan the data of size of a medicinal tablet, the data was then imported into the software "SolidWorks", a plan of a drug-loaded component that can load the drug part, and the plan was created and saved as a "standard attachment" file; the "3Shape TRIOS^{®}" intraoral scanner was used to scan the teeth of the subject, the data was imported into the software "3Shape Dental System", and a tooth-matching component was designed according to the data of teeth; on the software "3Shape Dental System", the "standard attachment" was added and assembled with the tooth-matching component to form an integrated oral retention device, and a 3D printable file was exported; and the 3D printable file was imported into the 3D printer, and the oral retention device was printed. The 3D printing preferably uses a laser sintering process.

Injection molding is a method of injecting and molding, that is, at a certain temperature, the polymer material that is completely molten is stirred by a screw, injected into a mold cavity with high pressure, and cooled and solidified to get the molding products. This method is suitable for mass production of complex shape parts and is one of the important processing methods. The injection molding method has the advantages of fast production speed, high efficiency, automation of operation, a large variety of designs, a large variety of shapes including simple and complex shapes, and a large variety of sizes including large and small sizes. Moreover, dimension of product is precise, product is easy to be updated and module can be made into complex shapes. Injection molding is suitable for mass production and is applicable to the molding processing field of products with complex shape and the like.

Herein, the injection molding comprises the following steps:
(1) preparing a tooth-matching component model according to a teeth model;
(2) preparing a drug-loaded component model according to the size of the medicinal tablet;
(3) obtaining an oral retention device model integrating the tooth-matching component and the drug-loaded component;
(4) preparing a personalized oral retention device by means of a traditional injection molding process;

In some embodiments, the teeth model is prepared by means of the traditional model-taking technology, or prepared by obtaining teeth data of a subject by means of the oral scanning technology and printing.

In some embodiments, the materials of the tooth-matching component model, the drug-loaded component model and the oral retention device model are dental wax, and/or the material of the teeth model is gypsum or resin.

For impression molding, firstly, a drug fastened module suitable for the extended release platform is prepared. Secondly, elliptical thermoplastic sheet is processed by conventional technology. Finally, a personalized REP was prepared. A thermoplastic sheet is heated in hot water at about 70 °C to soften it so as to have good plasticity. When the thermoplastic sheet becomes translucent (about 1 minute), take it out and put it on the teeth, and press the softened thermoplastic sheet to completely wrap the teeth to form a retention enabling module that completely fitted to the teeth. The drug fastened module is then quickly embedded into the uncured retention enabling module and cooled to solidify into a personalized retention enabling platform (REP). Some water can be sprayed briefly to further accelerate the cooling, wait for the personalized retention enabling platform to cool and restore the original opaque plate state, and take out the cooled personalized retention enabling platform from the oral cavity.

In some embodiments, the method for preparing the oral retention device of the present disclosure is impression molding, comprising:
designing a drug-loaded component according to the size of the medicinal tablet; preparing a tooth-matching component from polycaprolactone (PCL) as the material, and preparing the drug-loaded component from cobalt-chromium alloy as the material; and assembling the tooth-matching component and the drug-loaded component into a complete oral retention device.

In some embodiments, at first, a drug-loaded component capable of loading an osmotic pump tablet is prepared by means of a traditional injection molding process; and then a thermoplastic sheet is heated and softened to prepare a tooth-matching component, and at the same time the softened tooth-matching component is fitted with the drug-loaded component in an embedded manner and is then cooled to form an integrated oral retention device, which is taken out of the oral cavity.

In some embodiments, the retention enabling platform or oral retention device is prepared from one or more oral stable materials including oral stable metals and thermoplastic elastomers.

In some embodiments, the oral stable metal comprises dental titanium, stainless steel, cobalt chromium alloy, cobalt chromium molybdenum alloy, nickel chromium alloy or precious metal, and the thermoplastic elastomer comprises polycaprolactone (PCL), ethylene vinyl acetate copolymer (EVA), high density polyethylene (HDPE), polypropylene (PP), polyacrylate, polyurethane, silicone polymer, polyester, poly (styrene-ethylene-butene-styrene)("SEBS"), poly (styrene-butadiene-styrene) ("SBS"), poly (styrene-isoprene-styrene) ("SIS"), or a copolymer of any two or more of the above , or a physical combination thereof.

In some embodiments, both the tooth-matching component and the drug-loaded component are made of cobalt-chromium alloy.

The pharmaceutical composition or the upper gastrointestinal tract (UGI) extended release drug delivery system of the present disclosure comprises the forms such as a single-layer elementary osmotic pump, a bi-layer push-pull osmotic pump, and a bi-layer push-pull osmotic pump comprising an immediate-release drug overcoat, but differs from the osmotic pump in the prior art.

In the extended release dosage form of the present disclosure as described above, preferably, in the extended release platform, the drug-containing layer comprises active pharmaceutical ingredient(s) and excipients.

In some embodiments, the active pharmaceutical ingredient(s) is one or more selected from levodopa or its ester or a salt thereof, carbidopa, baclofen, acyclovir, valacyclovir, ganciclovir, metformin, and gabapentin; or one or two of levodopa or its ester and carbidopa, wherein the ester of levodopa can be levodopa alkyl ester or deuterated levodopa alkyl ester, such as levodopa methyl ester hydrochloride. The salt of levodopa is, for example, levodopa ethyl ester hydrochloride. The active pharmaceutical ingredient(s) is, for example, selected from an antifungal drug and an antitumor drug. The antifungal drug is, for example, one or more selected from nystatin, fluconazole, posaconazole, isavuconazole, voriconazole, anidulafungin, caspofungin and micafungin. The antitumor drug is, for example, one or more selected from 5-fluorouracil, paclitaxel, and capecitabine.

In some embodiments, the active pharmaceutical ingredient(s) comprise levodopa and/or carbidopa.

In some embodiments, the excipient is one or more selected from filler, osmotic agent, hydrophilic polymer, binding agent, lubricant, preservative, flavoring agent, acidifying agent and antioxidant. More preferably, the excipient is one or more selected from filler, osmotic agent, hydrophilic polymer, binding agent, lubricant and preservative. Even more preferably, the excipients are filler, osmotic agent, hydrophilic polymer, binding agent, lubricant and preservative.

In one embodiment, when the active pharmaceutical ingredient(s) comprises levodopa, the content of the levodopa is 20-70 wt% based on the total weight of the drug-containing layer. In another embodiment, when the active ingredient comprises carbidopa, the content of carbidopa is 0-20 wt% but not 0 wt% based on the total weight of the drug-containing layer.

In one embodiment, the active pharmaceutical ingredient(s) comprises levodopa, and the content of the levodopa is 30-50 wt% based on the total weight of the drug-containing layer. In another embodiment, when the active pharmaceutical ingredient(s) comprises carbidopa, the content of the carbidopa is 1-10 wt% based on the total weight of the drug-containing layer.

Preferably, in the drug-containing layer described above, when the excipient comprises a filler, the filler is one or more selected from microcrystalline cellulose, hydroxypropyl cellulose and mannitol. The content of the filler is 0-50 wt% but not 0 wt% based on the total weight of the drug-containing layer.

In another embodiment, when the excipient comprises an osmotic agent, the osmotic agent is one or more selected from magnesium sulfate, magnesium chloride, sodium chloride, lithium chloride, potassium sulfate, sodium sulfate, mannitol, urea, sorbitol, inositol, sucrose and glucose. The content of the osmotic agent is 0-50 wt% but not 0 wt% based on the total weight of the drug-containing layer.

In another embodiment, when the excipient comprises a hydrophilic polymer, the hydrophilic polymer is one or more selected from hydroxypropyl cellulose, hydroxypropyl methyl cellulose, carboxymethyl cellulose, polyvinylpyrrolidone (such as povidone K30 or povidone K29/32, preferably povidone K29/32) and hydroxyethyl cellulose. The content of the hydrophilic polymer is 0-50 wt% but not 0 wt% based on the total weight of the drug-containing layer.

In another embodiment, when the excipient comprises an acidifying agent, the acidifying agent is one or more selected from citric acid, sodium citrate, potassium citrate, malic acid, fumaric acid, lactic acid, phosphoric acid and tartaric acid. The content of the acidifying agent is 0-10% but not 0% based on the total weight of the drug-containing layer.

Preferably, the pharmaceutical composition further comprises an osmotic push layer. The osmotic push layer preferably comprises a hydrophilic polymer, an osmotic agent and a binding agent and optionally a lubricant and/or colorant. The osmotic push layer and the drug-containing layer are components of a bi-layer tablet core, and the coating membrane is wrapped outside the tablet core. The pharmaceutical composition can be a kind of osmotic pump extended release drug delivery system, that is, a bi-layer push-pull osmotic pump.

As a pharmaceutical composition of the bi-layer push-pull osmotic pump, preferably, the hydrophilic polymer of the osmotic push layer is κ-carrageenan, sodium carboxymethyl cellulose or polyethylene oxide. The molecular weight of the hydrophilic polymer is 75,000-7,500,000. The content of the hydrophilic polymer is 25-85 wt% based on the total weight of the osmotic push layer.

In one embodiment, the osmotic agent in the osmotic pump push layer is one or more selected from magnesium sulfate, magnesium chloride, sodium chloride, lithium chloride, potassium sulfate, sodium sulfate, mannitol, urea, sorbitol, inositol, sucrose and glucose. The content of the osmotic agent is 5-65 wt% based on the total weight of the osmotic push layer.

In another embodiment, the osmotic pump push layer comprises a binding agent, the binding agent is one or more selected from methyl cellulose, hydroxypropyl methyl cellulose, hydroxypropyl cellulose, sodium carboxymethyl cellulose, povidone and gelatin. The content of the binding agent is 3-20 wt% based on the total weight of the osmotic push layer.

In another embodiment, the osmotic pump push layer comprises a lubricant, the lubricant is one or more selected from magnesium stearate, magnesium stearate fumarate, talc, and colloidal silica. The content of the lubricant is 0-2 wt% but not 0 wt% based on the total weight of the osmotic push layer.

In another embodiment, the osmotic push layer comprises a colorant, the colorant is one or more selected from iron oxide red, iron oxide yellow, and iron oxide black. The content of the colorant is 0-5 wt% but not 0 wt% based on the total weight of the osmotic push layer.

Preferably, the osmotic push layer comprises sodium carboxymethyl cellulose, sorbitol, povidone, iron oxide red and magnesium stearate; or comprises sodium carboxymethyl cellulose, hydroxypropyl cellulose, sorbitol, iron oxide red, and magnesium stearate; preferably, is composed of sodium carboxymethyl cellulose, povidone K30, sorbitol, iron oxide red and magnesium stearate; or is composed of sodium carboxymethyl cellulose, hydroxypropyl cellulose, sorbitol, iron oxide red and magnesium stearate. More preferably, the sodium carboxymethyl cellulose is sodium carboxymethyl cellulose 7H4XF or 9H4XF.

In one embodiment, the osmotic push layer comprises 25-85 wt% of sodium carboxymethyl cellulose, 5-65 wt% of sorbitol, 3-20 wt% of povidone, 0-5 wt% of iron oxide red and 0.5-2 wt% of magnesium stearate; or 25-85wt% of sodium carboxymethyl cellulose, 5-65 wt% of sorbitol, 3-20 wt% of hydroxypropyl cellulose, 0-5 wt% of iron oxide red, and 0.5-2 wt% of magnesium stearate based on the total weight of the osmotic push layer.

In another embodiment, the osmotic push layer comprises 55 wt% of sodium carboxymethyl cellulose, 39.0 wt% of sorbitol, 5.0 wt% of povidone and 0.5 wt% of iron oxide red and 0.5 wt% of magnesium stearate based on the total weight of the osmotic push layer.

In another embodiment, the osmotic push layer comprises 55 wt% of sodium carboxymethyl cellulose, 34.0 wt% of sorbitol, 10.0 wt% of povidone K30, 0.5 wt% of iron oxide red and 0.5 wt% of magnesium stearate based on the total weight of the osmotic push layer.

In another embodiment, the osmotic push layer comprises 55 wt% of sodium carboxymethyl cellulose, 34.0 wt% of sorbitol, 10.0 wt% of hydroxypropyl cellulose, 0.5 wt% of iron oxide red and 0.5 wt% of magnesium stearate based on the total weight of the osmotic push layer.

In another embodiment, the osmotic push layer comprises 49.0 wt% of sodium carboxymethyl cellulose, 30.0 wt% of sorbitol, 20.0 wt% of hydroxypropyl cellulose, 0.5 wt% of iron oxide red and 0.5 wt% of magnesium stearate based on the total weight of the osmotic push layer.

In some preferred embodiments, the coating membrane of the pharmaceutical composition is further provided with a drug-containing immediate release overcoat. This constitutes a three-layer structure in which the inner layer is a tablet core, the middle layer is a coating membrane, and the outer layer is an overcoat.

More preferably, the drug-containing immediate release overcoat comprises active pharmaceutical ingredient(s) and an excipient, the active pharmaceutical ingredient(s) comprises levodopa and/or carbidopa, and the excipient is one or more selected from hydroxypropyl cellulose, aspartame and the mint flavors.

In one embodiment, when the active pharmaceutical ingredient(s) is levodopa, the content of the levodopa is 0-75 wt% but not 0 wt%, preferably 23.78-75 wt% based on the total weight of the drug-containing immediate release overcoat. When the active pharmaceutical ingredient(s) is carbidopa, the content of carbidopa is 0-93 wt% but not 0 wt%, preferably 26.85-93 wt%. When the excipient of the overcoat comprises hydroxypropyl cellulose, the content of the hydroxypropyl cellulose is 2-20 wt%, preferably 10 wt%. When the excipient of the overcoat comprises aspartame, the content of the aspartame is 0-5 wt%, preferably 0.9-5 wt%. When the excipient of the overcoat comprises mint flavor, the content of the mint flavor is 0-5 wt%, preferably 0.1 wt%.

More preferably, the weight of the coating membrane is not less than 2.0% of the weight of the tablet core. The coating membrane has one or more orifices, and the diameter of orifice is preferably 0.5 mm-1.0 mm, more preferably 0.5 mm, 0.75 mm and 1.0 mm. Preferably, the weight of the coating membrane is 2.0-15.0% of the weight of the tablet core. More preferably, the weight of the coating membrane is 4.0-8.0% of the weight of the tablet core.

Preferably, in one embodiment, the pharmaceutical composition is composed of a drug-containing layer and a coating membrane. In another embodiment, the pharmaceutical composition is composed of a drug-containing layer, an osmotic push layer and a coating membrane. In another embodiment, the pharmaceutical composition is composed of a drug-containing layer, an osmotic push layer, a coating membrane, and an overcoat.

Preferably, the drug-containing layer is composed of levodopa, carbidopa, microcrystalline cellulose, mannitol, citric acid, sodium hydroxypropyl methyl cellulose and magnesium stearate. In another embodiment, the drug-containing layer is composed of levodopa, microcrystalline cellulose, hydroxypropyl methyl cellulose and magnesium stearate. In another embodiment, the drug-containing layer is composed of levodopa, carbidopa, mannitol, citric acid and magnesium stearate. In another embodiment, the drug-containing layer is composed of levodopa, carbidopa, hydroxypropyl cellulose, mannitol, citric acid and magnesium stearate. In another embodiment, the drug-containing layer is composed of levodopa, carbidopa, hydroxypropyl cellulose, mannitol, citric acid, and povidone K30. In another embodiment, the drug-containing layer is composed of levodopa, hydroxypropyl cellulose, mannitol, povidone K30, magnesium stearate, mint flavor and aspartame. In another embodiment, the drug-containing layer is composed of levodopa, mannitol, povidone K30 and magnesium stearate. In another embodiment, the drug-containing layer is composed of levodopa, carbidopa, hydroxypropyl cellulose, mannitol, aspartame and magnesium stearate. In another embodiment, the drug-containing layer is composed of levodopa, hydroxypropyl cellulose, mannitol, povidone K30, magnesium stearate, mint flavor and aspartame. In another embodiment, the drug-containing layer is composed of levodopa, hydroxypropyl cellulose, mannitol, povidone K30, magnesium stearate, and aspartame. In another embodiment, the drug-containing layer is composed of levodopa, hydroxypropyl cellulose, mannitol, magnesium stearate, mint flavor and aspartame. In another embodiment, the drug containing layer is composed of levodopa, hydroxypropyl cellulose, povidone, mannitol and magnesium stearate.

Preferably, the osmotic push layer is composed of sodium carboxymethyl cellulose, povidone, sorbitol, iron oxide red and magnesium stearate, or the osmotic push layer is composed of sodium carboxymethyl cellulose, hydroxypropyl cellulose, sorbitol, iron oxide red and magnesium stearate. Preferably, the sodium carboxymethyl cellulose is sodium carboxymethyl cellulose 7H4XF or 9H4XF. The povidone is preferably povidone K30. The hydroxypropyl cellulose is hydroxypropyl cellulose EXF.

In one embodiment, the overcoat is composed of levodopa, carbidopa, hydroxypropyl cellulose, aspartame, and mint flavor; or the overcoat is composed of levodopa, carbidopa, hydroxypropyl cellulose and aspartame. In another embodiment, the overcoat is composed of carbidopa, hydroxypropyl cellulose and aspartame. In another embodiment, the overcoat is composed of levodopa, hydroxypropyl cellulose, and mint flavor. It is well known to those skilled in the art that the above "include" can be replaced by "consist of".

Preferably, based on the total weight of the drug-containing layer, the drug-containing layer is composed of 40 wt% of levodopa, 10.8 wt% of carbidopa, 20 wt% of microcrystalline cellulose, 18.7 wt% of mannitol, 5 wt% of citric acid, 5 wt% of sodium hydroxypropyl methyl cellulose and 0.5 wt% of magnesium stearate; or
the drug-containing layer is composed of 38 wt% of levodopa, 50 wt% of microcrystalline cellulose, 10 wt% of hydroxypropyl methyl cellulose and 2 wt% of magnesium stearate; or
the drug-containing layer is composed of 19.5 wt% of levodopa, 20 wt% of carbidopa, 50 wt% of mannitol, 10 wt% of citric acid and 0.5 wt% of magnesium stearate; or
the drug-containing layer is composed of 40 wt% of levodopa, 10.8 wt% of carbidopa, 31 wt% of hydroxypropyl cellulose, 12.7 wt% of mannitol, 5 wt% of citric acid and 0.5 wt% of magnesium stearate; or
the drug-containing layer is composed of 40 wt% of levodopa, 10.8 wt% of carbidopa, 31 wt% of hydroxypropyl cellulose, 12.7 wt% of mannitol, 5 wt% of citric acid, and 0.5 wt% of povidone K30; or
the drug-containing layer is composed of 45 wt% of levodopa, 31 wt% of hydroxypropyl cellulose, 16 wt% of mannitol, 5 wt% of povidone K30, 1 wt% of magnesium stearate, 1 wt% of mint flavor and 1 wt% of aspartame; or
the drug-containing layer is composed of 70 wt% of levodopa, 9 wt% of mannitol, 20 wt% of povidone K30 and 1 wt% of magnesium stearate; or
the drug-containing layer is composed of 20 wt% of levodopa, 20 wt% of carbidopa, 50 wt% of hydroxypropyl cellulose, 4 wt% of mannitol, 5 wt% of aspartame and 1 wt% of magnesium stearate; or
the drug-containing layer is composed of 45 wt% of levodopa, 31 wt% of hydroxypropyl cellulose, 17 wt% of mannitol, 5 wt% of povidone K30, 1 wt% of magnesium stearate and 1wt% of aspartame; or
the drug-containing layer is composed of 62.5 wt% of levodopa, 31 wt% of hydroxypropyl cellulose, 4.5 wt% of mannitol, 1 wt% of magnesium stearate, 0.1 wt% of mint flavor and 0.9 wt% of aspartame; or
the drug-containing layer is composed of 46.9 wt% of levodopa, 31 wt% of hydroxypropyl cellulose, 20.1 wt% of mannitol, 1 wt% of magnesium stearate, 0.1 wt% of mint flavor and 0.9 wt% of aspartame; or
the drug-containing layer is composed of 45 wt% of levodopa, 31 wt% of hydroxypropyl cellulose, 17 wt% of mannitol, 5 wt% of povidone K30, 1 wt% of magnesium stearate and 1wt% of aspartame; or
the drug-containing layer is composed of 45 wt% of levodopa, 31 wt% of hydroxypropyl cellulose, 12 wt% of mannitol, 5 wt% of povidone K30, 1 wt% of magnesium stearate, 5 wt% of mint flavor and 1 wt% of aspartame; or
the drug-containing layer is composed of 45 wt% of levodopa, 31 wt% of hydroxypropyl cellulose, 22 wt% of mannitol, 1 wt% of magnesium stearate, 0.1 wt% of mint flavor and 0.9 wt% of aspartame.

More preferably, based on the total weight of the coating membrane, the coating membrane is composed of 50 wt% of cellulose acetate and 50 wt% of copovidone VA64; the coating membrane is composed of 70 wt% of cellulose acetate and 30 wt% of copovidone VA64; the coating membrane is composed of 60 wt% of cellulose acetate and 40 wt% of copovidone VA64.

More preferably, the weight of the coating membrane is 2.0%, 4.2%, 4.5%, 4.6%, 4.8%, 5.0%, 5.9%, 6.5%, 6.7%, 7.0%, 7.7%, 7.9%, 8.0%, 9.5%, 9.7% or 10% of the weight of the tablet core.

Preferably, based on the total weight of the osmotic push layer, the osmotic push layer is composed of 55 wt% of sodium carboxymethyl cellulose 7H4XF, 5 wt% of povidone K30, 39 wt% of sorbitol, 0.5 wt% of iron oxide red and 0.5 wt% of magnesium stearate; or
the osmotic push layer is composed of 55 wt% of sodium carboxymethyl cellulose 7H4XF, 10 wt% of povidone K30, 34 wt% sorbitol, 0.5 wt% of iron oxide red and 0.5 wt% of magnesium stearate; or
the osmotic push layer is composed of 85 wt% of sodium carboxymethyl cellulose, 3 wt% of povidone K30, 5 wt% of sorbitol, 5 wt% of iron oxide red and 2 wt% of magnesium stearate; or
the osmotic push layer is composed of 25 wt% of sodium carboxymethyl cellulose, 9.5 wt% of povidone K30, 65 wt% of sorbitol and 0.5 wt% of magnesium stearate; or
the osmotic push layer is composed of 60 wt% of sodium carboxymethyl cellulose 7H4XF, 10 wt% of povidone K30, 26 wt% of sorbitol, 2 wt% of iron oxide red and 2 wt% of magnesium stearate; or
the osmotic push layer is composed of 40 wt% of sodium carboxymethyl cellulose 7H4XF, 20 wt% of povidone K30, 36 wt% of sorbitol, 3.5 wt% of iron oxide red and 0.5 wt% of magnesium stearate; or
the osmotic push layer is composed of 55 wt% of sodium carboxymethyl cellulose 9H4XF, 5 wt% of povidone K30, 39 wt% of sorbitol, 0.5 wt% of iron oxide red and 0.5 wt% of magnesium stearate; or
the osmotic push layer is composed of 55 wt% of sodium carboxymethyl cellulose 7H4XF, 10 wt% of hydroxypropyl cellulose, 34 wt% of sorbitol, 0.5 wt% of iron oxide red and 0.5 wt% of magnesium stearate.

Preferably, based on the total weight of the overcoat, the overcoat is composed of 23.78 wt% of levodopa, 64.22 wt% of carbidopa, 10 wt% of hydroxypropyl cellulose, 1 wt% of aspartame and 1 wt% of mint flavor; or
the overcoat is composed of 93 wt% of carbidopa, 2 wt% of hydroxypropyl cellulose and 5 wt% of aspartame; or
the overcoat is composed of 75 wt% of levodopa, 20 wt% of hydroxypropyl cellulose and 5 wt% of mint flavor; or
the overcoat is composed of 62.15 wt% of levodopa, 26.85 wt% of carbidopa, 10 wt% of hydroxypropyl cellulose, 0.9 wt% of aspartame and 0.1 wt% of mint flavor; or
the overcoat is composed of 24 wt% of levodopa, 65 wt% of carbidopa, 10 wt% of hydroxypropyl cellulose and 1 wt% of aspartame; or
the overcoat is composed of 54 wt% of levodopa, 35 wt% of carbidopa, 10 wt% of hydroxypropyl cellulose, 0.9 wt% of aspartame and 0.1 wt% of mint flavor; or
the overcoat is composed of 42.8 wt% of levodopa, 46.2 wt% of carbidopa, 10 wt% of hydroxypropyl cellulose, 0.9 wt% of aspartame and 0.1 wt% of mint flavor; or
the overcoat is composed of 28.2 wt% of levodopa, 60.8 wt% of carbidopa, 10 wt% of hydroxypropyl cellulose, 0.9 wt% of aspartame and 0.1 wt% of mint flavor.

More preferably, the weight gain of the overcoat relative to the tablet core is 12.9% or 13.2% or 15.7% or 21.0% of weight percentage.

Preferably, the pharmaceutical composition is composed of a drug-containing layer and a coating membrane, the drug-containing layer is composed of 40 wt% of levodopa, 10.8 wt% of carbidopa, 20 wt% of microcrystalline cellulose, 18.7 wt% of mannitol, 5 wt% of citric acid, 5 wt% of hydroxypropyl methyl cellulose sodium and 0.5 wt% of magnesium stearate, and wt% is the weight percentage of each component of the drug-containing layer. The coating membrane is composed of 50 wt% of cellulose acetate membrane and 50 wt% of copovidone VA64, and wt% is the weight percentage of each component of the coating membrane. The weight of the coating membrane is 2.0% of the weight of the tablet core. A dosage form containing the pharmaceutical composition has a 0.5 mm exit orifice mechanically drilled on the drug-containing layer side of the coated tablet, and levodopa and carbidopa are delivered at an average rate of 14.17 mg/hr and 4.59 mg/hr, with 85% of the drug delivered in 12 and 10 hours, respectively. The dosage form can be maintained in the oral cavity until the osmotic layer reaches the delivery orifice, or maintained in the oral cavity for 8-9 hours and then swallowed.

The pharmaceutical composition is composed of a drug-containing layer and a coating membrane, the drug-containing layer is composed of 38 wt% of levodopa, 50 wt% of microcrystalline cellulose, 10 wt% of hydroxypropyl methyl cellulose and 2 wt% of magnesium stearate, wherein wt% is the weight percentage of each component of the drug-containing layer. The coating membrane is composed of 50 wt% of cellulose acetate membrane and 50 wt% of copovidone VA64, wherein wt% is the weight percentage of each component of the coating membrane, and the weight of the coating membrane is 4.5% of the weight of tablet core. The dosage form with a membrane weight gain of 4.5% delivered levodopa at an average rate of 9.4 mg/hr, with 85% of levodopa delivered in 9.0 hours.

Or, the pharmaceutical composition is composed of a drug-containing layer and a coating membrane, the drug-containing layer comprises 19.5 wt% of levodopa, 20 wt% of carbidopa, 50 wt% of mannitol, 10 wt% of citric acid and 0.5 wt% of magnesium stearate, wherein wt% is the weight percentage of each component of the drug-containing layer. The coating membrane is composed of 50 wt% of cellulose acetate membrane and 50 wt% of copovidone VA64, wherein wt% is the weight percentage of each component of the coating membrane, and the weight of the coating membrane is 4.5% of the weight of tablet core. The dosage form with a membrane weight gain of 4.5% delivered levodopa at an average rate of 22.9 mg/hr, with 85% of levodopa delivered in 13.0 hours.

Preferably, in one embodiment, the pharmaceutical composition is composed of a drug-containing layer, an osmotic push layer, and a coating membrane, and the drug-containing layer is composed of 40 wt% of levodopa, 10.8 wt% of carbidopa, 31 wt% of hydroxypropyl cellulose, 12.7 wt% of mannitol, 5 wt% of citric acid and 0.5 wt% of magnesium stearate, wherein wt% is the weight percentage of each component of the drug-containing layer. The osmotic push layer is composed of 55 wt% of sodium carboxymethyl cellulose 7H4XF or 9H4XF, 5 wt% of povidone K30, 39 wt% of sorbitol, 0.5 wt% of iron oxide red and 0.5 wt% of magnesium stearate, wherein wt% is the weight percentage of each component of the osmotic push layer. The coating membrane is composed of 70 wt% of cellulose acetate membrane and 30 wt% of copovidone VA64, wherein wt% is the weight percentage of each component of the coating membrane. The weight of the coating membrane is 2.0%, 4.0% or 5.0% of the weight of the tablet core. When the sodium carboxymethyl cellulose is 7H4XF, one side of the drug-containing layer contains an exit orifice of 0.5 mm, the dosage form with a tablet core weight gain of 5.0% delivered levodopa and carbidopa at an average rate of 17.0 mg/hr and 4.6 mg/hr, with 85% of levodopa and carbidopa delivered in 10.0 hours. The dosage form can be maintained in the oral cavity until the osmotic layer reaches the delivery orifice, or maintained in the oral cavity for 6-7 hours and then swallow. The sizes of delivery orifice vary from 0.5 mm, 0.75 mm and 1.0 mm. The dosage form with a tablet core weight gain of 4.0% delivered levodopa and carbidopa at an average rate of 21.3 mg/hr and 5.7 mg/hr, with 85% of levodopa and carbidopa delivered in 8.0 hours. The dosage form can be maintained in the oral cavity until the osmotic layer reaches the delivery orifice, or maintained in the oral cavity for 4-5 hours and then swallow. When the sodium carboxymethyl cellulose is 9H4XF, the dosage form with a coating membrane weight gain of 2.0% delivered levodopa and carbidopa at an average rate of 24.3 mg/hr and 6.6 mg/hr, with 85% levodopa and carbidopa delivered in 7.0 hours. The dosage form can be maintained in the oral cavity until the osmotic layer reaches the delivery orifice, or maintained in the oral cavity for 3-4 hours and then swallow.

In another embodiment, the pharmaceutical composition is composed of a drug-containing layer, an osmotic push layer, and a coating membrane, the drug-containing layer is composed of 40 wt% of levodopa, 10.8 wt% of carbidopa, 31 wt% of hydroxypropyl cellulose, 12.7 wt% of mannitol, 5 wt% of citric acid and 0.5 wt% of povidone K30, wherein wt% is the weight percentage of each component of the drug-containing layer. The osmotic push layer is composed of 55 wt% of sodium carboxymethyl cellulose 7H4XF, 5 wt% of povidone K30, 39 wt% of sorbitol, 0.5 wt% of iron oxide red, and 0.5 wt% of magnesium stearate, wherein wt% is the weight percentage of each component of the osmotic push layer; the coating membrane is composed of 60 wt% of cellulose acetate membrane and 40% copovidone VA64, wherein wt% is the weight percentage of each component of the coating membrane, and the weight of the coating membrane is 5.0% of the weight of the tablet core. The dosage form containing the pharmaceutical composition delivered 85% of the drug in 6 hours. The dosage form can be maintained in the oral cavity until the osmotic layer reaches the delivery orifice, or maintained in the oral cavity for 2-3 hours and then swallowed.

In another embodiment, the pharmaceutical composition is composed of a drug-containing layer, an osmotic push layer, and a coating membrane, the drug-containing layer is composed of 45 wt% of levodopa, 31 wt% of hydroxypropyl cellulose, 16 wt% of mannitol, 5 wt% of povidone K30, and 1 wt% of magnesium stearate, 1 wt% of mint flavor and 1 wt% of aspartame, wherein wt% is the weight percentage of each component of the drug-containing layer; the osmotic push layer is composed of 55 wt% of sodium carboxymethyl cellulose 7H4XF, 10 wt% of povidone K30, 34 wt% of sorbitol, 0.5 wt% of iron oxide red and 0.5 wt% of magnesium stearate, wherein wt% is the weight percentage of each component of the osmotic push layer; the coating membrane is composed of 60 wt% of cellulose acetate membrane and 40 wt% of copovidone VA64, wherein wt% is the weight percentage of each component of the coating membrane; and the weight of the coating membrane is 4.2%, 6.7% or 9.7% of the weight of the tablet core. The dosage form containing the pharmaceutical composition with a membrane weight gain of 4.2%, 6.7% and 9.7% delivered levodopa at an average rate of 38.3 mg/hr, 27.3 mg/hr, and 21.3 mg/hr, with 85% of levodopa delivered in 5.0 hours, 7.0 hours and 9.0 hours, respectively.

In another embodiment, the pharmaceutical composition is composed of a drug-containing layer, an osmotic push layer, and a coating membrane, the drug-containing layer is composed of 45 wt% of levodopa, 31 wt% of hydroxypropyl cellulose, 16 wt% of mannitol, 5 wt% of povidone K30, 1 wt% of magnesium stearate, 1 wt% of mint flavor and 1 wt% of aspartame, wherein wt% is the weight percentage of each component of the drug-containing layer; the osmotic push layer is composed of 55 wt% of sodium carboxymethyl cellulose 7H4XF, 10 wt% of povidone K30, 34 wt% of sorbitol, 0.5 wt% of iron oxide red and 0.5 wt% of magnesium stearate, wherein wt% is the weight percentage of each component of the osmotic push layer; the coating membrane is composed of 70 wt% of cellulose acetate membrane and 30 wt% of copovidone VA64, wherein wt% is the weight percentage of each component of the coating membrane; and the weight of the coating membrane is 4.6% or 7.9% of the weight of the tablet core. The dosage form containing the pharmaceutical composition with a membrane weight gain of 4.6% and 7.9% delivered levodopa at an average rate of 25.5 mg/hr and 16.9 mg/hr, with 85% of levodopa delivered in 7.5 hours and 11.5 hours, respectively.

In another embodiment, the pharmaceutical composition is composed of a drug-containing layer, an osmotic push layer, and a coating membrane, the drug-containing layer is composed of 70 wt% of levodopa, 9 wt% of mannitol, 20 wt% of povidone K30, and 1 wt% of magnesium stearate, wherein wt% is the weight percentage of each component of the drug-containing layer; the osmotic push layer is composed of 85 wt% of sodium carboxymethyl cellulose, 3 wt% of povidone K30, 5 wt% of sorbitol, 5 wt% of iron oxide red and 2 wt% of magnesium stearate, wherein wt% is the weight percentage of each component of the osmotic push layer; the coating membrane is composed of 70 wt% of cellulose acetate membrane and 30 wt% of copovidone VA64, wherein wt% is the weight percentage of each component of the coating membrane; and the weight of the coating membrane is 4.5% of the weight of the tablet core. The dosage form containing the pharmaceutical composition delivered levodopa at an average rate of 35.0 mg/hr, with 85% of levodopa delivered in 8.5 hours.

In another embodiment, the pharmaceutical composition is composed of a drug-containing layer, an osmotic push layer, and a coating membrane, the drug-containing layer is composed of 20 wt% of levodopa, 20 wt% of carbidopa, 50 wt% of hydroxypropyl cellulose, 4 wt% of mannitol, 5 wt% of aspartame and 1 wt% of magnesium stearate, wherein wt% is the weight percentage of each component of the drug-containing layer; the osmotic push layer is composed of 25 wt% of sodium carboxymethyl cellulose, 9.5 wt% of povidone K30, 65 wt% of sorbitol and 0.5 wt% of magnesium stearate, wherein wt% is the weight percentage of each component of the osmotic push layer; the coating membrane is composed of 90 wt% of cellulose acetate membrane and 10 wt% of copovidone VA64, wherein wt% is the weight percentage of each component of the coating membrane; and the weight of the coating membrane is 4.5% of the weight of the tablet core. The dosage form containing the pharmaceutical composition delivered levodopa and carbidopa at an average rate of 7.1 mg/hr, with 85% of levodopa and carbidopa delivered in 12 hours.

Preferably, in one embodiment, the pharmaceutical composition is composed of a drug-containing layer, an osmotic push layer, a coating membrane and an overcoat, the drug-containing layer is composed of 45 wt% of levodopa, 31 wt% of hydroxypropyl cellulose, 16 wt% of mannitol, 5 wt% of povidone K30, 1 wt% of magnesium stearate, 1 wt% of mint flavor and 1 wt% of aspartame, wherein wt% is the weight percentage of each component of the drug-containing layer; the osmotic push layer is composed of 55 wt% of sodium carboxymethyl cellulose 7H4XF, 10 wt% of povidone K30, 34 wt% of sorbitol, 0.5 wt% of iron oxide red and 0.5 wt% of magnesium stearate, wherein wt% is the weight percentage of each component of the osmotic push layer; the coating membrane is composed of 70 wt% of cellulose acetate membrane and 30 wt% of copovidone VA64, wherein wt% is the weight percentage of each component of the coating membrane; and the weight of the coating membrane is 4.8% or 7.7% of the weight of the tablet core, the overcoat is composed of 23.78 wt% of levodopa, 64.22 wt% of carbidopa, 10 wt% of hydroxypropyl cellulose, 1 wt% of aspartame and 1 wt% of mint flavor immediate release composition, wherein wt% is the weight percentage of each component of the overcoat, and the weight gain of the overcoat relative to the tablet core is 13.2 % and 12.9 % by weight, respectively. The release profile of the dosage form containing the pharmaceutical composition showed a rapid release of levodopa/carbidopa, the dosage form with 4.8% and 7.7% weight gain in coating membrane followed by sustained release with a release duration of approximately 8.5 hours and 12.0 hours, respectively. The dosage form with a 4.8% membrane weight gain can be maintained in the oral cavity for 4-5 hours and then maintained in the oral cavity for the duration of the meal or throughout the release period. Dosage forms with a 7.7 % membrane weight gain can be maintained in the oral cavity for 8-9 hours before swallowing, or be maintained in the oral cavity for the entire release period.

In another embodiment, the pharmaceutical composition is composed of a drug-containing layer, an osmotic push layer, a coating membrane and an overcoat, the drug-containing layer is composed of 45 wt% of levodopa, 31 wt% of hydroxypropyl cellulose, 17 wt% of mannitol, 5 wt% of povidone K30, 1 wt% of magnesium stearate and 1 wt% of aspartame, wherein wt% is the weight percentage of each component of the drug-containing layer; the osmotic push layer is composed of 60 wt% of sodium carboxymethyl cellulose 7H4XF, 10 wt% of povidone K30, 26 wt% of sorbitol, 2 wt% of iron oxide red and 2 wt% of magnesium stearate, wherein wt% is the weight percentage of each component of the osmotic push layer; the coating membrane is composed of 70 wt% of cellulose acetate membrane and 30 wt% of copovidone VA64, wherein wt% is the weight percentage of each component of the coating membrane; and the weight of the coating membrane is 4.8% of the weight of the tablet core, the overcoat is composed of 93 wt% of CD, 2 wt% of hydroxypropyl cellulose and 5 wt% of aspartame immediate release composition, wherein wt% is the weight percentage of each component of the overcoat, and the weight increase of the overcoat relative to the tablet core is 13.2 % by weight. The immediate release overcoat of the dosage form is rapidly released first, followed by sustained releasing for approximately 8 hours. The dosage form can be maintained in the oral cavity for 4-5 hours and then swallow before eating or be maintained in the oral cavity for the entire release period.

In another embodiment, the pharmaceutical composition is composed of a drug-containing layer, an osmotic push layer, a coating membrane and an overcoat, the drug-containing layer is composed of 45 wt% of levodopa, 31 wt% of hydroxypropyl cellulose, 12 wt% of mannitol, 5 wt% of povidone K30, 5 wt% of mint flavor, 1 wt% of aspartame and 1 wt% of magnesium stearate, wherein wt% is the weight percentage of each component of the drug-containing layer; the osmotic push layer is composed of 40 wt% of sodium carboxymethyl cellulose 7H4XF, 20 wt% of povidone K30, 36 wt% of sorbitol, 3.5 wt% of iron oxide red and 0.5 wt% of magnesium stearate, wherein wt% is the weight percentage of each component of the osmotic push layer; the coating membrane is composed of 70 wt% of cellulose acetate membrane and 30 wt% of copovidone VA64, wherein wt% is the weight percentage of each component of the coating membrane; and the weight of the coating membrane is 4.8% of the weight of the tablet core, the overcoat is composed of 75 wt% of levodopa, 20 wt% of hydroxypropyl cellulose and 5 wt% of mint flavor immediate release composition, wherein wt% is the weight percentage of each component of the overcoat, and the weight gain of the overcoat relative to the tablet core is 13.2 % by weight. The immediate release overcoat of the dosage form is rapidly released first, followed by sustained releasing for approximately 8 hours. The dosage form can be maintained in the oral cavity for 4-5 hours and then swallow before eating or be maintained in the oral cavity for the entire release period.

In another embodiment, the pharmaceutical composition is composed of a drug-containing layer, an osmotic push layer, a coating membrane and an overcoat, the drug-containing layer is composed of 62.5 wt% of levodopa, 31 wt% of hydroxypropyl cellulose, 4.5 wt% of mannitol, 0.1 wt% of mint flavor, 0.9 wt% of aspartame and 1 wt% of magnesium stearate, wherein wt% is the weight percentage of each component of the drug-containing layer; the osmotic push layer is composed of 55 wt% of sodium carboxymethyl cellulose 7H4XF, 10 wt% of hydroxypropyl cellulose, 34 wt% of sorbitol, 0.5 wt% of iron oxide red and 0.5 wt% of magnesium stearate, wherein wt% is the weight percentage of each component of the osmotic push layer; the coating membrane is composed of 70 wt% of cellulose acetate membrane and 30 wt% of copovidone VA64, wherein wt% is the weight percentage of each component of the coating membrane; and the weight of the coating membrane is 6.5% of the weight of the tablet core, the overcoat is composed of 62.15 wt% of levodopa, 26.85 wt% of carbidopa, 10 wt% of hydroxypropyl cellulose, 0.9 wt% of aspartame and 0.1 wt% of mint flavor immediate release composition, wherein wt% is the weight percentage of each component of the overcoat, and the weight gain of the overcoat relative to the tablet core is 21.0 % by weight.

In another embodiment, the pharmaceutical composition is composed of a drug-containing layer, an osmotic push layer, a coating membrane and an overcoat, the drug-containing layer is composed of 46.9 wt% of levodopa, 31 wt% of hydroxypropyl cellulose, 20.1 wt% of mannitol, 0.1 wt% of mint flavor, 0.9 wt% of aspartame and 1 wt% of magnesium stearate, wherein wt% is the weight percentage of each component of the drug-containing layer; the osmotic push layer is composed of 55 wt% of sodium carboxymethyl cellulose 7H4XF, 10 wt% of hydroxypropyl cellulose, 34 wt% of sorbitol, 0.5 wt% of iron oxide red and 0.5 wt% of magnesium stearate, wherein wt% is the weight percentage of each component of the osmotic push layer; the coating membrane is composed of 70 wt% of cellulose acetate membrane and 30 wt% of copovidone VA64, wherein wt% is the weight percentage of each component of the coating membrane; and the weight of the coating membrane is 6.5% of the weight of the tablet core, the overcoat is composed of 62.15 wt% of levodopa, 26.85 wt% of carbidopa, 10 wt% of hydroxypropyl cellulose, 0.9 wt% of aspartame and 0.1 wt% of mint flavor immediate release composition, wherein wt% is the weight percentage of each component of the overcoat, and the weight increase of the overcoat relative to the tablet core is 15.7 % by weight.

In a specific preferred embodiment, the pharmaceutical composition is composed of a drug-containing layer, an osmotic push layer, a coating membrane and an overcoat; wherein the drug-containing layer is composed of 45 wt% of levodopa, 31 wt% of hydroxypropyl cellulose, 16 wt% of mannitol, 5 wt% of povidone K30, 1 wt% of magnesium stearate, 1 wt% of mint flavor and 1 wt% of aspartame, wherein wt% is the weight percentage of each component of the drug-containing layer; the osmotic push layer is composed of 55 wt% of sodium carboxymethyl cellulose 7H4XF, 10 wt% of hydroxypropyl cellulose, 34 wt% of sorbitol, 0.5 wt% of iron oxide red and 0.5 wt% of magnesium stearate, wherein wt% is the weight percentage of each component of the osmotic push layer; the coating membrane is composed of 70 wt% of cellulose acetate membrane and 30 wt% of copovidone VA64, wherein wt% is the weight percentage of each component of the coating membrane; wherein, the cellulose acetate is a cellulose acetate membrane containing 39.8 wt% of acetyl, and the weight of the coating membrane is 5.9 % of the weight of the tablet core; and, the overcoat is composed of 64.22 wt% of levodopa, 23.78 wt% of carbidopa, 10 wt% of hydroxypropyl cellulose, 1 wt% of aspartame and 1 wt% of mint flavor, wherein wt% is the weight percentage of each component of the overcoat, the weight of the overcoat is 13.1% of the total weight of the tablet core and the coating membrane. When the solvent for the coating suspension of the overcoat is absolute ethanol, the content of carbidopa-related genotoxic impurity hydrazine in the obtained dosage form is 1.7 ppm, the content of carbidopa-related impurity dihydroxyphenylacetone (DHPA) is 0.21%. When the solvent for the coating suspension of the overcoat is purified water, the concentration of the solid content of the overcoat is 10.0 wt%, including 24.0% wt% of levodopa, 65.0 wt% of carbidopa monohydrate, 10.0 wt% of hydroxylpropyl cellulose and 1.0 wt% of aspartame in weight percentage; the content of carbidopa-related genotoxic impurity hydrazine in the obtained dosage form is 3.8 ppm, and the content of carbidopa-related impurity DHPA is 0.28%. When the coating solution solvent of the overcoat is absolute ethanol, the carbidopa-related genotoxic impurity hydrazine and impurity DHPA of the obtained dosage form are significantly lower than the coating solution solvent of the overcoat is purified water. The immediate release overcoat of the dosage form is rapidly released first, followed by a sustained release with a duration of approximately 8 hours. The osmotic delivery system can be maintained in the oral cavity for 3-5 hours and then swallow before eating or be maintained in the oral cavity for the entire release period.

In a specific preferred embodiment, the pharmaceutical composition is composed of a drug-containing layer, an osmotic push layer, a coating membrane and an overcoat; the drug-containing layer is composed of 45 wt% of levodopa, 31 wt% of hydroxypropyl cellulose, 22 wt% of mannitol, 0.9 wt% of aspartame, 0.5 wt% of magnesium stearate and 0.1 wt% of mint flavor, wherein the weight percentage is the weight percentage of each component of the drug-containing layer; the osmotic push layer is composed of 55 wt% of sodium carboxymethyl cellulose 7H4XF, 10 wt% of hydroxypropyl cellulose, 34 wt% of sorbitol, 0.5 wt% of iron oxide red and 0.5 wt% of magnesium stearate, wherein wt% is the weight percentage of each component of the osmotic push layer; the coating membrane is composed of 70 wt% of cellulose acetate membrane and 30 wt% of copovidone VA64, wherein wt% is the weight percentage of each component of the coating membrane; wherein, the cellulose acetate is a cellulose acetate membrane containing 39.8 wt% of acetyl, and the weight of the coating membrane is 6.5 % of the weight of the tablet core; and, the overcoat is composed of 54 wt% of levodopa, 35 wt% of carbidopa, 10 wt% of hydroxypropyl cellulose, 0.9 wt% of aspartame and 0.1 wt% of mint flavor, wherein wt% is the weight percentage of each component of the overcoat, the weight of the overcoat is 13.1% of the total weight of the tablet core and the coating membrane. The final dosage form is composed of an immediate release overcoat of 62.5 mg levodopa and 37.5 mg carbidopa, and 187.5 mg levodopa was contained in an extended release drug-containing layer. The immediate release overcoat of the dosage form is rapidly released first, followed by sustained releasing for approximately 8 hours. The osmotic delivery system can be maintained in the oral cavity for 4-5 hours and then swallow before eating or be maintained in the oral cavity for the entire release period.

In a specific preferred embodiment, the pharmaceutical composition is composed of a drug-containing layer, an osmotic push layer, a coating membrane and an overcoat; the drug-containing layer is composed of 45 wt% of levodopa, 31 wt% of hydroxypropyl cellulose, 22 wt% of mannitol, 0.9 wt% of aspartame, 1 wt% of magnesium stearate and 0.1 wt% of mint flavor, wherein wt% is the weight percentage of each component of the drug-containing layer; the osmotic push layer is composed of 55 wt% of sodium carboxymethyl cellulose 7H4XF, 10 wt% of hydroxypropyl cellulose, 34 wt% of sorbitol, 0.5 wt% of iron oxide red and 0.5 wt% of magnesium stearate, wherein wt% is the weight percentage of each component of the osmotic push layer; the coating membrane is composed of 70 wt% of cellulose acetate membrane and 30 wt% of copovidone VA64, wherein wt% is the weight percentage of each component of the coating membrane; wherein, the cellulose acetate is a cellulose acetate membrane containing 39.8 wt% of acetyl, and the weight of the coating membrane is 7.0 % of the weight of the tablet core; and, the overcoat is composed of 42.8 wt% of levodopa, 46.2 wt% of carbidopa, 10 wt% of hydroxypropyl cellulose, 0.9 wt% of aspartame and 0.1 wt% of mint flavor, wherein wt% is the weight percentage of each component of the overcoat, the weight of the overcoat is 13.1% of the total weight of the tablet core and the coating membrane. The final dosage form is composed of an immediate release overcoat of 37.5 mg levodopa and 37.5 mg carbidopa, and 112.5 mg levodopa was contained in an extended release drug-containing layer. The immediate release overcoat of the dosage form is rapidly released first, followed by sustained releasing for approximately 8 hours. The osmotic delivery system can be maintained in the oral cavity for 4-5 hours and then swallow before eating or be maintained in the oral cavity for the entire release period.

In a specific preferred embodiment, the pharmaceutical composition is composed of a drug-containing layer, an osmotic push layer, a coating membrane and an overcoat; the drug-containing layer is composed of 45 wt% of levodopa, 31 wt% of hydroxypropyl cellulose, 22 wt% of mannitol, 0.9 wt% of aspartame, 1 wt% of magnesium stearate and 0.1 wt% of mint flavor, wherein wt% is the weight percentage of each component of the drug-containing layer; the osmotic push layer is composed of 55 wt% of sodium carboxymethyl cellulose 7H4XF, 10 wt% of hydroxypropyl cellulose, 34 wt% of sorbitol, 0.5 wt% of iron oxide red and 0.5 wt% of magnesium stearate, wherein wt% is the weight percentage of each component of the osmotic push layer; the coating membrane is composed of 70 wt% of cellulose acetate membrane and 30 wt% of copovidone VA64, wherein wt% is the weight percentage of each component of the coating membrane; wherein, the cellulose acetate is a cellulose acetate membrane containing 39.8 wt% of acetyl, and the weight of the coating membrane is 9.0 % of the weight of the tablet core; and, the overcoat is composed of 28.2 wt% of levodopa, 60.8 wt% of carbidopa, 10 wt% of hydroxypropyl cellulose, 0.9 wt% of aspartame and 0.1 wt% of mint flavor, wherein wt% is the weight percentage of each component of the overcoat, the weight of the overcoat is 13.1% of the total weight of the tablet core and the coating membrane. The final dosage form is composed of an immediate release overcoat of 18.75 mg levodopa and 37.5 mg carbidopa, and 56.25 mg levodopa was contained in an extended release drug-containing layer. The immediate release overcoat of the dosage form is rapidly released first, followed by sustained releasing for approximately 8 hours. The osmotic delivery system can be maintained in the oral cavity for 4-5 hours and then swallow before eating or be maintained in the oral cavity for the entire release period.

Herein, the preparation method of the overcoat contains: dissolving each component of the above-mentioned weight percent of the overcoat in absolute ethanol to prepare a coating suspension. Preferably, the ratio of the components of the overcoat to the absolute ethanol is 1:10. In the pharmaceutical composition, carbidopa is only present in an immediate release overcoat, and the core portion of the extended release tablet does not contain carbidopa. The dosage form has the following advantages: the content of carbidopa related genotoxic impurity hydrazine and impurity dihydroxyphenylacetone (DHPA) is lower. When the solvent for the coating suspension of the overcoat is absolute ethanol, the carbidopa related genotoxic impurity hydrazine and impurity DHPA of the obtained dosage form are significantly lower than that of the obtained dosage form when the solvent for the coating suspension is pure water.

Unless otherwise specified, the method for preparing the aforementioned pharmaceutical composition is a conventional preparation method in the art.

Preferably, as described above, the pharmaceutical composition is an osmotic pump extended release drug delivery system. Preferably, the osmotic pump extended release drug delivery system is an extended release tablet. More preferably, the extended release tablet is a cylinder with a diameter of 5-10 mm and a height of 5-30 mm, or a caplet with a length of 10-25 mm and a width of 5-10 mm. Most preferably, each of the extended release tablets contains 62.5 mg CD and 500 mg LD, or 62.5 mg CD and 375 mg LD, or 62.5 mg carbidopa and 250 mg levodopa, or 50 mg carbidopa and 500 mg levodopa, or 37.5mg carbidopa and 375mg levodopa.

To solve the above technical problems, one of the technical solutions of the present invention is: a method for using the above extended release dosage form, that is, placing the extended release platform in the personalized retention enabling platform, and fastening the retention enabling platform on the corresponding teeth in the oral cavity; taking out the extended release dosage form after keeping for 4-24 hours, replacing the extended release platform with a new one and fastening the retention enabling platform on the corresponding teeth in the oral cavity again, so that the drug can be released sustainably and stably.

In order to solve the problem in the prior art that a drug delivery device is at a risk of falling off for some patients wearing a drug delivery device after an action such as opening their mouths widely, another aspect of the present disclosure is: an oral drug delivery device, which comprises a core component and a fixing component, and the core component comprises a molar-fittingfunctional area and a drug-loaded functional area which are integrally formed;
When the oral drug delivery device is fixed in the oral cavity, the drug-loaded functional area is located in the space between the teeth and the cheek, or the drug-loaded functional area is located in the space between the teeth and the tongue;
The molar-fitting functional area comprises a first section close to the drug-loaded functional area and a second section far away from the drug-loaded functional area; one end of the first section and the second section are connected to each other, and the other end is an open end, which forms a U-shaped structure; the first section and the second section are used to anastomose with the buccal and lingual sides of the teeth respectively, and are used to fix the drug-loaded functional area on the teeth;
The drug-loaded functional area comprises a first ring and a second ring arranged coaxially; the axial clamping space formed between the first ring and the second ring is used to fix the drug;
The fixing component is a fixator or a fixing layer, used to strengthen the fixing effect of the core component on the teeth;
When the fixing component is a fixator:
   When the oral drug delivery device is in use, the lingual, buccal and occlusal surfaces of the fixator correspond to the lingual, buccal and occlusal surfaces of the teeth and cover the outer peripheral surface of the teeth; the fixator simultaneously covers the molar-fitting functional area;
   When the fixing component is a fixing layer:
      The fixing component is attached to the outer surfaces of the first section and the second section for contact with teeth.

In the present disclosure, the oral drug delivery device is similar to the oral retention device described above.

In the present disclosure, the first ring and the second ring are preferably located on the buccal side of the first or second molar.

In the present disclosure, the fixator and the molar-fitting functional area are preferably connected by means of mechanical assembly, mechanical connection or adhesive bonding.

Herein, the mechanical assembly is preferably embedment, and slotted holes are provided in the fixator at the position corresponding to the drug-loaded functional area on the fixator to achieve removable fixation between the fixator and the core component. Embedment means that the molar-fitting functional area is embedded in the space formed by both sides of the fixator.

Herein, the mechanical connection is preferably welding, riveting or bolting.

Herein, the adhesive is preferably one or more selected from pressure-sensitive adhesive, starch glue, latex, epoxy resin and polyurethane acrylate.

In the present disclosure, the fixing layer is preferably also attached to other outer surfaces of the first section and the second section.

In the present disclosure, the first ring is close to the open end of the molar-fitting functional area, and the second ring is far away from the open end of the molar-fitting functional area. The first ring and the second ring are preferably solid or hollow rings.

Herein, the hollow ring is a closed ring or an open ring.

Herein, the shape of the ring is preferably one or more selected from circle, ellipse, and polygon.

Herein, the shapes of the ring of the first ring and the second ring are preferably circular; the hollow area of the first ring is preferably smaller than that of the second ring, and the medicinal tablet can be inserted from the second ring to the first ring.

In the present disclosure, the material of the core component is preferably one of titanium, stainless steel, cobalt chromium alloy, cobalt chromium molybdenum alloy or precious metal, more preferably cobalt chromium alloy, and the materials of the core component are all meet the standards for dental materials.

In the present disclosure, the material of the fixator is preferably one or more selected from polyvinyl chloride, polyethylene terephthalate, polyethylene terephthalate-1,4-cyclohexanedimethpolyvinyl chloride, polyurethane, polyamide, ethylene-vinyl acetate copolymer, polycaprolactone, high-density polyethylene, polypropylene, epoxy acrylate, methacrylate, and polyurethane acrylate; more preferably, polyethylene terephthalate or ethylene-vinyl acetate copolymer, the materials of the fixator all meet standards for medical grade.

In the present disclosure, the material of the fixing layer is preferably one or more selected from polyvinyl chloride, polyethylene terephthalate, polyethylene terephthalate-1,4-cyclohexanedimethylene terephthalate, polyurethane, polyamide, ethylene-vinyl acetate copolymer, polycaprolactone, high-density polyethylene, polypropylene, cellulose acetate, hydroxypropyl cellulose and copovidone; more preferably, polyethylene terephthalate or copovidone, the materials of the fixing layer all meet standards for medical grade.

In the present disclosure, the thickness of the fixing layer is preferably 0.01mm-1mm.

In the present disclosure, the number of teeth covered by the fixator can be adjusted according to the condition of the teeth and the wearing firmness. The length of the fixator is preferably corresponding to the length of 4-16 teeth in the upper jaw or lower jaw, and more preferably is corresponding to the length of 5-9 teeth in the maxillary and mandible.

In the present disclosure, the length of the molar-fitting functional area is preferably corresponding to the length of 2-5 teeth in the mandible.

Those skilled in the art would understand that the length is a length that enables the fixator or the molar-fitting functional area to cover the number of teeth, and does not exceed the number of teeth within the target range.

In a preferred embodiment, the fixator is connected to the molar-fitting functional area in an embedded manner, and slotted holes are provided in the retainer at the position corresponding to the drug-loaded functional area on the fixator to achieve removable fixation between the fixator and the core component; the length of the molar-fitting functional area is corresponding to the length of the mandibular first and second premolars and the first and second molars; the first ring and the second ring are circular closed rings.

In a preferred embodiment, the fixator is connected to the molar-fitting functional area in an embedded manner; the length of the molar-fitting functional area is corresponding to the length of the mandibular first and second premolars and the first and second molars; the first ring and the second ring are elliptical closed rings.

In a preferred embodiment, the fixator is connected to the molar-fitting functional area in an embedded manner; the length of the molar-fitting functional area is corresponding to the length of the maxillary second premolar and the first, second, and third molars; the first ring and the second ring are circular closed rings.

In a preferred embodiment, the fixator is connected to the molar-fitting functional area using pressure-sensitive adhesive as an adhesive; the length of the molar-fitting functional area is corresponding to the length of the first, second and third molars; the first ring and the second ring are polygonal closed rings.

In a preferred embodiment, the fixing layer is attached to all outer surfaces of the first section of the U-shaped structure and the second section of the U-shaped structure; the length of the molar-fitting functional area is corresponding to the length of the mandibular first and second premolars and the first and second molars; the first ring and the second ring are circular closed rings.

In a preferred embodiment, a pair of clamping walls are symmetrically provided on the first ring in the direction of the open end of the molar-fitting functional area.

Another technical solution of the present disclosure is: a method for preparing the above-mentioned oral drug delivery device, which comprises the following steps: when the fixing component is a fixator, combining the fixator with the integrally formed first section and the second section in a wrapped form; when the fixing component is a fixing layer, the fixing layer is attached to the outer surfaces of the integrally formed first section and second section.

In the present disclosure, a method for preparing the core component preferably comprises laser casting, injection molding or impression molding.

Herein, the steps of laser casting are preferably as follows: scanning the subject's oral cavity or taking an oral mold to make a plaster model and then scanning to obtain the subject's oral data; using three-dimensional design software and dental design software to respectively design the drug-loaded functional area and the molar-fitting functional area of the core component, and combining them in the dental design software to generate a design file for the core component; and preparing the core component by laser casting, grinding, polishing, and cleaning.

Herein, the steps of injection molding are preferably as follows: taking an oral mold of the subject to make a plaster model; preparing a dental wax model on the plaster model using dental wax as a material, and preparing the core component through a traditional injection molding process.

In the present disclosure, a method for preparing the fixator preferably comprises thermoforming, impression, cutting or 3D printing.

Herein, the steps of thermoforming are preferably as follows: heating and softening the raw material of the fixator through a thermoforming machine and then vacuuming, and covering the raw material of the fixator on the dental mold and the molar-fitting functional area.

Herein, the steps of impression are preferably as follows: heating and softening the raw material of the fixator, and covering it on the subject's teeth and the molar-fitting functional area.

In the present disclosure, the raw material of the fixator is preferably as described above.

In the present disclosure, a method for preparing the fixing layer preferably comprises dipping, spraying or thermoforming.

Herein, the steps of dipping are preferably as follows: after dissolving the raw material of the fixing layer, immersing the molar-fitting functional area into the raw material solution of the fixing layer, taking it out, and drying it to form a membrane.

Herein, the steps of spraying are preferably as follows: after dissolving the raw material of the fixing layer, coating it to the molar-fitting functional area by spraying, and drying it to form a membrane.

Herein, the steps of thermoforming are preferably as follows: heating and softening the raw material of the fixing layer, covering it on the anastomosis functional area, and then cooling it.

In the present disclosure, the raw material of the fixing layer is preferably as described above.

In the present disclosure, the wrap structure of the fixator and the first section and the second section is preferably formed by mechanical assembly, mechanical connection or adhesive bonding.

Herein, the mechanical assembly is preferably in the form of embedment.

When the combination method is mechanical assembly, the fixator fits with the dental mold and the core component during the molding process, naturally forming a slot-like structure. At the same time, a buckle design can be added to the molar-fitting functional area as needed.

Herein, the mechanical connection is preferably in the form of welding, riveting or bolting.

Herein, the coating position of the adhesive is preferably the outer surfaces of the first section and the second section for contact with the teeth, and more preferably is the entire outer surfaces of the first section and the second section.

Herein, the coating thickness of the adhesive is preferably no more than 1.0 mm.

Another technical solution of the present disclosure is: an application of the above-mentioned oral drug delivery device, characterized in that: when the fixing component is a fixator, anastomosing the lingual, buccal and occlusal sides of the fixator with the lingual, buccal and occlusal surfaces of the teeth and wrapping around the peripheral surfaces of the teeth; placing the drug-loaded functional area in the space between the teeth and the cheek, or placing the drug-loaded functional area in the space between the teeth and the tongue; and wrapping the molar-fitting functional area with the fixator;

When the fixing component is a fixing layer: placing the drug-loaded functional area in the space between the teeth and the cheek, or placing the drug-loaded functional area in the space between the teeth and the tongue.

Another technical solution of the present disclosure is: an extended release platform, which is as described above.

As mentioned above, the extended release platform or pharmaceutical composition of the present disclosure can also called osmotic pump tablet. The osmotic pump tablet of the present disclosure can be used in conjunction with the above-mentioned oral retention device or oral drug delivery device.

Another aspect of the present disclosure relates to an osmotic pump tablet, which comprises a tablet core and a coating membrane wrapped around the tablet core, the coating membrane has a drug-release orifice, the tablet core comprises a drug-containing layer, the drug-containing layer comprises active pharmaceutical ingredient(s), a hydrophilic polymer, and a surfactant, wherein the hydrophilic polymer comprises hydroxypropyl cellulose and the surfactant comprises poloxamer.

In some embodiments, based on the total weight of the drug-containing layer, the content of the hydrophilic polymer is 5 wt%-25 wt%, such as 10 wt%-20 wt%, such as 10 wt%-15 wt%.

In some embodiments, the hydroxypropyl cellulose preferably has a weight average molecular weight of about 80,000. Brookfield viscosity of the hydroxypropyl cellulose is about 300-600 mPa.s. The concentration is about 10%.

In some embodiments, the hydroxypropyl cellulose is hydroxypropyl cellulose EXF.

In some embodiments, the hydrophilic polymer may further comprise conventional hydrophilic polymer in the art, such as one or more selected from hydroxypropylmethyl cellulose, carboxymethyl cellulose, polyvinyl pyrrolidone and hydroxyethyl cellulose. That is, the hydrophilic polymer is a mixture of hydroxypropyl cellulose and one or more selected from hydroxypropylmethyl cellulose, carboxymethyl cellulose, polyvinyl pyrrolidone and hydroxyethyl cellulose.

In some embodiments, based on the total weight of the drug-containing layer, the content of the surfactant is 1 wt%-15 wt%, such as 2 wt%-10 wt%, such as 5 wt%-10 wt%.

In some embodiments, the poloxamer is poloxamer 407 (P407).

In some embodiments, the surfactant may further comprise conventional surfactant in the art, such as one or more selected from polysorbates, fatty acid glycerides, sodium dodecyl benzene sulfonate and sodium dodecyl sulfate. That is, the surfactant is a mixture of poloxamer and one or more selected from polysorbates, fatty acid glycerides, sodium dodecyl benzene sulfonate, and sodium dodecyl sulfate.

In some embodiments, based on the total weight of the drug-containing layer, the content of the active pharmaceutical ingredient(s) is 50 wt%-75 wt%, such as 55 wt%-65 wt%, such as 58 wt%-63 wt%.

In some embodiments, the active pharmaceutical ingredient(s) is active pharmaceutical ingredient(s) that are released in the oral cavity.

In some embodiments, the active pharmaceutical ingredient(s) is drugs with an oral topical treatment or an absorption site in the upper gastrointestinal tract.

In some embodiments, the active pharmaceutical ingredient(s) is one or more selected from levodopa or its ester or its salt, carbidopa, baclofen, acyclovir, valacyclovir, ganciclovir, metformin, and gabapentin; preferably one or two of levodopa or its ester, and carbidopa. Wherein the ester of levodopa can be levodopa alkyl ester or deuterated levodopa alkyl ester, such as levodopa methyl ester hydrochloride. The salt of levodopa is, for example, levodopa ethyl ester hydrochloride.

In some embodiments, the active pharmaceutical ingredient(s) is, for example, selected from antifungal drugs and antitumor drugs. Herein the antifungal drug is, for example, one or more selected from nystatin, fluconazole, posaconazole, isavuconazole, voriconazole, anidulafungin, caspofungin and micafungin. The antitumor drug is, for example, one or more selected from 5-fluorouracil, paclitaxel and capecitabine.

In some embodiments, the drug-containing layer further comprises other excipients, other excipients are one or more selected from an osmotic agent, a pharmaceutical carrier, a binding agent, a lubricant, an antioxidant, and a flavoring agent.

In some embodiments, the osmotic agent is one or more selected from magnesium sulfate, magnesium chloride, sodium chloride, lithium chloride, potassium sulfate, sodium sulfate, mannitol, urea, sorbitol, inositol, sucrose, glucose, lactose, starch, pregelatinized starch, dextrin and microcrystalline cellulose, such as sorbitol or mannitol. Preferably, based on the total weight of the drug-containing layer, the content of the osmotic agent is 0-50 wt% but not 0 wt%, such as 5-20 wt%, and for example 10.0 wt%, 14.87 wt%, 15.0 wt% and 19.87 wt%.

In some embodiments, the pharmaceutical carrier is one or more selected from povidone (such as povidone K30 or povidone K29/32), copovidone, carbomer, hydroxypropyl methyl cellulose, hydroxyethyl cellulose, polyoxyethylene and sodium alginate. Preferably, based on the total weight of the drug-containing layer, the content of the pharmaceutical carrier is 0-50 wt%, but not 0%; for example 0.5-50 wt%, for another example 5-50 wt%, or yet another example 5-10 wt%.In some embodiments, the binding agent is one or more selected from methyl cellulose, hydroxypropyl methyl cellulose, hydroxyethyl cellulose, hydroxypropyl cellulose, sodium carboxymethyl cellulose, povidone (such as povidone K30 or povidone K29/32), copovidone and gelatin. Preferably, based on the total weight of the drug-containing layer, the content of the binding agent is 0-50 wt%, but not 0%; for example 0.5-50 wt%, for another example 5-50 wt%, or yet another example 5-10 wt%.

In some embodiments, povidone serves as a hydrophilic polymer, a pharmaceutical carrier and a binding agent.

In some embodiments, hydroxypropyl cellulose serves as both a hydrophilic polymer and a binding agent.

In some embodiments, the lubricant is one or more selected from stearic acid, magnesium stearate, magnesium fumarate stearate, calcium stearate, sodium stearate fumarate, polyethylene glycol, talc and colloidal silica, such as one or both of magnesium stearate and colloidal silica. Preferably, based on the total weight of the drug-containing layer, the content of the lubricant is 0-3 wt% but not 0 wt%, such as 0.5 wt%-2.5 wt%, another for example 1-2wt%.

In some embodiments, the antioxidant can be one or more selected from dibutylhydroxytoluene, butylhydroxyanisole, tert-butylhydroquinone, propyl gallate, vitamin C and vitamin E, such as dibutylhydroxytoluene. Preferably, based on the total weight of the drug-containing layer, the content of the antioxidant is 0-1 wt%, but not 0, such as 0.01-0.1 wt%.

In some embodiments, the flavoring agent is one or more selected from aspartame, apple flavor, orange flavor, banana flavor, mint flavor, saccharin sodium and stevioside, for example, one or two of aspartame and mint flavor. Preferably, based on the total weight of the drug-containing layer, the content of the flavoring agent is 0-10wt%, e.g. 0-2 wt%, but not 0; such as 1.0 wt%. When the flavoring agent is a combination of aspartame and mint flavor, the content of aspartame is 0.9 wt% and the content of mint flavor is 0.1 wt%.

In some embodiments, the drug-containing layer comprises active pharmaceutical ingredient(s), a hydrophilic polymer, a surfactant, a binding agent, an osmotic agent, a lubricant, and a flavoring agent. Alternatively, the drug-containing layer comprises active pharmaceutical ingredient(s), a hydrophilic polymer, a surfactant, a binding agent, an osmotic agent, a lubricant, an antioxidant and a flavoring agent. Alternatively, the drug-containing layer comprises active pharmaceutical ingredient(s), a hydrophilic polymer, a surfactant, an osmotic agent, a lubricant, an antioxidant and a flavoring agent. Alternatively, the drug-containing layer comprises the active pharmaceutical ingredient(s), a hydrophilic polymer, a surfactant, a binding agent, an osmotic agent and a lubricant.

In some embodiments, the drug-containing layer comprises any one of the following combinations:
(1) 50-75 wt% of the active pharmaceutical ingredient(s), 5-25 wt% of the hydrophilic polymer, 1-15 wt% of the surfactant, 0.5-50 wt% of the binding agent, 5-20 wt% of the osmotic agent, 0.5-3 wt% of the lubricant, and 1-10 wt% of the flavoring agent;
(2) 50-75 wt% of the active pharmaceutical ingredient(s), 5-25 wt% of the hydrophilic polymer, 1-15 wt% of the surfactant, 0.5-50 wt% of the binding agent, 5-20 wt% of the osmotic agent, 0.5-3 wt% of the lubricant, 0.01-1 wt% of the antioxidant, and 1-10 wt% of the flavoring agent;
(3) 50-75 wt% of the active pharmaceutical ingredient(s), 5-25 wt% of the hydrophilic polymer, 1-15 wt% of the surfactant, 5-20 wt% of the osmotic agent, 0.5-3 wt% of the lubricant, 0.01-1 wt% of the antioxidant, and 1-10 wt% of the flavoring agent; and
(4) 50-75 wt% of the active pharmaceutical ingredient(s), 5-25 wt% of the hydrophilic polymer, 1-15 wt% of the surfactant, 0.5-50 wt% of the binding agent, 5-20 wt% of the osmotic agent, and 0.5-3 wt% of the lubricant.

In some embodiments, the drug-containing layer comprises 63.0 wt% of levodopa, 10.0 wt% of hydroxypropyl cellulose (EXF, with a weight average molecular weight of 80,000), 10.0 wt% of poloxamer (P407), 5.0 wt% of povidone (K29/32), 10.0 wt% of sorbitol, 0.9 wt% of aspartame, 0.1 wt% of mint flavor and 1.0 wt% of magnesium stearate.

In some embodiments, the drug-containing layer comprises 58.0 wt% of levodopa, 15.0 wt% of hydroxypropyl cellulose (EXF, with a weight average molecular weight of 80,000), 5.0 wt% of poloxamer (P407), 5.0 wt% of povidone (K29/32), 15.0 wt% of sorbitol, 0.9 wt% of aspartame, 0.1 wt% of mint flavor and 1.0 wt% of magnesium stearate.

In some embodiments, the drug-containing layer comprises 58.0 wt% of levodopa, 15.0 wt% of hydroxypropyl cellulose (EXF, with a weight average molecular weight of 80,000), 5.0 wt% of povidone (K29/32), 5.0 wt% of poloxamer (P407), 15.0 wt% of sorbitol, 0.9 wt% of aspartame, 0.1 wt% of mint flavor and 1.0 wt% of magnesium stearate.

In some embodiments, the drug-containing layer comprises 54.9 wt% of levodopa, 3.16 wt% of carbidopa, 15.0 wt% of hydroxypropyl cellulose (EXF, with a weight average molecular weight of 80,000), 5.0 wt% of povidone (K29/32), 14.87 wt% of sorbitol, 5.0 wt% of poloxamer (P407), 0.9 wt% of aspartame, 0.1 wt% of mint flavor, 0.1 wt% of dibutylhydroxytoluene and 1.0 wt% of magnesium stearate.

In some embodiments, the drug-containing layer comprises 54.9 wt% of levodopa, 3.16 wt% of carbidopa, 15.0 wt% of hydroxypropyl cellulose (EXF, with a weight average molecular weight of 80,000), 19.87 wt% of mannitol, 0.9 wt% of aspartame, 5.0 wt% of poloxamer (P407), 0.1 wt% of mint flavor, 0.1 wt% of dibutylhydroxytoluene and 1.0 wt% of magnesium stearate.

In some embodiments, the drug-containing layer comprises 63.0wt% of levodopa, 11.0wt% of hydroxypropyl cellulose (EXF), 5.00wt% of povidone (K29/32), 10.0wt% of sorbitol, 10.0wt% of poloxamer (P407), and 1.00wt% of magnesium stearate.

In some embodiments, the drug-containing layer comprises 63.0wt% of levodopa, 11.0wt% of hydroxypropyl cellulose (EXF), 5.00wt% of povidone (K29/32), 8.50wt% of sorbitol, 10.0wt% of poloxamer (P407), 0.50wt% of colloidal silica, and 2.00wt% of magnesium stearate.

In some embodiments, the tablet core further comprises a push layer. The drug-containing layer and the push layer are sequentially laminated together to obtain a bi-layer tablet core. The coating membrane is wrapped around the outside of the tablet core. In the present disclosure, the terms "push layer" and "osmotic push layer" as previously described are used interchangeably and have the same meaning.In some embodiments, the mass ratio of the drug-containing layer to the push layer is about 0.5:1-4:1, such as 1.5:1-3.5:1.

In some embodiments, the push layer comprises one or more selected from a swelling agent, an osmotic agent, a binding agent, a lubricant and a coloring agent, preferably a combination of a swelling agent, an osmotic agent, a binding agent and a coloring agent, or a combination of a swelling agent, an osmotic agent, a binding agent, a lubricant and a coloring agent.

In some embodiments, the swelling agent is one or more selected from a sodium carboxymethyl starch, hydroxypropylmethyl cellulose, sodium carboxymethyl cellulose, hydroxyethyl cellulose, carbomer, sodium alginate, κ-carrageenan, sodium carboxymethyl cellulose and polyethylene oxide; for example, sodium carboxymethyl cellulose (7H4XF). Preferably, based on the total weight of the push layer, the content of the swelling agent is 30-95 wt%, such as 49.0-68.5 wt%.

In some embodiments, the osmotic agent is one or more selected from magnesium sulfate, magnesium chloride, sodium chloride, lithium chloride, potassium sulfate, sodium sulfate, mannitol, urea, sorbitol, inositol, sucrose, glucose, lactose, starch, pregelatinized starch, dextrin and microcrystalline cellulose, such as sorbitol or mannitol. Preferably, based on the total weight of the push layer, the content of the osmotic agent is 5-70 wt%, such as 10.0-30.0 wt%.

In some embodiments, the binding agent is one or more selected from methyl cellulose, hydroxypropyl methyl cellulose, hydroxyethyl cellulose, hydroxypropyl cellulose, sodium carboxymethyl cellulose, povidone, copovidone and gelatin, such as hydroxypropyl cellulose (EXF). Preferably, based on the total weight of the push layer, the content of the binding agent is 3-25 wt%, such as 20 wt%.

In some embodiments, the lubricant is stearic acid, magnesium stearate, magnesium fumarate stearate, calcium stearate, sodium stearyl fumarate, polyethylene glycol, talc and colloidal silica, such as one or two of magnesium stearate and colloidal silica. Preferably, based on the total weight of the push layer, the content of the lubricant is 0-7 wt% but not 0 wt%, such as 0 wt%-3 wt%, such as 0.5 wt%-1 wt%.

In some embodiments, the coloring agent is one or more selected from iron oxide red, iron oxide yellow, iron oxide violet and iron oxide black, such as iron oxide red. Preferably, based on the total weight of the push layer, the content of the coloring agent is 0-2 wt% but not 0 wt%, such as 0.5 wt%.

In some embodiments, the push layer comprises a swelling agent, an osmotic agent, a binding agent, a lubricant and a coloring agent.

In some embodiments, the push layer comprises 49.0 wt% of sodium carboxymethyl cellulose (7H4XF), 30 wt% of sorbitol, 20 wt% of hydroxypropyl cellulose (EXF), 0.5 wt% of iron oxide red and 0.5 wt% of magnesium stearate.

In some embodiments, the push layer comprises 68.5 wt% of sodium carboxymethyl cellulose (7H4XF), 10.0 wt% of sorbitol, 20.0 wt% of hydroxypropyl cellulose (EXF), 0.5 wt% of iron oxide red, 0.5 wt% of colloidal silica and 0.5 wt% of magnesium stearate.

In some embodiments, the push layer comprises 55.0 wt% of sodium carboxymethyl cellulose (7H4XF), 34.0 wt% of sorbitol, 10.0 wt% of hydroxypropyl cellulose (EXF), 0.50 wt% of iron oxide red and 0.50 wt% of magnesium stearate.

In some embodiments, the osmotic pump tablet further comprises an isolation layer. The drug-containing layer, the push layer and the isolation layer are sequentially laminated together to obtain a three-layer tablet core. The coating membrane is wrapped around the outside of the tablet core. Preferably, the isolation layer comprises one or more selected from ethyl cellulose, cellulose acetate, acrylic resin and microcrystalline cellulose, such as ethyl cellulose. Preferably, the mass ratio of the drug-containing layer to the isolation layer is (0.01-0.15):1, such as 0.05:1-0.1:1.

In some embodiments, the coating membrane comprises a membrane-forming material and a porogen. Preferably, the membrane-forming material is one or more selected from cellulose acetate, ethyl cellulose and acrylic resin, such as cellulose acetate, or cellulose acetate containing 39.8 wt% acetyl. Preferably, based on the total weight of the coating membrane, the content of the membrane-forming material is 50-70 wt%, such as 50wt%-55 wt%. Preferably, the porogen is copovidone, such as copovidone VA64. Preferably, based on the total weight of the coating membrane, the content of the porogen is 30-50 wt%, such as 45-50 wt%.

In some embodiments, the coating membrane further comprises a plasticizer. The plasticizer can be a conventional plasticizer in the field of pharmaceuticals, for example, one or more selected from polyethylene glycol, methyl phthalate, ethyl phthalate, dibutyl sebacate, triethyl citrate, tributyl citrate, tributyl acetyl citrate, glyceryl acetate and castor oil, such as triethyl citrate or polyethylene glycol 400. Preferably, based on the total weight of the coating membrane, the content of the plasticizer is 0-20 wt%, such as 0-5 wt%, but not 0 wt%.

In some embodiments, the coating membrane comprises a membrane-forming material and a porogen, or comprises a membrane-forming material, a porogen and a plasticizer.

In some embodiments, the tensile strength of the coating membrane is 1-10 MPa.

In some embodiments, the break elongation of the coating membrane is 1.1-2.0.

In some embodiments, the average thickness of the coating membrane is 100±8 µm to 200±10 µm.

In some embodiments, the pore size of the drug-release orifice may be 0.3 mm-1.2 mm.

In some embodiments, based on the total weight of the osmotic pump tablet, the content of the coating membrane is 2 wt%-10 wt%, such as 4.5 wt%, 5.5 wt%, 6.0 wt%, 6.5 wt%, 7.0 wt%, 8.0wt%, 9.0 wt% or 9.5wt%.

In some embodiments, the osmotic pump tablet may further comprise a drug-containing immediate release overcoat. The drug-containing immediate release overcoat comprises active pharmaceutical ingredient(s) and a pharmaceutical excipient, the active pharmaceutical ingredient(s) comprises one or more selected from levodopa, ester of levodopa, salt of levodopa and carbidopa, and the pharmaceutical excipient comprises one or more selected from a binding agent, an antioxidant, a plasticizer and a flavoring agent.

In some embodiments, the active pharmaceutical ingredient(s) comprises levodopa, carbidopa and a combination of the two. The pharmaceutical excipient preferably comprises one or more of a binding agent, an antioxidant and a plasticizer, preferably a binding agent.

The binding agent, the antioxidant and the flavoring agent are as described above (e.g., those mentioned for the drug-containing layer in the osmotic pump tablet). The plasticizer is as described above (e.g. those mentioned for the coating membrane in the osmotic tablet). Here, the binding agent is preferably hydroxypropyl cellulose (EXF). The antioxidant is preferably dibutylhydroxytoluene. The plasticizer is preferably triethyl citrate. The flavoring agent is preferably one or two of aspartame and mint flavor.

In some embodiments, the content of the active pharmaceutical ingredient(s) is preferably 70-90 wt%, such as 77.3-77.5 wt%.

In some embodiments, the content of the binding agent is 10-20 wt%.

In some embodiments, the content of the antioxidant is preferably 0.5-2.0 wt%, such as 1.47-1.66 wt%.

In some embodiments, the content of the plasticizer is preferably 1-2 wt%, such as 1.0 wt%.

In some embodiments, in the drug-containing immediate release overcoat, the active pharmaceutical ingredient(s) comprises levodopa and/or carbidopa, wherein when comprising two active pharmaceutical ingredients, the mass of levodopa is 12.5-200 mg; the mass of carbidopa is 10.5-200 mg. The mass ratio of levodopa and carbidopa is preferably 1:1-4:1.

In some embodiments, in the drug-containing immediate release overcoat, the mass of levodopa and carbidopa are 12.5 mg and 12.5 mg, 18.75 mg and 10.8 mg, 25 mg and 25 mg, 37.5 mg and 37.5 mg, 50 mg and 50 mg, 62.5 mg and 62.5 mg, 75 mg and 75 mg, 87.5 mg and 87.5 mg, 100 mg and 100 mg, 125 mg and 125 mg, 150 mg and 150 mg, 200 mg and 200 mg, 25 mg and 12.5 mg, 50 mg and 25 mg, 75 mg and 37.5 mg, 100 mg and 50 mg, 150 mg and 75 mg, 200 mg and 100 mg, 50 mg and 12.5 mg, 100 mg and 25 mg, 150 mg and 37.5 mg, or 200 mg and 50 mg, respectively. If carbidopa is used in the form of carbidopa monohydrate, the mass of carbidopa shall be replaced by the mass of carbidopa monohydrate (equivalent proportion is replaced according to the mass of carbidopa).

Preferably, the drug-containing immediate release overcoat comprises the active pharmaceutical ingredient(s) and an excipient, wherein the excipient is a binding agent, hydroxypropyl cellulose (EXF). In some embodiments, the content of the active pharmaceutical ingredient(s) is preferably 80-90 wt%. In some embodiments, the content of the binding agent is 10-20.0 wt%.

In some embodiments, the drug-containing immediate release overcoat comprises 46.7 wt% carbidopa monohydrate, 43.3 wt% levodopa, and 10.0 wt% hydroxypropyl cellulose (EXF).

In some embodiments, the drug-containing immediate release overcoat comprises 37.7 wt% carbidopa monohydrate, 52.3 wt% levodopa, and 10.0 wt% hydroxypropyl cellulose (EXF).

In some embodiments, the drug-containing immediate release overcoat comprises 27.2 wt% carbidopa monohydrate, 62.9 wt% levodopa, and 10.0 wt% hydroxypropyl cellulose (EXF).

In some embodiments, the drug-containing immediate release overcoat comprises 90.0 wt% carbidopa monohydrate and 10.0 wt% hydroxypropyl cellulose (EXF).

Another aspect of the present disclosure relates to an osmotic pump tablet, which comprises any one of the following Schemes:
Scheme (A): the osmotic pump tablet comprises a tablet core, a coating membrane wrapping the tablet core, and a drug-containing immediate release overcoat, wherein the coating membrane has a drug delivery orifice, the tablet core comprises a drug-containing layer, the drug-containing layer comprises the active pharmaceutical ingredient(s) and a hydrophilic polymer, but does not comprise a surfactant; the hydrophilic polymer comprises povidone K29/32; or
Scheme (B): the osmotic pump tablet comprises a tablet core, a coating membrane wrapping the tablet core, and a drug-containing immediate release overcoat, wherein the coating membrane has a drug delivery orifice, the tablet core comprises a drug-containing layer, the drug-containing layer comprises active pharmaceutical ingredient(s) and an excipient; the drug-containing immediate release overcoat comprises active pharmaceutical ingredient(s) and a pharmaceutical excipient, wherein the pharmaceutical excipient is a binding agent.

In Scheme (A), the active pharmaceutical ingredient(s) and the excipient are each as described in the present disclosure.

In Scheme (A), the active pharmaceutical ingredient(s) preferably comprises levodopa and/or carbidopa. In Scheme (A), when the active pharmaceutical ingredient(s) comprises levodopa, based on the total weight of the drug-containing layer, the content of levodopa is 19.5-75 wt%; for example, 19.5-70 wt% (such as 19.5 wt%, 20 wt%, 38 wt%, 40 wt%, 45 wt%, 46.9 wt%, 62.5 wt%, or 70 wt%). In another embodiment, when the active pharmaceutical ingredient(s) comprises carbidopa, based on the total weight of the drug-containing layer, the content of carbidopa is 0-20 wt% but not 0%, for example, 10%-20 wt% (for example, 10.8 wt% carbidopa monohydrate).

In Scheme (A), based on the total weight of the drug-containing layer, the content of the active pharmaceutical ingredient(s) is 30-75 wt%, for example, 38-65 wt% (such as 38 wt%, 39.5 wt%, 40 wt%, 45 wt%, 50 wt%, or 70 wt%).

In Scheme (A), based on the total weight of the drug-containing layer, the content of the hydrophilic polymer povidone K29/32 is preferably 0.5-50 wt%; for example, 0.5-20 wt%, or for yet another example, 5-10 wt%.

In Scheme (A), the hydrophilic polymer may also be a mixture of povidone K29/32 and one or more selected from hydroxypropyl cellulose, Hydroxypropylmethyl cellulose, carboxymethyl cellulose, and hydroxyethyl cellulose.

In Scheme (A), the excipient is selected from one or more of a filler, an osmotic agent, an acidifying agent, a lubricant, and a flavoring agent.

Preferably, when the excipient comprises a filler, the filler is one or more of microcrystalline cellulose, hydroxypropyl cellulose, and mannitol. Based on the total weight of the drug-containing layer, the content of the filler is preferably 0-50 wt% but not 0%; preferably 20-50 wt%, or for yet another example, 31 wt%.

Preferably, when the excipient comprises an osmotic agent, the osmotic agent is one or more of magnesium sulfate, magnesium chloride, sodium chloride, lithium chloride, potassium sulfate, sodium sulfate, mannitol, urea, sorbitol, inositol, sucrose, and glucose. Based on the total weight of the drug-containing layer, the content of the osmotic agent is preferably 0-50 wt% but not 0%; for example, 4-50 wt%; or for yet another example, 4 wt%, 4.5 wt%, 9 wt%, 12 wt%, 12.7 wt%, 14.87 wt%, 15 wt%, 16 wt%, 16.5 wt%, 17 wt%, 18 wt%, 18.7 wt%, 19.87 wt%, 20.1 wt%, 22 wt%, or 50 wt%.

Preferably, when the excipient comprises an acidifying agent, the acidifying agent is one or more of citric acid, sodium citrate, potassium citrate, malic acid, fumaric acid, lactic acid, phosphoric acid, and tartaric acid. Based on the total weight of the drug-containing layer, the content of the acidifying agent is preferably 0-10 wt% but not 0%; for example, 5-10 wt%.

Preferably, when the excipient comprises a lubricant, the lubricant is one or more of magnesium stearate, magnesium stearate fumarate, talc, and colloidal silica, for example, one or two of magnesium stearate and colloidal silica. Based on the total weight of the drug-containing layer, the content of the lubricant is 0.5-3 wt%; for example, 0.5-2.5 wt%, or for yet another example, 1-2 wt%.

Preferably, when the excipient comprises a flavoring agent, the flavoring agent is one or more of aspartame, apple flavor, orange flavor, banana flavor, mint flavor, sodium saccharin, and stevioside, for example, one or two of aspartame and mint flavor (when two are selected, they may be present in any ratio); preferably, based on the total weight of the drug-containing layer, the content of the flavoring agent is 0-10 wt%, but not 0; for example, 1-6 wt%; or for yet another example, 2-5 wt%.

In Scheme (A), the excipient is preferably any one of the following combinations: (1) a filler, an osmotic agent, a hydrophilic polymer, an acidifying agent, and a lubricant; (2) a filler, a hydrophilic polymer, and a lubricant; (3) a filler, an osmotic agent, a hydrophilic polymer, a lubricant, and a flavoring agent; (4) an osmotic agent, a hydrophilic polymer, and a lubricant; (5) a filler, a hydrophilic polymer, an osmotic agent, and a lubricant; (6) a filler, an osmotic agent, an acidifying agent, and a lubricant; (7) a filler, an osmotic agent, a flavoring agent, and a lubricant; and (8) a filler, an acidifying agent, and a lubricant. The excipient is preferably a filler, a hydrophilic polymer, an osmotic agent, and a lubricant.

In Scheme (A), the drug-containing layer comprises 30-75 wt% active pharmaceutical ingredient(s), 0.5-20 wt% hydrophilic polymer, 20-50 wt% filler, 4-50 wt% osmotic agent, and 0.5-3 wt% lubricant; for example, the drug-containing layer comprises 45.0 wt% levodopa, 31.0 wt% hydroxypropyl cellulose (EXF), 5.00 wt% povidone K29/32, 18.0 wt% mannitol, and 1.00 wt% magnesium stearate; or alternatively, the drug-containing layer comprises 45.0 wt% levodopa, 31.0 wt% hydroxypropyl cellulose (EXF), 5.00 wt% povidone K29/32, 16.5 wt% mannitol, 0.50 wt% colloidal silica, and 2.00 wt% magnesium stearate.

In Schemes (A) and (B), the tablet core further comprises a push layer. The drug-containing layer and the push layer are sequentially laminated to obtain a bilayer tablet core. The coating membrane is wrapped around the outside of the tablet core. In the present disclosure, the push layer and the osmotic push layer as described above have the same meaning and may be used interchangeably. The push layer in the osmotic pump tablet is as described in the present disclosure.

In Schemes (A) and (B), the coating membrane in the osmotic pump tablet is as described in the present disclosure.

In Schemes (A) and (B), the osmotic pump tablet further comprises an isolation layer. The isolation layer is as described in the present disclosure.

In Scheme (A), the osmotic pump tablet may further comprise a drug-containing immediate release overcoat on the outside thereof. The drug-containing immediate release layer in the osmotic pump tablet is as described in the present disclosure.

In Scheme (B), in the drug-containing immediate release overcoat, the binding agent is preferably hydroxypropyl cellulose (EXF).

In Scheme (B), in the drug-containing immediate release overcoat, except for the pharmaceutical excipient, the drug-containing immediate release layer in the osmotic pump tablet is as described in the present disclosure.

In Scheme (B), in the drug-containing immediate release overcoat, the content of the active pharmaceutical ingredient(s) is preferably 80-90 wt%; and the content of the pharmaceutical excipient is preferably 10-20.0 wt%.

In Scheme (B), in the drug-containing immediate release overcoat, the active pharmaceutical ingredient(s) comprises levodopa and/or carbidopa, wherein, when two active pharmaceutical ingredient(s) is present, the mass ratio of levodopa to carbidopa is preferably 1:1 to 4:1.

In Scheme (B), the drug-containing layer in the osmotic pump tablet is as described in the present disclosure.

In Scheme (B), the drug-containing layer is preferably any one of the following combinations:
(1) 30-75 wt% of the active pharmaceutical ingredient, 20-50 wt% of the filler, 4-50 wt% of the osmotic agent, 0.5-20 wt% of the hydrophilic polymer, 5-10 wt% of the acidifying agent, and 0.5-3 wt% of the lubricant; for example, 40.0 wt% of levodopa (LD), 10.8 wt% of carbidopa monohydrate (CD), 20.0 wt% of microcrystalline cellulose, 18.7 wt% of mannitol, 5.0 wt% of hydroxypropyl methyl cellulose (HPMC E5), 5.0 wt% of citric acid, and 0.5 wt% of magnesium stearate;
(2) 30-75 wt% of the active pharmaceutical ingredient, 20-50 wt% of the filler, 0.5-20 wt% of the hydrophilic polymer, and 0.5-3 wt% of the lubricant; for example, 38.0 wt% of levodopa, 50.0 wt% of microcrystalline cellulose, 10.0 wt% of hydroxypropyl methyl cellulose, and 2.0 wt% of magnesium stearate;
(3) 30-75 wt% of the active pharmaceutical ingredient, 20-50 wt% of the filler, 4-50 wt% of the osmotic agent, 5-10 wt% of the acidifying agent, and 0.5-3 wt% of the lubricant; for example, 40.0 wt% of levodopa, 10.8 wt% of carbidopa monohydrate, 31.0 wt% of hydroxypropyl cellulose with an average molecular weight of 80,000, 12.7 wt% of mannitol, 5.0 wt% of citric acid, and 0.5 wt% of magnesium stearate;
(4) 30-75 wt% of the active pharmaceutical ingredient, 20-50 wt% of the filler, 4-50 wt% of the osmotic agent, 0.5-20 wt% of the hydrophilic polymer, 0.5-3 wt% of the lubricant, and 1-6 wt% of the flavoring agent, for example, 45.0 wt% of levodopa, 31.0 wt% of hydroxypropyl cellulose (Klucel EXF), 16.0 wt% of mannitol, 5.0 wt% of povidone K30, 1.0 wt% of aspartame, 1.0 wt% of mint flavor, and 1.0 wt% of magnesium stearate; or 45.0 wt% of levodopa, 31.0 wt% of hydroxypropyl cellulose, 17.0 wt% of mannitol, 5.0 wt% of povidone K30, 1.0 wt% of magnesium stearate, and 1.0 wt% of aspartame; or 45.0 wt% of levodopa, 31.0 wt% of hydroxypropyl cellulose, 17.0 wt% of mannitol, 5.0 wt% of povidone K30, 1.0 wt% of magnesium stearate, and 1.0 wt% of aspartame;
(5) 30-75 wt% of the active pharmaceutical ingredient, 4-50 wt% of the osmotic agent, 5-20 wt% of the hydrophilic polymer, and 0.5-3 wt% of the lubricant; for example, 70.0 wt% of levodopa, 9.0 wt% of mannitol, 20.0 wt% of povidone K30, and 1.0 wt% of magnesium stearate;
(6) 30-75 wt% of the active pharmaceutical ingredient, 20-50 wt% of the filler, 4-50 wt% of the osmotic agent, 1-6 wt% of the flavoring agent, and 0.5-3 wt% of the lubricant; for example, 20.0 wt% of levodopa, 20.0 wt% of carbidopa, 50.0 wt% of hydroxypropyl cellulose, 4.0 wt% of mannitol, 5.0 wt% of aspartame, and 1.0 wt% of magnesium stearate; or, 45.0 wt% of levodopa, 31.0 wt% of hydroxypropyl cellulose, 22.0 wt% of mannitol, 0.9 wt% of aspartame, 0.1 wt% of mint flavor, and 1.0 wt% of magnesium stearate;
(7) 30-75 wt% of the active pharmaceutical ingredient, 20-50 wt% of the filler, 5-10 wt% of the acidifying agent, and 0.5-3 wt% of the lubricant; for example, 19.5 wt% of levodopa, 20.0 wt% of carbidopa, 50.0 wt% of mannitol, 10.0 wt% of citric acid, and 0.5 wt% of magnesium stearate;
(8) 30-75 wt% of the active pharmaceutical ingredient, 0.5-20 wt% of the hydrophilic polymer, 20-50 wt% of the filler, 4-50 wt% of the osmotic agent, and 0.5-3 wt% of the lubricant; for example, 45.0 wt% of levodopa, 31.0 wt% of hydroxypropyl cellulose (such as hydroxypropyl cellulose EXF), 5.00 wt% of povidone (such as povidone K29/32), 18.0 wt% of mannitol, and 1.00 wt% of magnesium stearate; or, 45.0 wt% of levodopa, 31.0 wt% of hydroxypropyl cellulose (such as hydroxypropyl cellulose EXF), 5.00 wt% of povidone (such as povidone K29/32), 16.5 wt% of mannitol, 0.50 wt% of colloidal silica, and 2.00 wt% of magnesium stearate.

One of the technical solutions of the present disclosure is: a use of the osmotic pump tablet as described above in the manufacture of a medicament for the treatment of fluctuations in motor symptoms in patients with advanced Parkinson's disease.

One of the technical solutions of the present disclosure is: a method for preparing the osmotic pump tablet as described above. The method for preparing the osmotic pump tablet comprises the following steps:
S1. preparing the drug-containing layer granules, which comprises the following steps: active pharmaceutical ingredient(s) and a excipient are mixed and granulated to obtain drug-containing layer granules;
S2. preparing push layer granules, which comprises the following steps: at least one of the push layer excipients is granulated to obtain push layer granules;
S3. preparing a bi-layer tablet core: the drug-containing layer granules and the push layer granules obtained from steps S1 and S2 are pressed to obtain a bi-layer tablet core;
S4. preparing an osmotic pump tablet: wrapping the tablet core with a coating membrane and perforating a drug-release orifice, thereby obtaining the osmotic pump tablet;
S5. optionally, after the completion of step S4, further comprising the step of wrapping a drug-containing immediate release overcoat on the outside of the osmotic pump tablet;

Optionally, between step S3 and step S4, further comprising the step of preparing an isolation layer; the step of preparing the isolation layer comprises pressing the isolation layer material and the bi-layer tablet core.

Step S5 is mandatory in Scheme (B).

The method for preparing the osmotic pump tablet preferably comprises the following steps:
S1. preparing drug-containing layer granules, which comprises the following steps: active pharmaceutical ingredient(s), a hydrophilic polymer, a surfactant, and optionally other excipients are mixed and granulated to obtain drug-containing layer granules;
S2. preparing push layer granules, which comprises the following steps: at least one of a swelling agent, an osmotic agent, a binding agent, a lubricant and a coloring agent are granulated to obtain push layer granules;
S3. preparing a bi-layer tablet core: drug-containing layer granules and push layer granules obtained from steps S1 and S2 are pressed to obtain a bi-layer tablet core;
S4. preparing an osmotic pump tablet: wrapping the tablet core with a coating membrane and perforating a drug-release orifice, thereby obtaining the osmotic pump tablet.

In some embodiments, in steps S1 and S2, it preferably comprises the step of screening each component prior to the mixing. The screening is preferably carried out through a 40 mesh sieve. Granulating is each independently dry granulating, wet granulating or fluidized bed granulating. When wet granulating is used, fluidized bed drying is preferred after the granulation is completed. After the granulation is completed, a step of further granulating is preferably included. The further granulating is preferably passed through a 1.2 mm sieve.

In some embodiments, in step S3, the process of pressing may be a conventional process in the art. Wherein the tablet pressing die used is preferably a 7.0 mm round punch.

In some embodiments, the drug-release orifice is obtained by laser perforation or mechanical perforation.

In some embodiments, in step S1, when the active pharmaceutical ingredient(s) comprise levodopa and carbidopa, the following steps are preferably included:
(a) levodopa, a hydrophilic polymer, a binding agent (or a pharmaceutical carrier,) or an osmotic agent, and a flavoring agent are mixed and granulated to obtain levodopa-containing granules;
(b) carbidopa monohydrate, a surfactant and an osmotic agent are mixed and granulated to obtain carbidopa-containing granules;
(c) after mixing the granules obtained from steps (a) and (b), mixing them with a lubricant, a flavoring agent, and optionally an antioxidant to obtain drug-containing layer granules.

Specifically, the raw material components of the drug-containing immediate release overcoat are mixed with an alcoholic solvent to obtain a solution, and then the obtained solution is sprayed onto the osmotic pump tablet. Wherein, the alcoholic solvent is preferably ethanol. The solid content of the obtained solution is about 10 wt% (the solid content refers to the sum of the weights of all solid components of the coating solution divided by the total weight of the coating solution).

Another technical solution of the present disclosure is: a method of using the above-mentioned osmotic pump tablet, that is, placing the osmotic pump tablet in the oral retention device or the oral drug delivery device for use together.

Without violating the common knowledge in the art, the preferred parameters described above may be optionally combined to obtain the preferred embodiments in the present disclosure.

The reagents and raw materials employed in the present disclosure are commercially available.The positive and progressive effects of the present disclosure are as follows:

The osmotic pump tablet of the present disclosure has at least one of the following characteristics:
(1) the osmotic pump tablet is kept in the oral cavity for 2-24 hours, and 85% of the active pharmaceutical ingredients are released within 4-24 hours, such as 5-16 hours, and the active pharmaceutical ingredients are sustainably released in the oral cavity;
(2) the active pharmaceutical ingredients quickly enter the digestive tract during the drug release process and are not easily retained or accumulated in the oral cavity, the retention amount of the active pharmaceutical ingredients at the drug release site is less than 10%. The drug content uniformity of the drug-containing immediate release overcoat is excellent, with an SD value as low as 4%;
(3) the coating membrane used for controlled release does not break; the active pharmaceutical ingredient residue in the osmotic pump tablet is less than 15%;
(4) the released active pharmaceutical ingredients are sustainably swallowed into the gastrointestinal tract, providing sustained drug absorption and stable blood drug concentration;
(5) the osmotic pump tablet further comprises a drug-containing immediate release overcoat, the drug release and the blood drug concentration in the body are easily adjusted to quickly reach the therapeutic concentration and maintain within the therapeutic concentration range for 5-16 hours;
(6) the osmotic pump tablet is a high-dose osmotic pump tablet with active pharmaceutical ingredients up to 200-2000 mg in a single osmotic pump tablet; in particular, when the active pharmaceutical ingredients contain levodopa, the drug loading of the levodopa reaches 200 mg-1500 mg; when the active pharmaceutical ingredients contain carbidopa, the drug loading of the carbidopa reaches 1 mg-200 mg;
(7) the drug loading of active pharmaceutical ingredients in the drug-containing layer reaches 50-75%;
(8) the permeability of the drug in the tablet in the aqueous medium is 8-150 mg/h (the permeability refers to the amount of mg of drug released in the dissolution experiment per hour per unit time).

### BRIEF DESCRIPTION OF THE DRAWINGS

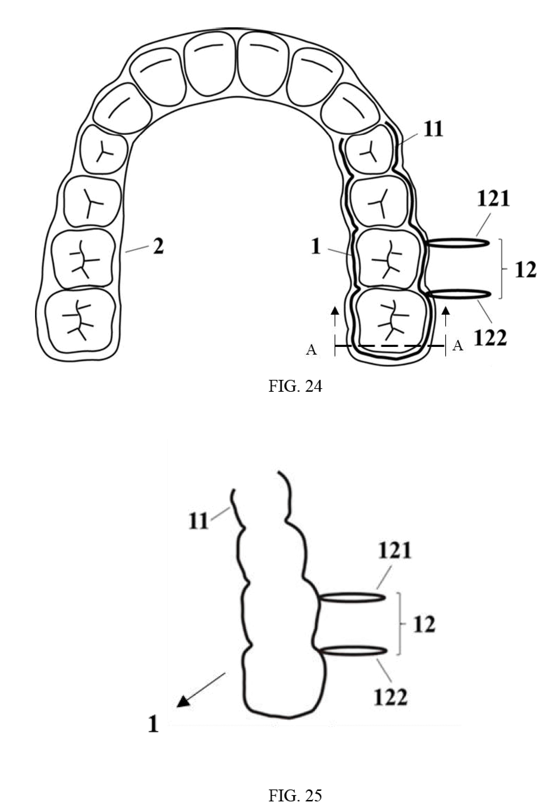

FIG. 1A and FIG. 1B are schematic diagrams of an extended release platform (ERP): FIG. 1A. a single-layer elementary osmotic pump (EOP); FIG. 1B. a bi-layer osmotic push-pull system.
FIG. 2 is a schematic diagram of an extended release dosage form (combination of extended release platform ERP and retention enabling platform REP): 1. personalized retention enabling modules of retention enabling platform (REP); 2. extended release platform (ERP); 3. drug fastened modules of retention enabling platform (REP).
FIGS. 3A-3C are flow charts of manufacturing the extended release dosage form (ERP + REP) of the present disclosure: FIG. 3A. the extended release platform is a single-layer elementary osmotic pump (EOP); FIG. 3B. the extended release platform is a bi-layer osmotic push-pull system; FIG. 3C. the extended release platform is a bi-layer push-pull system with an immediate-release drug overcoat.
FIG. 4 shows the preparation of the retention enabling platform (REP) described in Embodiment 1: 4A. drug fastened module; 4B. drug fastened module; 4C. drug fastened module; 4D. drug fastened module; 4E. thermoplastic tablet; 4F. personalized retention enabling platform (REP).
FIG. 5 is a drug fastened module described in Embodiment 1: 5A. drug fastened module; 5B. extended release platform fastened in the drug fastened module.

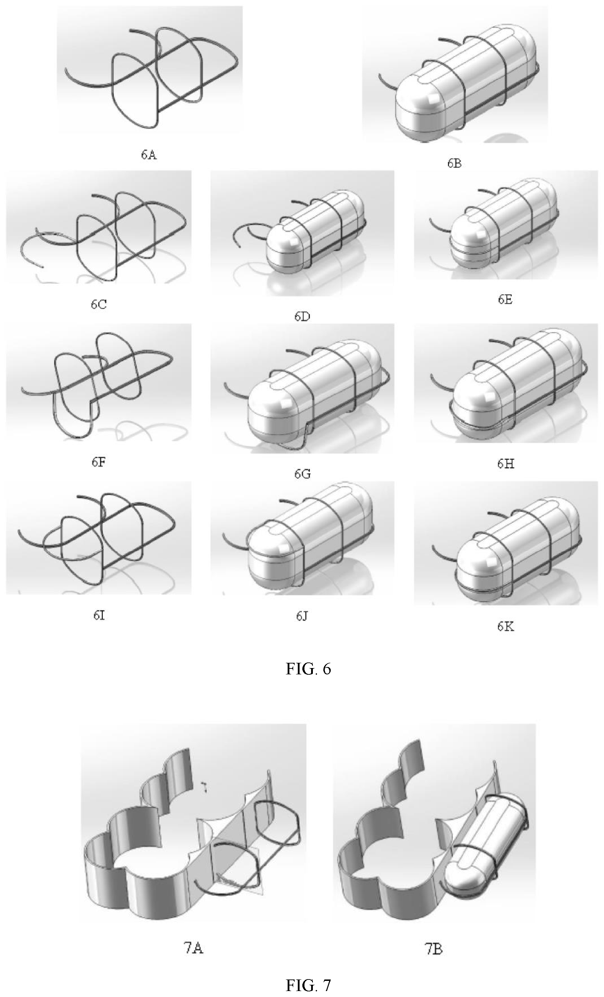

FIG. 6 shows the retention enabling platform (REP) described in Embodiment 2 and Embodiment 3: 6A. retention enabling platform (REP); 6B. extended release platform fastened in the retention enabling platform (REP); 6C. retention enabling platform (REP) with self-locking cover; 6D. extended release platform fastened in the retention enabling platform (REP); 6E. extended release platform with the cover closed + retention enabling platform (REP); 6F. retention enabling platform (REP) with reversible cover; 6G. extended release platform fastened in the retention enabling platform (REP); 6H. extended release platform with the cover closed + retention enabling platform (REP); 6I. retention enabling platform (REP) with a cover that can slide up and down; 6J. extended release platform fastened to the retention enabling platform (REP); 6K. extended release platform with cover closed + retention enabling platform (REP).
FIG. 7 is a retention enabling platform (REP) described in Embodiment 4 and Embodiment 5: 7A. retention enabling platform (REP); 7B. extended release platform fastened in the retention enabling platform (REP).
FIG. 8 is a release profile of the extended release platform (ERP) described in Embodiment 6, and error bars indicate a standard deviation of n = 3.
FIG. 9 is a release profile of the extended release platform (ERP) described in Embodiment 7, and error bars indicate a standard deviation of n = 3.
FIG. 10 is a release profile of the extended release platform (ERP) described in Embodiment 8, and error bars indicate a standard deviation of n = 3.
FIG. 11 is a release profile of the extended release platform (ERP) described in Embodiment 9, and error bars indicate a standard deviation of n = 3.
FIG. 12 is a release profile of the extended release platform (ERP) described in Embodiment 10, and error bars indicate a standard deviation of n = 3.
FIG. 13 is a release profile of the extended release platform (ERP) described in Embodiment 11, and error bars indicate a standard deviation of n = 3.
FIG. 14 is a release profile of the extended release platform (ERP) described in Embodiment 12, and error bars indicate a standard deviation of n = 3.
FIG. 15 is a release profile of the extended release platform (ERP) described in Embodiment 13, and error bars indicate a standard deviation of n = 3.
FIG. 16 is a release profile of the extended release platform (ERP) described in Embodiment 23, and error bars indicate a standard deviation of n = 3.
FIG. 17 is a release profile of the extended release platform (ERP) described in Embodiment 24, and error bars indicate a standard deviation of n = 3.
FIG. 18 is a release profile of the extended release platform (ERP) described in Embodiment 25, and error bars indicate a standard deviation of n = 3.
FIG. 19 is a release profile of the extended release platform (ERP) described in Embodiment 26, and error bars indicate a standard deviation of n = 3.
FIG. 20 is a release profile of the extended release platform (ERP) described in Embodiment 27, and error bars indicate a standard deviation of n = 3.
FIG. 21 is a release profile of the extended release dosage form (ERP+REP) described in Embodiment 29, and error bars indicate a standard deviation of n = 3.
FIG. 22 is an oral retention device with a medicinal tablet being inserted from the back according to an embodiment of the present disclosure, the device being composed of a tooth-matching component 11 and a drug-loaded component 41, wherein the drug-loaded component 41 is composed of a retainer 21 and a ring body 31, the ring body 31 being provided with an opening 311.
FIG. 23 is an oral retention device with a medicinal tablet being inserted from the front according to an embodiment of the present disclosure, the device being composed of a tooth-matching component 12 and a drug-loaded component 42, wherein the drug-loaded component 42 is composed of a retainer 22 and a ring body 32, the ring body 32 being provided with an opening 321.
   List of reference numerals:
   11: tooth-matching component of back-insertion oral retention device
   21: retainer of back-insertion oral retention device
   31: ring body of back-insertion oral retention device
   311: opening on ring body of back-insertion oral retention device
   41: drug-loaded component of back-insertion oral retention device
   12: tooth-matching component of front-insertion oral retention device
   22: retainer of front-insertion oral retention device
   32: ring body of front-insertion oral retention device
   321: opening on ring body of front-insertion oral retention device
   42: drug-loaded component of front-insertion oral retention device
FIG. 24 is a schematic diagram of each component of the oral drug delivery device of Embodiment 35 of the present disclosure, which is composed of a core component 1 and a fixing component 2, wherein the core component 1 comprises a molar-fitting functional area 11 and a drug-loaded functional area 12, and the drug-loaded functional area 12 is composed of a first ring 121 and a second ring 122.
FIG. 25 is a schematic diagram of the core component in the oral drug delivery device of Embodiment 35 of the present disclosure.

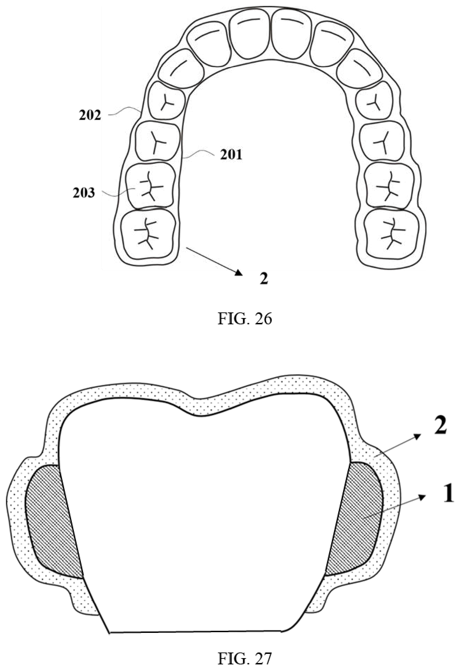

FIG. 26 is a schematic diagram of a fixator in the oral drug delivery device of Embodiment 35 of the present disclosure when the fixing component is a fixator.
FIG. 27 is a cross-sectional view taken along plane A-A in FIG. 24.

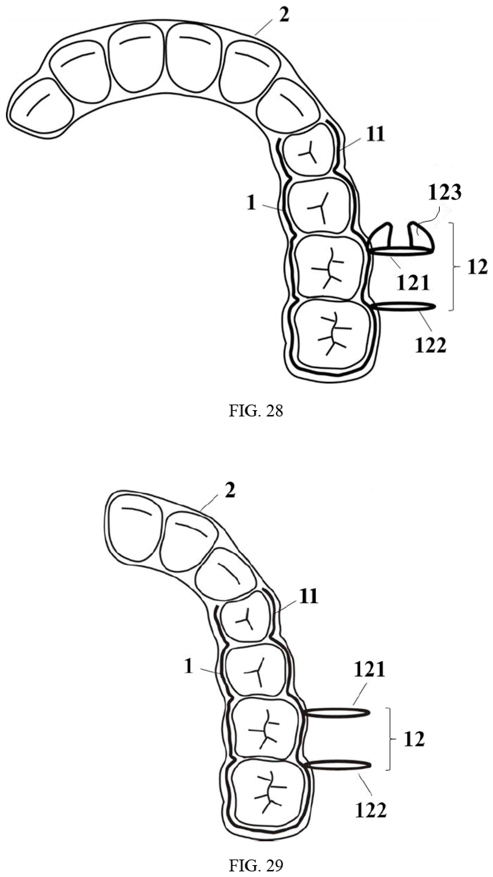

FIG. 28 is a schematic diagram of the oral drug delivery device of Embodiment 36.
FIG. 29 is a schematic diagram of the oral drug delivery device of Embodiment 37.

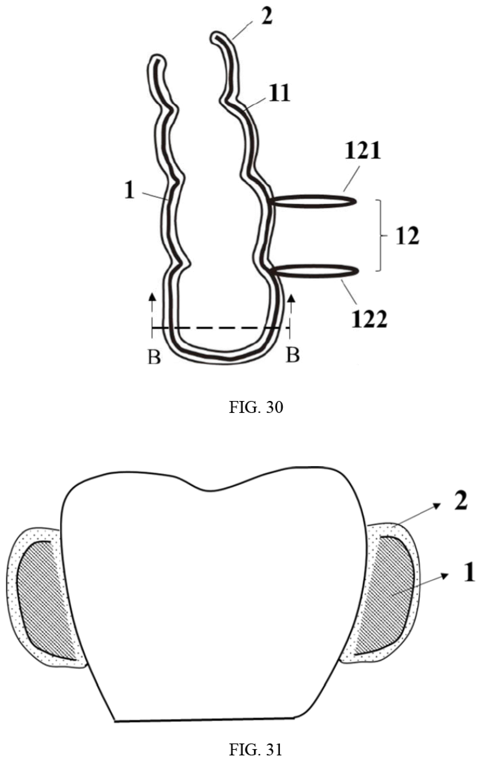

FIG. 30 is a schematic diagram of the oral drug delivery device of Embodiment 40.
FIG. 31 is a cross-sectional view taken along plane B-B of FIG. 30.

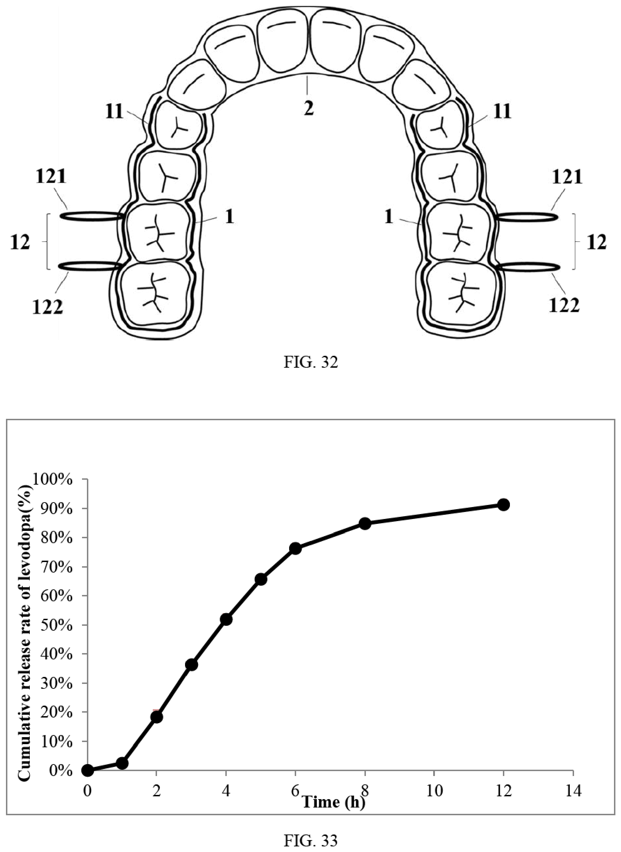

FIG. 32 is a schematic diagram of the oral drug delivery device of Embodiment 43.
   List of reference numerals:
   1-core component; 2-fixing component; 201-lingual side of the fixing component; 202-buccal side of the fixing component; 203-occlusal surface of the fixing component; 11-molar-fitting functional area; 12-drug-loaded functional area; 121-first ring; 122-second ring; 123-clamping wall.
FIG. 33 is the release profile of the osmotic pump tablet of Embodiment 47 of the present disclosure.

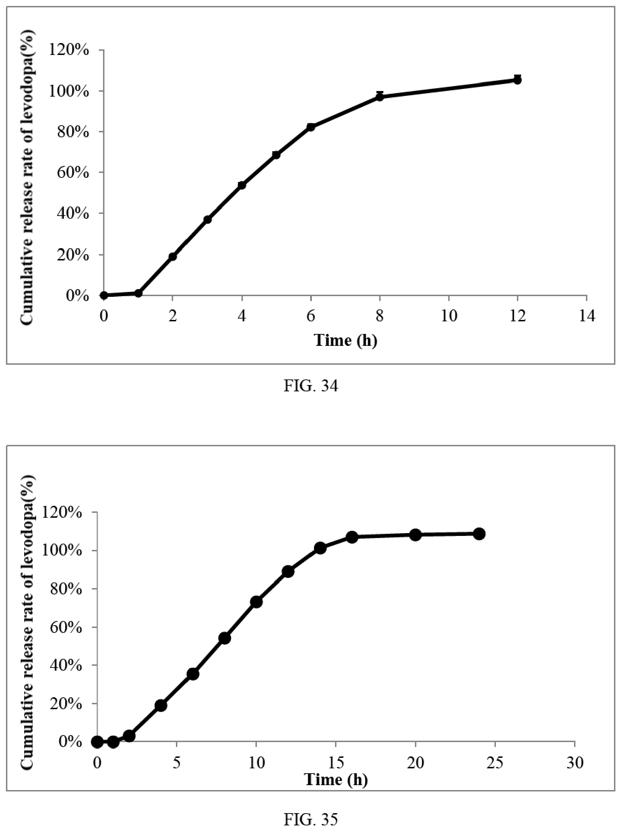

FIG. 34 is the release profile of the osmotic pump tablet of Embodiment 48 of the present disclosure.
FIG. 35 is the release profile of the osmotic pump tablet of Embodiment 49 of the present disclosure.

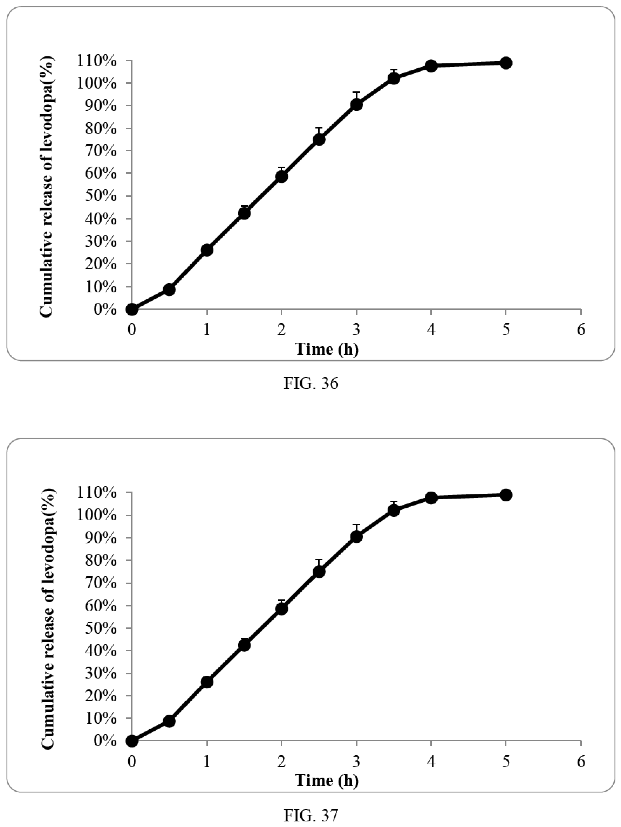

FIG. 36 is the release profile of the dosage form of Embodiment 50 of the present disclosure.
FIG. 37 is the release profile of the osmotic pump tablet of Embodiment 51 of the present disclosure.

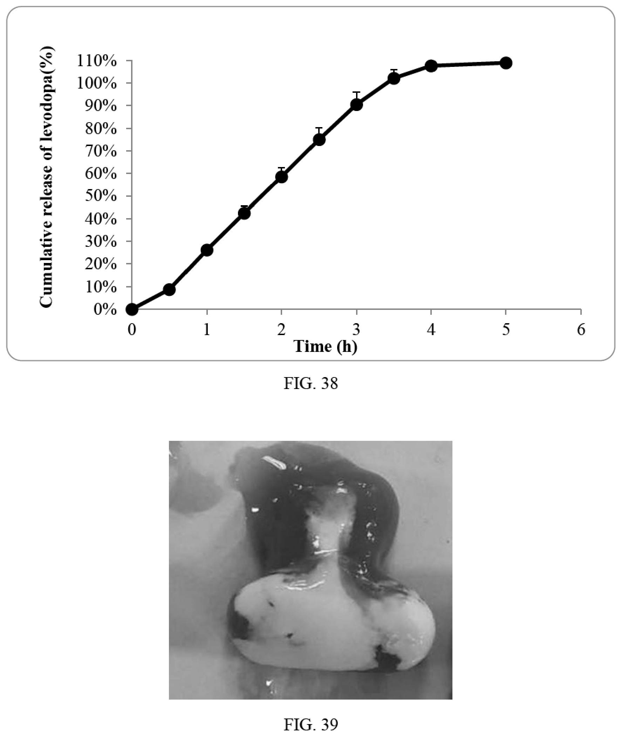

FIG. 38 is the release profile of the osmotic pump tablet of Embodiment 52 of the present disclosure.
FIG. 39 is the release profile of the osmotic pump tablet of Embodiment 57 of the present disclosure.

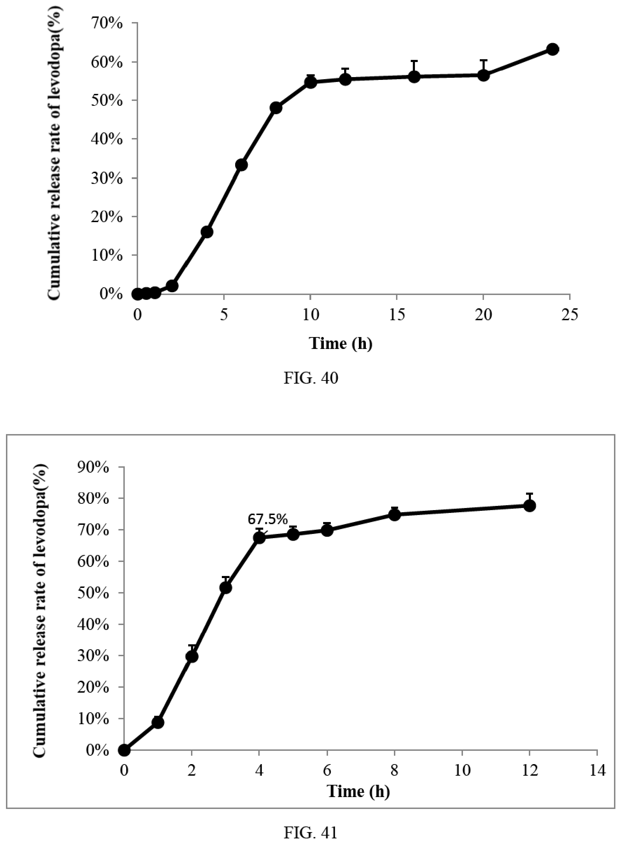

FIG. 40 is the release profile of the osmotic pump tablet of Embodiment 58 of the present disclosure.
FIG. 41 is the release profile of the osmotic pump tablet of Embodiment 59 of the present disclosure.

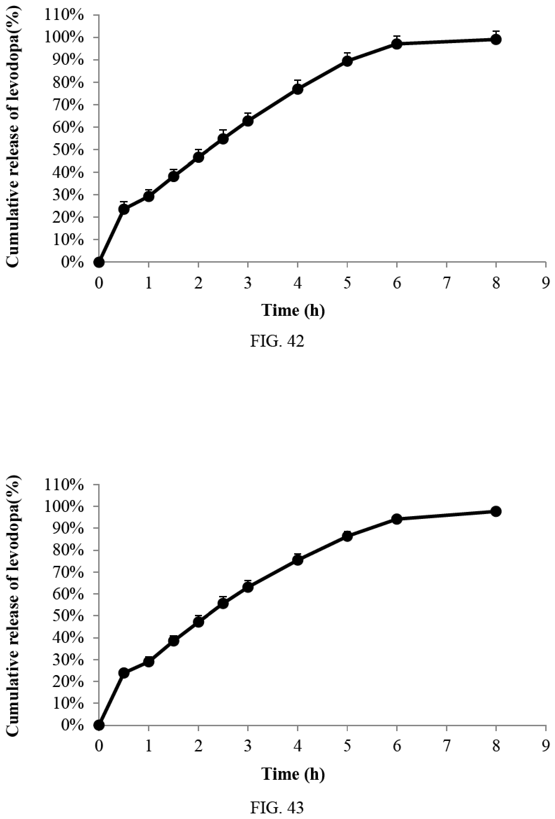

FIG. 42 is the release profile of the osmotic pump tablet of Embodiment 60 of the present disclosure.
FIG. 43 is the release profile of the osmotic pump tablet of Embodiment 61 of the present disclosure.

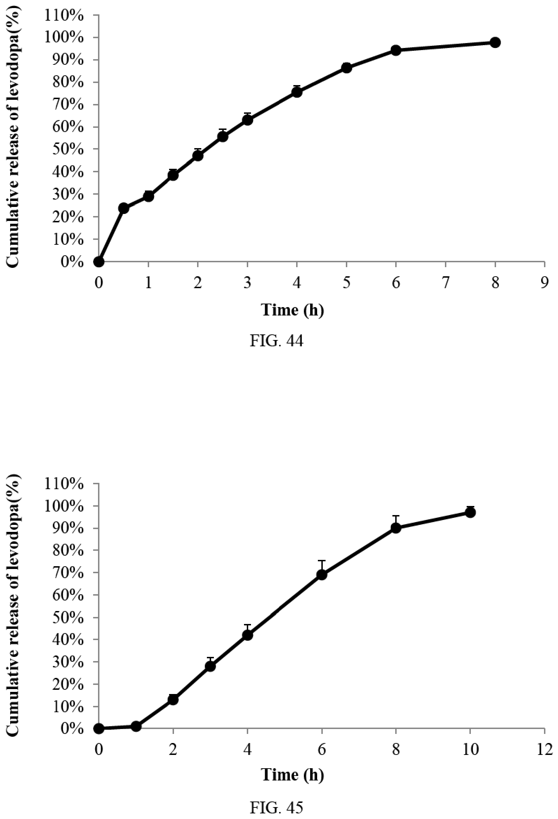

FIG. 44 is the release profile of the osmotic pump tablet of Embodiment 62 of the present disclosure.
FIG. 45 is the release profile of the osmotic pump tablet of Embodiment 63 of the present disclosure.

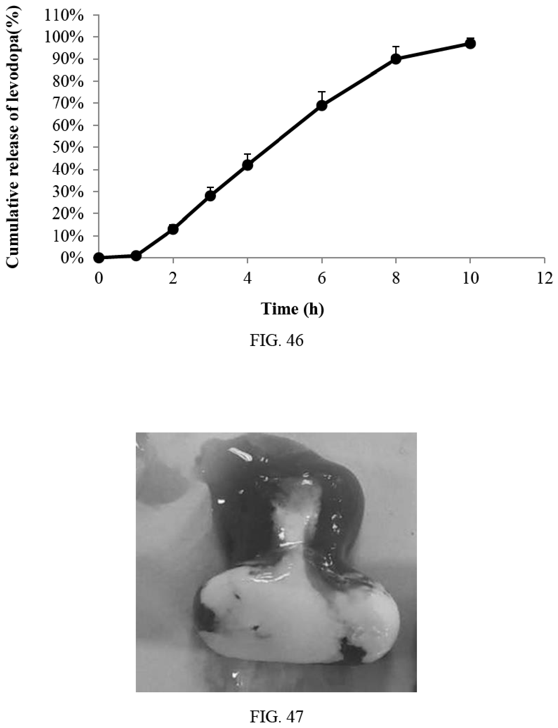

FIG. 46 is the release profile of the osmotic pump tablet of Embodiment 64 of the present disclosure.
FIG. 47 is the immersion experiment of the osmotic pump tablet of Comparative Embodiment A.

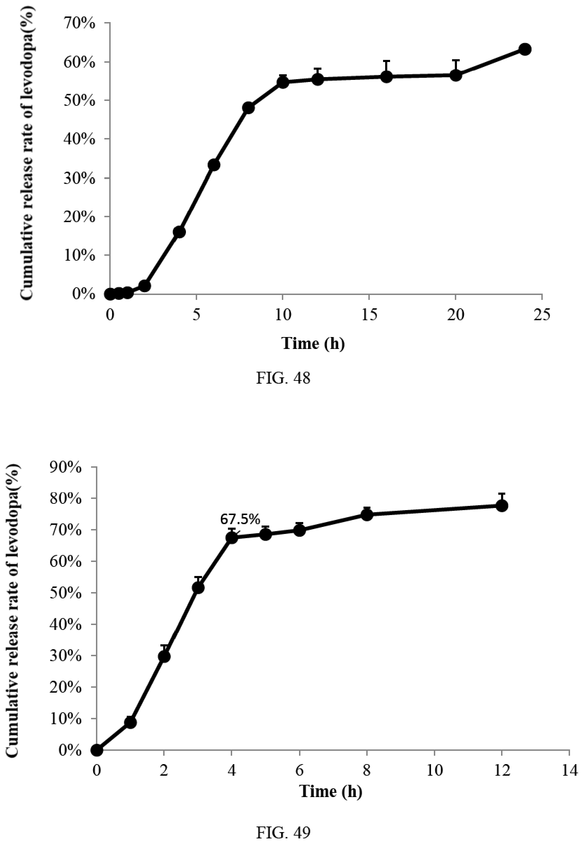

FIG. 48 is the release profile of the osmotic pump tablet of Comparative Embodiment A.
FIG. 49 is the release profile of the osmotic pump tablet of Comparative Embodiment B.

### DETAILED DESCRIPTION OF THE PREFERRED EMBODIMENT

The present disclosure is further illustrated by the following embodiments. However, the embodiments described herein are not intended to limit the scope of the present disclosure. In the following embodiments, where experimental conditions are not specified, standard procedures and conditions were applied, or the manufacturer's instructions were followed.

The preferred embodiments will be listed below, and the present disclosure will be illustrated more clearly and completely in conjunction with the drawings. It should be noted that unless otherwise specified, the relative arrangement and numerical values of the parts and steps set forth in these embodiments do not limit the scope of the present disclosure. The following embodiments further illustrate the present disclosure, but the present disclosure is not limited thereto. The experimental methods without specific conditions specified in the following embodiments were selected according to conventional methods and conditions, or according to product specifications. Active pharmaceutical ingredient(s) (APIs) used for the present disclosure include, but are not limited to, levodopa/carbidopa, baclofen, acyclovir, valaciclovir, ganciclovir, metformin and gabapentin.

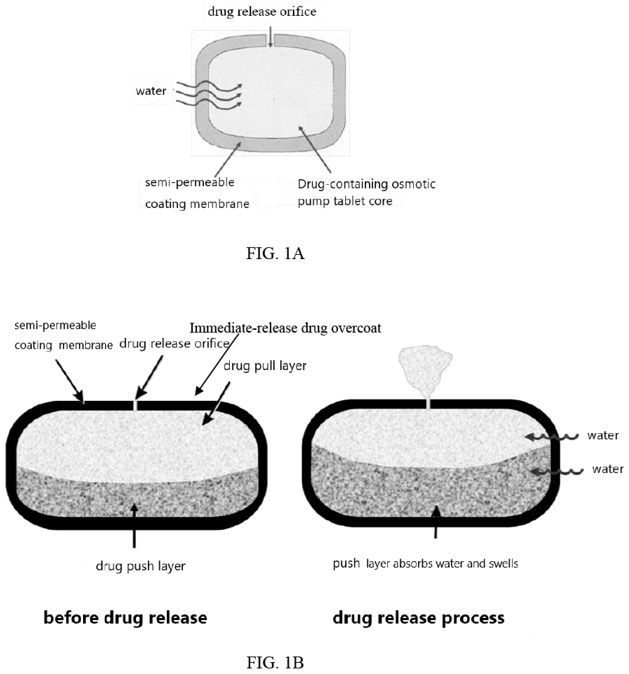

In one embodiment, the active pharmaceutical ingredient (API) is levodopa/carbidopa, which is incorporated in a single-layer elementary osmotic pump (EOP) known in the art (U.S. patents 3,845,770 and 3,916,899). As shown in FIG. 1A, EOP comprises an API tablet core and a rate control membrane encompassing the tablet core. The EOP comprises at least one orifice drilling through the membrane so that LD/CD can be released into the oral cavity through the orifice. The tablet core comprises LD/CD, osmotic agent, microcrystalline cellulose (MCC), binding agent, lubricant, flavoring agent (optional), acidifying agent (optional) and antioxidant (optional). The rate control membrane comprises a complete or at least a part of a semipermeable polymer that can penetrate water or moisture present in the oral cavity, while substantially impermeable to drugs and other optional components that can be present in the core. The representative semipermeable polymer is cellulose acetate with 32.0-39.8 wt% acetyl content.

In another embodiment, LD/CD is incorporated into a bi-layer (push-pull) osmotic delivery system known in the prior art (U.S. patents 4,327,725 and 4,612,008). As shown in FIG. 1B, the push-pull system is composed of two layers of core (including drug-containing pull layer and osmotic push layer) and a rate control membrane encompassing the core. The push-pull system comprises at least one orifice through the membrane on the side containing the drug-containing layer, so that the contents of the pull layer can be released into the oral cavity through the orifice. The pull layer comprises LD/CD, a hydrophilic polymer, an osmotic agent, a binding agent, a lubricant, a flavoring agent (optional), an acidifying agent (optional), and an antioxidant (optional). The push layer comprises a high molecular weight hydrophilic polymer, an osmotic agent, a binding agent, a lubricant, and a colorant (optional). The push-pull osmotic delivery system operates by absorbing water or moisture through a rate control membrane into a bi-layer core, wherein it hydrates the two layers, thereby expanding the osmotic push layer and push the hydrated, dispensable drug-containing layer formulation through orifice from the system.

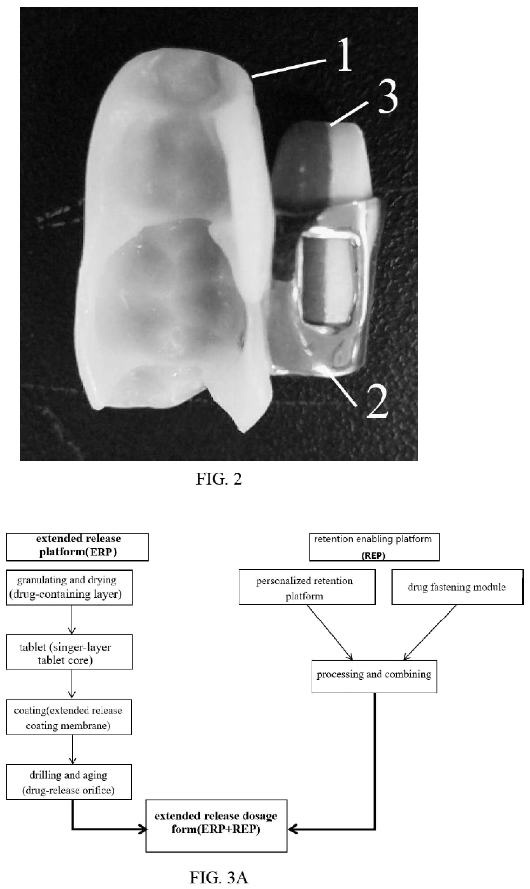

FIG. 2 is a schematic diagram of an extend release dosage form (a combination of an extended release platform ERP and a retention enabling platform REP). The retention enabling platform comprises a personalized retention enabling module and a drug fastened module. The retention enabling module can be personalized to match the corresponding teeth. The drug fastened module serves as a reservoir to fasten the extended release platform ERP for maintaining the ERP in a right place. The retention enabling platform REP personalized retention enabling module can match the second molar and anterior and posterior teeth. Due to the length of the retention enabling platform REP, it (the REP that maintains the ERP) is in a non-inhalable position, thus eliminating the possibility of blocking. The preferred structure of the REP is a buckle-type or nut-type basket with various mesh sizes so that the ERP does not fall out. Other structures of the REP can be, but are not limited to, a holder with two clamping arms.

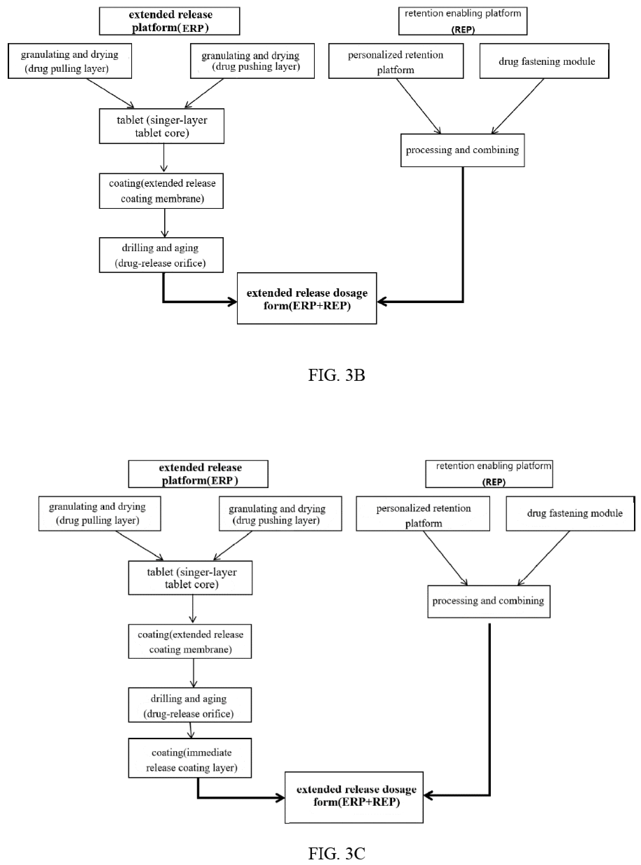

FIG. 3A-3C is a flow chart of manufacturing the extended release dosage form (ERP + REP) of the present disclosure. FIG. 3A shows an extended release dosage form for manufacturing an extended release platform as a single-layer elementary osmotic pump (EOP); FIG. 3B shows an extended release dosage form for manufacturing an extended release platform as a bi-layer push-pull osmotic system; FIG. 3B shows an extended release dosage form for manufacturing an extended release platform as a bi-layer push-pull system with an immediate release drug overcoat.

The retention enabling module REP can be made of polymer materials or dental titanium using 3D printing technology, injection molding processes or impression molding. Polymer materials include, but are not limited to, polycaprolactone (PCL), ethylene-vinyl acetate copolymer (EVA), high density polyethylene (HDPE), polypropylene (PP), polyacrylate and any other tissue compatible polymers. PCL is a good material because of its low melting point and good biocompatibility. Therefore, the personalized retention enabling module of REP made by PCL is easily softened in hot water, and then matches the molar or premolar.

One of the ERP in the present disclosure is a single-layer elementary osmotic pump, and can be manufactured by standard manufacturing techniques. First of all, in the conventional wet granulation method, a high-shear granulator or a fluidized bed granulator can be used to prepare tablet core granules. In the second step, the granules are pressed into a single-layer tablet core in the press. Next, the tablet core is coated with a coating membrane coating composition. Finally, an orifice was drilled through the coating membrane.

Another ERP in the present disclosure is a bi-layer push-pull osmotic pump, and can be manufactured as follows. First of all, a high-shear granulator or fluidized bed granulator can be used to prepare granules of drug-containing layer and osmotic push layer. Secondly, the granules of these two layers are pressed into a bi-layer tablet core in a press. Next, the bi-layer tablet core is coated with a coating membrane coating composition, followed by a drying process. Finally, an orifice was drilled through the membrane of drug-containing layer.

Coating membranes of osmotic dosage forms can be formed by air suspension technology. The method comprises suspending and tumbling a single-layer tablet core or a bi-layer tablet core in an air flow and coating composition until the membrane is homogenously formed around the core. The air suspension process can be realized by the fluidized bed granulator with a Wurster plug-in. Acetone or acetone ethanol mixed solubilizer can be used as coating solvent, in which 2.0-5 wt% of the membrane-forming composition is dissolved. Other membrane forming techniques, such as pan coating, can also be used. In the pan coating system, the membrane is continuously sprayed onto the tablet core of the rotary pan and deposited into a membrane composition. Generally, the membrane formed by these techniques with a thickness of 25 to 250 µm, preferably 100 to 150 µm.

Optionally, a single-layer EOP or bi-layer push-pull system with drilled orifice can be coated with an immediate release LD/CD drug-containing layer.

The personalized retention enabling platform (REP) in the present disclosure can be manufactured as follows. REP was prepared by 3D printing technology. First, an oral image of an individual was obtained by an oral scanner. Next, the CAD/CAM software was used to design the REP, which was the holder of the ERP. Then, according to CAD/CAM design, 3D printing technology or injection molding process was used to make REP from histocompatible polymer or dental titanium, or cobalt chromium alloy, or cobalt chromium molybdenum alloy.

The personalized retention enabling platform (REP) in the present disclosure can also be manufactured by injection molding technology. First, the dental impression material was used to make a mold to prepare the same plaster tooth model as the patient; secondly, REP shape of dental wax was prepared on plaster dental model by conventional process; then, the embedded powder and water mixture were used to wrap the dental wax REP, and the embedded dental wax was heated to melt after standing and curing to obtain a REP-shaped injection mold cavity; finally, at a certain temperature, the completely molten cobalt-chromium alloy was poured into the mold cavity, cooled and cured, and then polished.

The personalized retention enabling platform (REP) in the present disclosure can also be manufactured by impression molding technology. Firstly, a drug fastened module was manufactured, and then, an oval thermoplastic sheet was processed by a conventional process. Then, the thermoplastic sheet was heated to soften it, and the softened thermoplastic sheet was pressed to completely cover the teeth to form a personalized retention enabling module that is completely fitted to the teeth. The drug fastened module was then quickly embedded on the uncured retention enabling module and cooled and solidify to form a personalized retention enabling platform (REP). Wait for the personalized retention enabling platform to cool and restore the original opaque hard membrane state, and remove the cooled personalized retention enabling platform from the oral cavity.

### Embodiment 1 Preparation of retention enabling platform (impression molding)

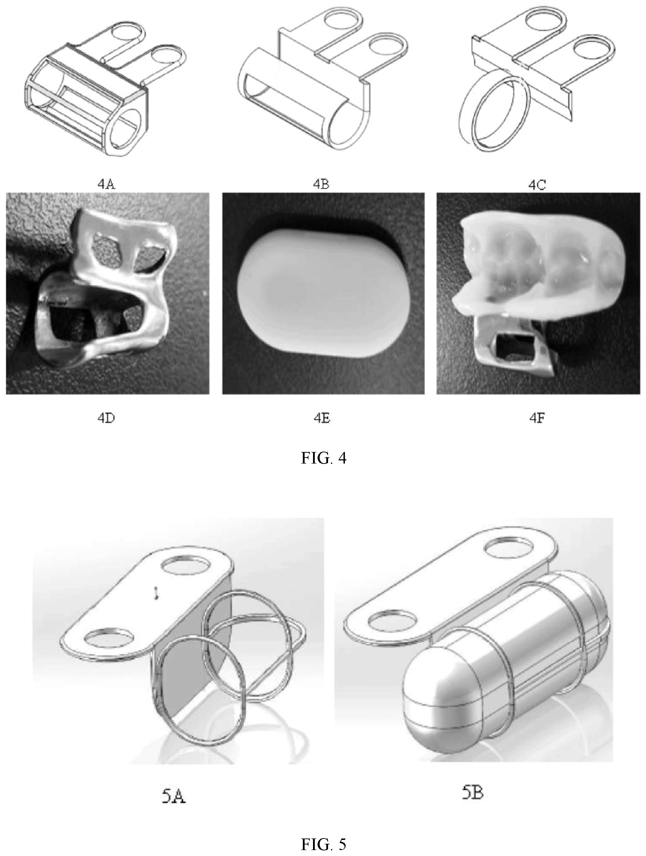

1. Drug fastened modules as shown in FIG. 4A, 4B, 4C and 4D, which is suitable for the extended release platform, are prepared. The drug fastened modules is made of stainless steel, dental titanium, cobalt chromium alloy, or cobalt chromium molybdenum alloy, and the shape of which can be one or more reservoirs with one or both ends open. The shape of the cross section of the reservoir can be polygon, circular closed ring or open ring with the opening smaller than the minimum diameter of the tablet.
2. 50g polycaprolactone iss processed into an oval thermoplastic sheet with a size of 2.5cm×1.5cm by conventional process (as shown in FIG. 4E);
3. Preparation of personalized REP: a piece of thermoplastic sheet is heated in hot water at about 70 °C to soften it with good moldability. When the thermoplastic sheet becomes translucent (about 1 minute), take it out and put it on the teeth, and press the softened thermoplastic sheet to completely wrap the teeth to form a retention enabling module that completely fitted to the teeth. Then, the stainless-steel drug fastened module is quickly embedded on the uncured retention enabling module, and cooled and cured to form a personalized retention enabling platform (REP). Some water can be briefly sprayed to further accelerate the cooling, then wait for the personalized retention enabling platform to cool and restore the original opaque hard sheet state, and the cooled personalized retention enabling platform is taken out the from the oral cavity (as shown in FIG. 4F).

FIG. 5 is the aforementioned drug fastened module: FIG.5A. the drug fastened module; FIG.5B. the extended release platform was fastened in the drug fastened module.

### Embodiment 2 Preparation of retention enabling platform (injection molding)

According to the following procedures, a retention enabling platform that fitted to the mandibular molar teeth is prepared by an injection molding process, as shown in FIG. 6.

First, a plaster dental model with the same dental properties as a patient is prepared by using a dental impression material to form a mold;
REP shape of dental wax is prepared on a plaster dental model by conventional process using dental wax;
The above dental wax REP is then wrapped with a mixture of embedding powder and water, and after standing and curing, the embedded dental wax is melted to obtain an injection mold cavity in the shape of REP;
At a certain temperature, the completely melted cobalt chromium alloy is poured into the mold cavity, cooled and cured, and then polished.

### Embodiment 3 Preparation of retention enabling platform (3D printing)

According to the following procedures, a retention enabling platform that fitted to the mandibular molar teeth is prepared by oral scanning and 3D printing processes, as shown in FIG. 6.

Firstly, an individual oral image is obtained by the oral scanner;
CAD/CAM software or dental software is used to process scanning data, generate electronic tooth model, transform the date into editable file, and design a personalized retention enabling platform device that completely fitted to the teeth;
Cobalt chromium molybdenum 3D printing material is used to print the designed personalized device by 3D printer laser sintering technology.

### Embodiment 4 Preparation of retention enabling platform (injection molding)

According to the following procedures, the retention enabling platform that fitted to the periphery of the non-occlusal surface of the mandibular molar is prepared by injection molding process, as shown in FIG. 7.

First, a plaster dental model with the same dental properties as a patient is prepared by using a dental impression material to form a mold;
REP shape of dental wax is prepared on a plaster dental model by conventional process using dental wax;
The above dental wax REP is then wrapped with a mixture of embedding powder and water, and after standing and curing, the embedded dental wax is melted to obtain an injection mold cavity in the shape of REP;
At a certain temperature, the completely melted cobalt chromium alloy is poured into the mold cavity, cooled and cured, and then polished.

### Embodiment 5 Preparation of retention enabling platform (3D printing)

According to the following procedures, a retention enabling platform that fitted to the periphery of the non-occlusal surface of the mandibular molar is prepared by oral scanning and 3D printing processes, as shown in FIG. 7.

Firstly, an individual oral image is obtained by the oral scanner;
CAD/CAM software or dental software is used to process scanning data, generate electronic tooth model, transform the date into editable file, and design a personalized retention enabling platform device that completely fitted to the teeth;
Cobalt chromium molybdenum 3D printing material is used to print the designed personalized device by 3D printer laser sintering technology.

### Embodiment 6 Preparation of extended release platform

A dosage form for distributing the beneficial drugs levodopa and carbidopa to oral cavity is manufactured as follows: first, a tablet core is prepared, comprising, in weight percentage, 40.0 wt% of levodopa (LD), 10.8 wt% of carbidopa monohydrate (CD), 20.0 wt% of microcrystalline cellulose, 18.7 wt% of mannitol, 5.0 wt% of hydroxypropyl methylcellulose (HPMC E5) and 5.0 wt% of citric acid that are each passed through a 40-mesh stainless steel sieve, then blended and granulated with pure water until homogeneous wet mass is formed; the wet mass is passed through a 20-mesh stainless steel sieve and dried at 80 °C for 2 hours; the dried granules are passed through an 18-mesh stainless steel sieve and then mixed with 0.5 wt% of magnesium stearate.

Then, 500 mg of the drug core granules are compressed into a single-layer tablet core with a 9.0 mm round punch using a tablet press.

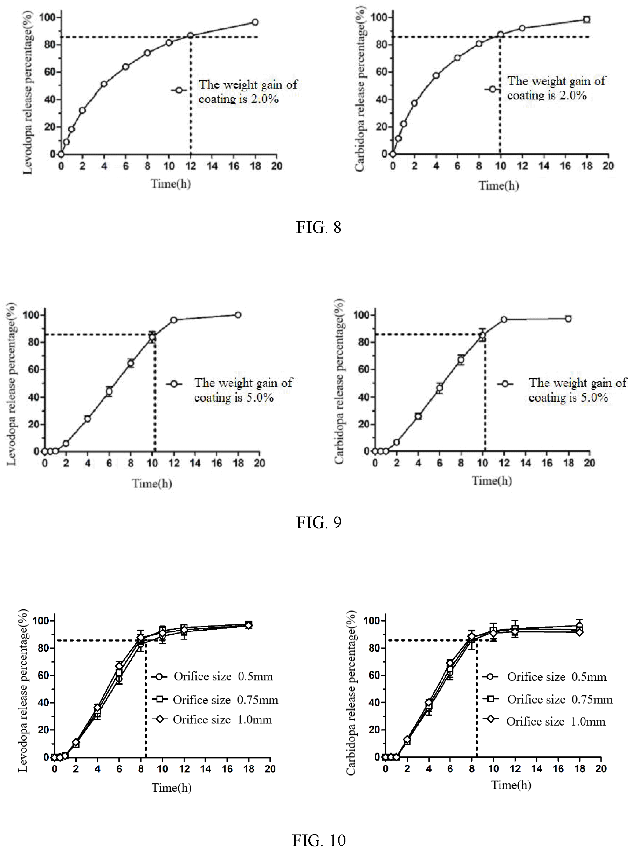

Next, the single-layer tablet core is coated with a coating membrane. The membrane-forming composition comprises, in weight percentage, 50 wt% of cellulose acetate and 50 wt% of Copovidone VA64. The membrane-forming composition is mixed with acetone to make a 4% of solid suspension. Using the process parameters listed in the table below, the membrane-forming composition is sprayed onto the tablet cores in a Glatt GC 1 pan coater to form a coating membrane (see Table 1 for coating process parameters). The membrane weight gain of the coated tablet is 2.0%. Finally, a 0.5 mm exit orifice is mechanically drilled on the drug-containing layer side of the coated tablet. Residual solvents are removed by drying the dosage form at 40 °C and ambient humidity for 24 hours. The release profile of the final manufactured dosage form is measured in 0.1N HCl aqueous solution using USP I pulp board. As shown in FIG. 8, the final manufactured ERP dosage form delivers LD and CD at an average rate of 14.17 mg/hr and 4.59 mg/hr, with 85% of the drugs delivered in 12 and 10 hours, respectively. The ERP osmotic delivery system can be kept in oral cavity until the push-layer reaches the delivery orifice, or kept there for 8-9 hours, and then swallowed; or it can be fasten on the corresponding teeth in the oral cavity after being combined with the retention enabling platform REP and kept there for 12 hours.

**Table 1**

| parameters of coating process | |
|---|---|
| Inlet temperature (°C) | 20-40 |
| Exhaust temperature (°C) | 14-25 |
| Air flow rate (m³/h) | 20-40 |
| Fluid delivery rate (g/min) | 5-25 |
| Atomizing air pressure (bar) | 0.6-0.8 |
| Pattern air pressure (bar) | 0.6-0.8 |
| Rotating speed (rpm) | 6-15 |
| Batch size (g) | 400 |

### Embodiment 7 Preparation of extended release platform

A dosage form designed, shaped and adapted for dispensing the beneficial drugs levodopa and carbidopa monohydrate to oral cavity is manufactured as follows: first, a drug-containing layer composition is prepared, comprising, in weight percentage, 40.0 wt% of LD, 10.8 wt% of CD, 31.0 wt% of hydroxypropyl cellulose having a weight average molecular weight of 80,000, 12.7 wt% of mannitol and 5.0 wt% of citric acid, these excipients are each pass through a 40-mesh stainless steel sieve, then blended and granulated with 95% ethanol until homogeneous wet mass is formed; the wet mass is passed through a 20-mesh stainless steel sieve and dried at 80 °C for 2 hours; the dried granules are passed through an 18-mesh stainless steel sieve and then mixed with 0.5 wt% of magnesium stearate.

Next, a second composition, the osmotic layer, is prepared, comprising 55.0 wt% of sodium carboxymethyl cellulose 7H4XF, 39.0 wt% of sorbitol, 5.0 wt% of Povidone K30 and 0.5 wt% of iron oxide red; these components are each passed through a 40-mesh stainless steel sieve, then blended and granulated with 95% ethanol until homogeneous wet mass is formed; the wet mass is passed through a 20-mesh stainless steel sieve and dried at 80 °C for 2 hours; dried granules are passed through a 18-mesh stainless steel sieve and then mixed with 0.5 wt% of magnesium stearate.

Next, the drug-containing layer and the osmotic layer granules are pressed into a bi-layer tablet core. First, 500 mg of drug-containing layer granules are added to a 9 mm round punch of a tablet press and tamped, then 250 mg of osmotic layer granules are added to the punch, and the granules of both layers are pressed with a tablet press into a contacting bi-layer tablet core.

Next, the bi-layer tablet core is coated with a coating membrane. The membrane-forming composition comprises, in weight percentage, 70 wt% of cellulose acetate with 39.8 wt% of acetyl content and 30 wt% of copovidone VA64. The membrane-forming composition is mixed with acetone to form a 4% of solid suspension. Using the process parameters listed in embodiment 6, the membrane-forming composition is sprayed onto the bi-layer tablet cores in a Glatt GC 1 pan coater to form a coating membrane, and the membrane weight gain of the coated tablet is 5.0%. Finally, a 0.5 mm exit orifice is drilled mechanically on the drug-containing layer side of the dosage form. Residual solvents are removed by drying the dosage form at 40 °C and ambient humidity for 24 hours. The release profile of the final manufactured dosage form is measured using a USP I paddle method in an aqueous solution of 0.1N HCl. The final manufactured dosage form delivers LD and CD at an average rate of 17.0 mg/hr and 4.6 mg/hr, respectively, with 85% of LD/CD delivered in 10.0 hours. FIG. 9 depicts the consistent release profiles for both LD and CD. The osmotic delivery system can be kept in oral cavity until the osmotic layer reached the delivery orifice, or kept there for 6-7 hours, and then is swallowed, or it is fastened on the corresponding teeth in the oral cavity and maintained there for 10 hours after combination with the retention enabling platform REP.

### Embodiment 8 Preparation of extended release platform

In this embodiment, the procedures of Embodiment 7 are repeated, and the dosage form consisted of the drug-containing layer, osmotic layer, and coating membrane is identical to those provided in Embodiment 7. In this example, the membrane weight gain is 4.0%, and the size of the delivery orifice varied from 0.5 mm, 0.75 mm, to 1.0 mm. The final manufactured dosage form delivers LD and CD at an average rate of 21.3 mg/hr and 5.7 mg/hr, respectively, with 85% of LD/CD delivered in 8.0 hours. As shown in FIG. 10, the size of the delivery orifice has no significant impact on the release profile. The osmotic delivery system can be kept in oral cavity until the osmotic layer reached the delivery orifice, or kept there for 4-5 hours, and then is swallowed, or it is fastened on the corresponding teeth in the oral cavity and maintained there for 8 hours after combination with the retention enabling platform REP.

### Embodiment 9 Preparation of extended release platform

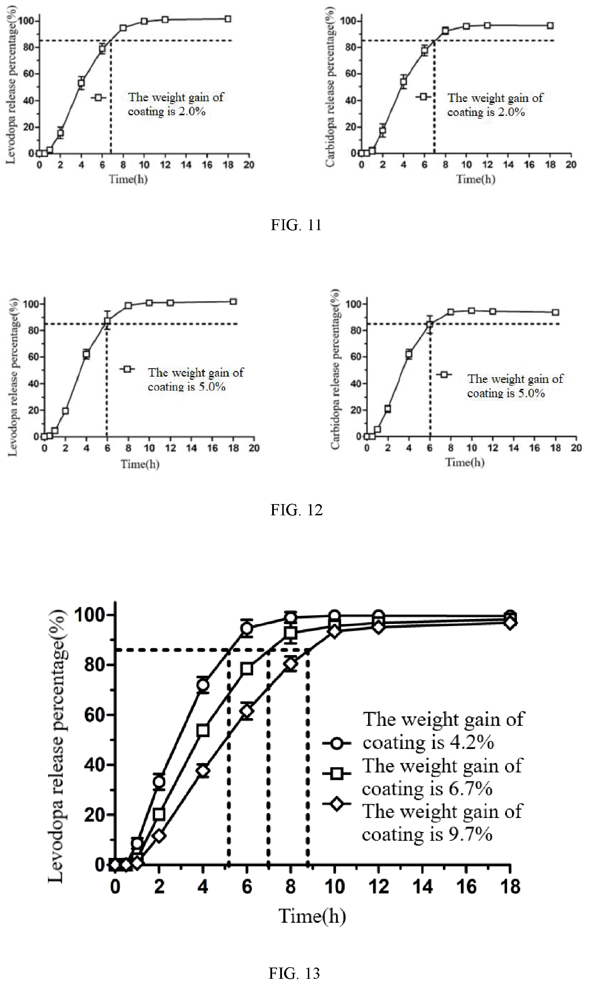

In this embodiment, the procedures of Embodiment 7 are repeated, and the dosage form consisted of the drug-containing layer is identical to those provided in Embodiment 7, while the osmotic layer comprises sodium carboxymethyl cellulose 9H4XF instead of 7H4XF. In this Embodiment, the membrane-forming composition and the size of the delivery orifice are also identical to those in Embodiment 6. The coating membrane weight gain of the dosage form is 2.0%. As shown in FIG. 11, the dosage form deliver LD and CD at an average rate of 24.3 mg/hr and 6.6 mg/hr, respectively, with 85% of LD/CD delivered in 7.0 hours. The osmotic delivery system can be kept in oral cavity until the osmotic layer reached the delivery orifice, or kept there for 3-4 hours, and then is swallowed, or it was fastened on the corresponding teeth in the oral cavity and maintained there for 7 hours after combination with the retention enabling platform REP.

### Embodiment 10 Preparation of extended release platform

In this embodiment, the procedures of Embodiment 7 are repeated, and the dosage form consisted of the drug-containing layer and osmotic layer is identical to those provided in Embodiment 7, while the membrane-forming composition comprises 60 wt% of cellulose acetate of cellulose acetate with an acetyl content of 39.8% and 40 wt% of copovidone VA64. The membrane weight gain is 5.0%. As shown in FIG. 12, the dosage form delivers 85% of LD/CD in 6 hours. The osmotic delivery system can be kept in oral cavity until the osmotic layer reached the delivery orifice, or kept there for 2-3 hours, and then is swallowed, or it was fastened on the corresponding teeth in the oral cavity and maintained there for 6 hours after combination with the retention enabling platform REP.

### Embodiment 11 Preparation of extended release platform

In this embodiment, the procedures of embodiment 7 are repeated to provide a dosage form.

In this embodiment, the drug-containing layer comprises 45.0 wt% of LD, 31.0 wt% of hydroxypropyl cellulose (Klucel EXF), 16.0 wt% of mannitol, 5.0 wt% of Povidone K30, 1.0 wt% of aspartame, 1.0 wt% of Mint flavor and 1.0 wt% of magnesium stearate. The osmotic layer comprises 55 wt% of sodium carboxymethyl cellulose 7H4XF, 34.0 wt% of sorbitol, 10.0 wt% of Povidone K30 and 0.5 wt% of iron oxide red and 0.5 wt% of magnesium stearate.

The drug-containing layer (500mg) and osmotic layer granules (250mg) are compressed into a bi-layer tablet core using a 16×7 capsule-shape tooling.

The bi-layer tablet core is wrapped with the coating membrane, and the weight gains are 4.2 wt%, 6.7 wt% and 9.7 wt%, respectively. The membrane-forming composition comprises 60 wt% of cellulose acetate having an acetyl content of 39.8%, 40 wt% of Copovidone VA64. A 1.0 mm exit orifice is drilled mechanically on the drug-containing layer side of the dosage form. Residual solvents are removed by drying the dosage form at 40 °C and ambient humidity for 24 hours.

As shown in FIG. 13, the dosage form delivers LD at an average rate of 38.3 mg/hr, 27.3 mg/hr, and 21.3 mg/hr, and the membrane weight gains are 4.2%, 6.7%, and 9.7%, respectively, with 85% of LD delivered in 5.0 hours, 7.0 hours and 9.0 hours.

### Embodiment 12 Preparation of extended release platform

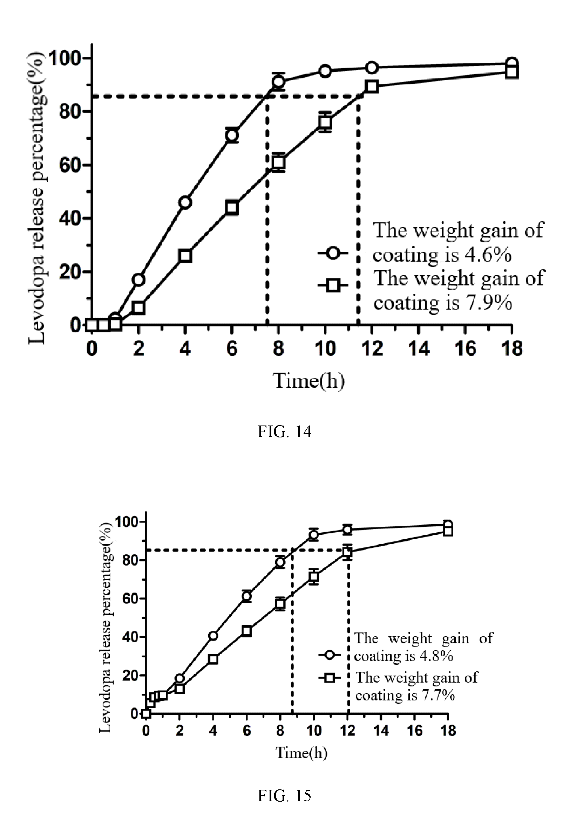

In this embodiment, the procedures of embodiment 11 are repeated to provide a dosage form other than a membrane-forming composition. In this embodiment, the bi-layer tablet core comprises a membrane-forming composition comprising 70 wt% of cellulose acetate with 39.8% acetyl content and 30 wt% of copovidone VA 64 in weight percentage. The membrane-forming composition is dissolved in a mixed solvent comprising 90% acetone, 9.0% ethanol and 1.0% deionized water to produce a 4% of solid suspension. As shown in FIG. 14, the dosage forms with membrane weight gain of 4.6 wt% and 7.9 wt% delivers LD at an average rate of 25.5 mg/hr and 16.9 mg/hr, respectively, and correspondingly with 85% of LD delivered at 7.5 hours and 11.5 hours.

### Embodiment 13 Preparation of extended release platform

In this embodiment, the procedures of embodiment 12 are repeated to provide a dosage form. In this embodiment, an immediate release composition comprising 23.78 wt% of LD, 64.22 wt% of CD, 10 wt% of hydroxypropyl cellulose, 1 wt% of aspartame and 1 wt% of mint flavor is used to overcoat the dried dosage form with 4.8% and 7.7% membrane weight gain (as shown in FIG. 3C). The immediate release overcoat composition is mixed with ethanol to form a 6.7% solid suspension. The final dosage form comprises an immediate-release coating comprising 62.5 mg of CD and 25 mg of LD, and a controlled-release drug-containing layer comprising 225 mg of LD. As shown in FIG. 15, the release profile of the dosage form shows rapid release of LD/CD, followed by an extended release with a release duration of approximately 8.5 hours and 12.0 hours, respectively. The osmotic delivery system having a membrane weight gain of 4.8% can be kept in oral cavity for 4-5 hours, and then kept in oral cavity at meal time or for the whole release duration. The osmotic delivery system having a membrane weight gain of 7.7% can be kept in oral cavity for 8-9 hours before swallowed, or kept in oral cavity for the whole release duration, or fastened on the corresponding teeth in the oral cavity after being combined with the retention enabling platform REP and maintained there for 12 hours.

### Embodiment 14 Preparation of extended release platform

In this embodiment, the procedures of Embodiment 6 are repeated, and the dosage form comprises a drug-containing layer and a membrane-forming composition are identical to those provided in Embodiment 6. The drug-containing layer comprises, in weight percentage, 38.0 wt% of levodopa, 50.0 wt% of microcrystalline cellulose, 2.0 wt% of magnesium stearate and 10.0 wt% of hydroxypropyl methylcellulose. The coating membrane comprises 50 wt% of acetyl acetate membrane with an acetyl content of 39.8 wt% and 50 wt% of Copovidone VA64. In this embodiment, the membrane weight gain is 4.50%. The final manufactured dosage form delivers levodopa at an average rate of 9.4 mg/hr, with 85% of levodopa delivered in 9.0 hours.

### Embodiment 15 Preparation of extended release platform

In this embodiment, the procedures of Embodiment 6 are repeated, and the dosage form comprises a drug-containing layer and a membrane-forming composition are identical to those provided in Embodiment 6. The drug-containing layer comprises, in weight percentage, 19.5 wt% of levodopa, 20.0 wt% of carbidopa, 50.0 wt% of mannitol and 10.0 wt% of citric acid. The coating membrane comprises 50 wt% of acetyl acetate membrane with an acetyl content of 39.8 wt% and 50 wt% of Copovidone VA64. In this embodiment, the membrane weight gain is 4.50%. The final manufactured dosage form delivers levodopa at an average rate of 22.9 mg/hr, with 85% of levodopa delivered in 13.0 hours.

### Embodiment 16 Preparation of extended release platform

In this embodiment, the procedures of Embodiment 7 are repeated, and the dosage form comprises a drug-containing layer, an osmotic layer and a membrane-forming composition are identical to those provided in Embodiment 7. The drug-containing layer comprises, in weight percentage, 70.0 wt% of levodopa, 9.0 wt% of mannitol, 20.0 wt% of Povidone K30 and 1.0 wt% of magnesium stearate. The osmotic layer comprises, in weight percentage, 85.0 wt% of sodium carboxymethyl cellulose (7H4XF), 3.0 wt% of Povidone K30, 5.0 wt% of sorbitol, 5.0 wt% of iron oxide red and 2.0 wt% of magnesium stearate. The coating membrane comprises, in weight percentage, 70 wt% of acetyl acetate membrane with an acetyl content of 39.8 wt% and 30 wt% of Copovidone VA64. In this embodiment, the membrane weight gain is 4.5%. The final manufactured dosage form delivers levodopa at an average rate of 35.0 mg/hr, with 85% of levodopa delivered in 8.5 hours.

### Embodiment 17 Preparation of extended release platform

In this embodiment, the procedures of Embodiment 7 are repeated, and the dosage form comprises a drug-containing layer, an osmotic layer and a membrane-forming composition are identical to those provided in Embodiment 7. The drug-containing layer comprises, in weight percentage, 20.0 wt% of levodopa, 20.0 wt% of carbidopa, 50.0 wt% of hydroxypropyl cellulose, 4.0 wt% of mannitol, 5.0 wt% of aspartame and 1.0 wt% of magnesium stearate. The osmotic layer comprises, in weight percentage, 25.0 wt% of sodium carboxymethyl cellulose (7H4XF), 9.5 wt% of Povidone K30, 65.0 wt% of sorbitol and 0.5 wt% of magnesium stearate. The coating membrane comprises, in weight percentage, 90 wt% of acetyl acetate membrane with an acetyl content of 39.8 wt% and 10 wt% of Copovidone VA64. In this embodiment, the membrane weight gain is 4.5%. The final manufactured dosage form delivers levodopa and CD at an average rate of 7.1 mg/hr, with 85% of levodopa/CD delivered in 12 hours.

### Embodiment 18 Preparation of extended release platform

In this embodiment, the procedures of Embodiment 13 are repeated, and the dosage form comprises a drug-containing layer, an osmotic layer, a membrane-forming composition and an overcoat are identical to those provided in Embodiment 13. The drug-containing layer comprises, in weight percentage, 45.0 wt% of levodopa, 31.0 wt% of hydroxypropyl cellulose, 17.0 wt% of mannitol, 5.0 wt% of Povidone K30, 1.0 wt% of magnesium stearate and 1.0 wt% of aspartame. The osmotic layer comprises, in weight percentage, 60.0 wt% of sodium carboxymethyl cellulose (7H4XF), 10.0 wt% of Povidone K30, 26.0 wt% of sorbitol, 2.0 wt% of iron oxide red and 2.0 wt% of magnesium stearate. The coating membrane comprises, in weight percentage, 70 wt% of acetyl acetate membrane with an acetyl content of 39.8 wt% and 30 wt% of Copovidone VA64. The weight of the coating membrane is 4.5% of the mass of the tablet core. The immediate-release overcoat comprises, in weight percentage, 93.0 wt% of CD, 2.0 wt% of hydroxypropyl cellulose and 5.0 wt% of aspartame; the mass of the overcoat is 13.2% of the mass of the tablet core (table core + first layer coating membrane comprising cellulose acetate and Copovidone VA64). The immediate-release overcoat of the dosage form is first released rapidly, followed by an extended release with a release duration of approximately 8 hours. The osmotic delivery system can be kept in oral cavity for 4-5 hours and then swallowed before meal time or kept in oral cavity for the whole release duration, or fastened on the corresponding teeth in the oral cavity after being combined with the retention enabling platform REP and maintained there for 8 hours.

### Embodiment 19 Preparation of extended release platform

In this embodiment, the procedures of Embodiment 13 are repeated, and the dosage form comprises a drug-containing layer, an osmotic layer, a membrane-forming composition and an overcoat are identical to those provided in Embodiment 13. The drug-containing layer comprises, in weight percentage, 45.0 wt% of levodopa, 31.0 wt% of hydroxypropyl cellulose, 12.0 wt% of mannitol, 5.0 wt% of Povidone K30, 5.0 wt% of mint flavor, 1.0 wt% of magnesium stearate and 1.0 wt% of aspartame. The osmotic layer comprises, in weight percentage, 40.0 wt% of sodium carboxymethyl cellulose (7H4XF), 20.0 wt% of Povidone K30, 36.0 wt% of sorbitol, 3.5 wt% of iron oxide red and 0.5 wt% of magnesium stearate. The coating membrane comprises, in weight percentage, 70 wt% of acetyl acetate membrane with an acetyl content of 39.8 wt% and 30 wt% of Copovidone VA64. The weight of the coating membrane is 4.5% of the mass of the tablet core. The immediate-release overcoat comprises, in weight percentage, 75.0 wt% of LD, 20.0 wt% of hydroxypropyl cellulose and 5.0 wt% mint flavor; the mass of the overcoat is 13.2% of the mass of the tablet core (table core + first layer coating membrane comprising cellulose acetate and Copovidone VA64). The immediate release overcoat of the dosage form is rapidly released first, followed by sustained releasing for approximately 8 hours. The osmotic delivery system can be kept in oral cavity for 4-5 hours and then swallowed before meal time or kept in oral cavity for the whole release duration, or fastened on the corresponding teeth in the oral cavity after being combined with the retention enabling platform REP and maintained there for 8 hours.

### Embodiment 20 Preparation of extended release platform

In this embodiment, the procedures of Embodiment 8 are repeated, and the dosage form comprises a drug-containing layer, an osmotic layer, a membrane-forming composition and an overcoat are identical to those provided in Embodiment 8. The drug-containing layer comprises, in weight percentage, 45.0 wt% of levodopa, 31.0 wt% of hydroxypropyl cellulose, 17.0 wt% of mannitol, 5.0 wt% of Povidone K30, 1.0 wt% magnesium stearate and 1.0 wt% of aspartame. The osmotic layer comprises, in weight percentage, 55.0 wt% of sodium carboxymethyl cellulose (7H4XF), 10.0 wt% of hydroxypropyl cellulose, 34.0 wt% sorbitol, 0.5 wt% of iron oxide red and 0.5 wt% of magnesium stearate. The coating membrane comprises, in weight percentage, 70 wt% of acetyl acetate membrane with an acetyl content of 39.8 wt% and 30 wt% of Copovidone VA64. The weight of the coating membrane is 5.9% of the mass of the tablet core. The solid content of the immediate-release overcoat suspension is 10.0 wt%, comprising, in weight percentage, 24.0 wt% of levodopa, 65.0 wt% of carbidopa monohydrate, 10.0 wt% of hydroxypropyl cellulose and 1.0 wt% of aspartame; the weight of the overcoat is 13.2% of the weight of the tablet core (table core + first layer coating membrane comprising cellulose acetate and Copovidone VA64). When the solvent for the overcoat suspension is absolute ethanol, the level of the carbidopa-related genotoxic impurity hydrazine in the obtained dosage form is 1.7 ppm, and the level of the carbidopa-related impurity dihydroxyphenylacetone (DHPA) is 0.21%. When the solvent for the coating suspension of the overcoat is purified water, the solid content of the overcoat is 10.0 wt%, comprising, in weight percentage, 24.0 wt% of levodopa, 65.0 wt% of carbidopa monohydrate, 10.0 wt% of hydroxypropyl cellulose and 1.0 wt% of aspartame; the level of the carbidopa-related genotoxic impurity hydrazine in the obtained dosage form is 3.8 ppm, and the level of the carbidopa-related impurity DHPA is 0.28%. The carbidopa-related genotoxic impurity hydrazine and impurity DHPA of the obtained dosage form when the solvent for the coating suspension of the overcoat is absolute ethanol, are significantly lower than those of the obtained dosage form when the solvent for the coating suspension is purified water. The immediate release overcoat of the dosage form is rapidly released first, followed by sustained releasing for approximately 8 hours. The dosage form can be held in the oral cavity for 3-5 hours, and then be swallowed before meal time or kept in oral cavity for the whole release duration.

### Embodiment 21 Preparation of extended release platform (Each tablet contains 62.5mgCD + 500mgLD)

Dosage forms that are designed, shaped and suitable for dispensing the beneficial drugs levodopa and carbidopa monohydrate to the oral cavity are prepared as follows: First, a drug-containing layer composition is prepared, which comprises, in weight percentage, 67.9 wt% of LD, 25.1 wt% of hydroxypropyl cellulose with a weight average molecular weight of 80,000, 5.0 wt% of povidone K30, and 1.0 wt% of aspartame. The excipients are sieved by a 40 mesh stainless steel sieve respectively, then mixed with pure water and granulated until a homogenous wet substance is formed; the wet substance is sieved by a 4×4mm sieve and dried at 60 °C for 1 hour; the dried granules are sieved by a Φ1.5mm sieve, and then mixed with 1.0 wt% of magnesium stearate.

Next, a second composition, i.e. an osmotic layer, which comprises 55.0 wt% of sodium carboxymethyl cellulose 7H4XF, 39.0 wt% of sorbitol, 5.0 wt% of povidone K30 and 0.5 wt% of iron oxide red, is prepared; each of these components is sieved by 40 mesh stainless steel sieve, and then mixed with 95% ethanol and granulated until a homogenous wet substance is formed; the wet substance is sieved by a 4×4mm sieve and dried at 80 °C for 2 hours; the dried granules are sieved by a Φ1.2mm sieve, and then mixed with 0.5 wt% of magnesium stearate.

Next, the drug-containing layer and the osmotic layer granules are pressed into a bi-layer tablet core. First, 700 mg of the drug-containing layer granules are added to the 19×7.5mm special-shaped punch of the tablet press and compacted, then 350 mg of the osmotic layer granules are added to the punch, and the two layers of granules are pressed into a contact bi-layer tablet core by the tablet press.

Next, the bi-layer tablet core is coated with a coating membrane. The membrane-forming composition comprises, in weight percentage, 70 wt% of cellulose acetate with 39.8 wt% of acetyl content and 30 wt% of copovidone VA64. The membrane-forming composition is mixed with acetone to form a 4% of solid suspension. Using the process parameters listed in Embodiment 6, the membrane-forming composition is sprayed onto the bi-layer tablet cores in a Glatt GC 1 pan coater to form a coating membrane, and the membrane weight gain of the coated tablet is 5.0%. Finally, a 1.0 mm exit orifice is mechanically drilled on the drug-containing layer side of the dosage form. Residual solvents are removed by drying the dosage form at 40 °C and ambient humidity for 72 hours. Immediate release overcoat comprises, in weight percentage, 24.1 wt% of LD, 64.9 wt% of CD, 10.0 wt% of hydroxypropyl cellulose and 1.0 wt% of aspartame; the weight of the overcoat is 9.4% of the weight of the tablet core (table core + first coating membrane cellulose acetate and copovidone VA64). The immediate release overcoat of the dosage form is rapidly released first, followed by sustained releasing for approximately 16 hours. The osmotic delivery system can be maintained in the oral cavity for 16 hours, maintained in the oral cavity during the entire release duration, or fastened on the corresponding teeth in the oral cavity after being combined with the retention enabling platform REP and maintained there for 16 hours.

### Embodiment 22 Preparation of extended release platform (Each tablet contains 62.5mgCD + 375mgLD)

In this embodiment, the procedures of Embodiment 20 are repeated, and the dosage form comprises a drug-containing layer, an osmotic layer, a membrane-forming composition and an overcoat are identical to those provided in Embodiment 20. The drug-containing layer comprises, in weight percentage, 53.5 wt% of LD, 39.5 wt% of hydroxypropyl cellulose with a weight average molecular weight of 80,000, 5.0 wt% of povidone K30, 1.0 wt% of aspartame and 1.0 wt% of magnesium stearate. The osmotic layer comprises, in weight percentage, 55.0 wt% of sodium carboxymethyl cellulose 7H4XF, 39.0 wt% of sorbitol, 5.0 wt% of povidone K30, 0.5 wt% of iron oxide red and 0.5 wt% of magnesium stearate. The coating membrane comprises, in weight percentage, 70 wt% of acetyl acetate membrane with an acetyl content of 39.8 wt% and 30 wt% of Copovidone VA64. The weight of the coating membrane is 8.0% of the mass of the tablet core. Immediate release overcoat comprises, in weight percentage, 24.05 wt% of LD, 64.95 wt% of CD, 10.0 wt% of hydroxypropyl cellulose and 1.0 wt% of aspartame; the weight of the overcoat is 9.4% of the weight of the tablet core (table core + first coating membrane cellulose acetate and copovidone VA64). The immediate release overcoat of the dosage form is rapidly released first, followed by sustained releasing for approximately 16 hours. The osmotic delivery system can be maintained in the oral cavity for 16 hours, maintained in the oral cavity during the entire release duration, or fastened on the corresponding teeth in the oral cavity after being combined with the retention enabling platform REP and maintained there for 16 hours.

### Embodiment 23 Preparation of extended release platform (Each tablet contains 50mgCD + 500mgLD)

Dosage forms that are designed, shaped and suitable for dispensing the beneficial drugs levodopa and carbidopa monohydrate to the oral cavity are prepared as follows: First, a drug-containing layer composition is prepared, which comprises 62.5 wt% of LD, 31 wt% of hydroxypropyl cellulose with a weight average molecular weight of 80,000, 4.5 wt% of mannitol, 0.9 wt% of aspartame, 0.1% wt% of mint flavor and 0.5 wt% of magnesium stearate, these excipients are made by dry granulation, then sieved by a 1.2mm sieve, and then mixed with 0.5 wt% of magnesium stearate.

Next, a second composition, an osmotic layer, which comprises 55.0 wt% of sodium carboxymethyl cellulose 7H4XF, 34.0 wt% of sorbitol, 10 wt% of hydroxypropyl cellulose and 0.5 wt% of iron oxide red, is prepared; these excipients are made by dry granulation, then sieved by a 1.2mm sieve, and then mixed with 0.5 wt% of magnesium stearate.

Next, the drug-containing layer and the osmotic layer granules are pressed into a bi-layer tablet core. First, 600 mg of the drug-containing layer granules are added to the 19×7.5mm special-shaped punch of the tablet press and compacted, then 300 mg of the osmotic layer granules are added to the punch, and the two layers of granules are pressed into a contact bi-layer tablet core by the tablet press.

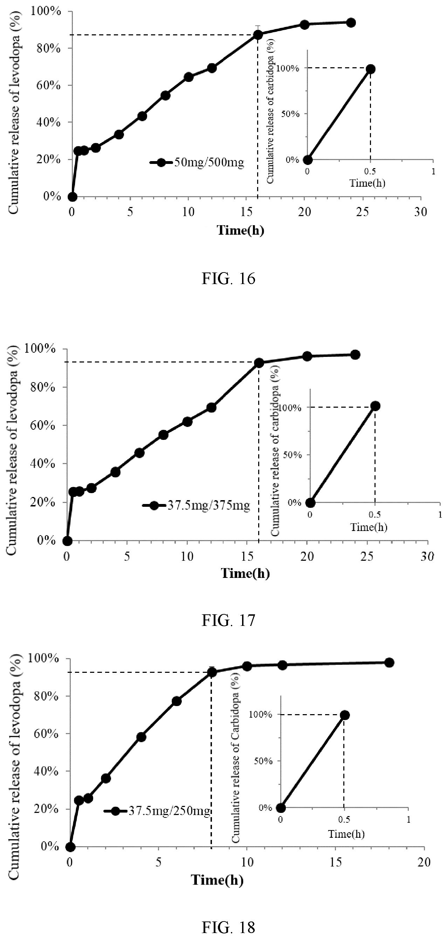

Next, the bi-layer tablet core is coated with a coating membrane. The membrane-forming composition comprises, in weight percentage, 70 wt% of cellulose acetate with 39.8 wt% of acetyl content and 30 wt% of copovidone VA64. The membrane-forming composition is mixed with acetone to form a 4% of solid suspension. Using the process parameters listed in Embodiment 6, the membrane-forming composition is sprayed onto the bi-layer tablet cores in a Glatt GC 1 pan coater to form a coating membrane, and the membrane weight gain of the coated tablet is 6.5%. Finally, a 1.0 mm exit orifice is mechanically drilled on the drug-containing layer side of the dosage form. The immediate release overcoat comprises, in weight percentage, 62.15 wt% of LD, 26.85 wt% of CD, 10.0 wt% of hydroxypropyl cellulose, 0.9 wt% of aspartame and 0.1% wt% of mint flavor; the weight of the overcoat is 21.0 % of the weight of the tablet core (table core + first coating membrane cellulose acetate and copovidone VA64). As shown in FIG. 16, the release profile of the dosage form shows rapid release of LD/CD, followed by an extended release with a duration of approximately 16 hours. The osmotic delivery system can be maintained in the oral cavity for 16 hours, maintained in the oral cavity during the entire release duration, or fastened on the corresponding teeth in the oral cavity after being combined with the retention enabling platform REP and maintained there for 16 hours.

### Embodiment 24 Preparation of extended release platform (Each tablet contains 37.5mgCD + 375mgLD)

Dosage forms that are designed, shaped and suitable for dispensing the beneficial drugs levodopa and carbidopa monohydrate to the oral cavity are prepared as follows: First, a drug-containing layer composition is prepared, which comprises 46.9 wt% of LD, 31.0 wt% of hydroxypropyl cellulose with a weight average molecular weight of 80,000, 20.1 wt% of mannitol, 0.9 wt% of aspartame, 0.1 wt% of mint flavor and 0.5 wt% of magnesium stearate, these excipients are made by dry granulation, then sieved by a 1.2mm sieve, and then mixed with 0.5 wt% of magnesium stearate.

Next, a second composition, an osmotic layer, which comprises 55.0 wt% of sodium carboxymethyl cellulose 7H4XF, 34.0 wt% of sorbitol, 10 wt% of hydroxypropyl cellulose and 0.5 wt% of iron oxide red, is prepared; these excipients are made by dry granulation, then sieved by a 1.2mm sieve, and then mixed with 0.5 wt% of magnesium stearate.

Next, the drug-containing layer and the osmotic layer granules are pressed into a bi-layer tablet core. First, 600 mg of the drug-containing layer granules are added to the 19×7.5mm special-shaped punch of the tablet press and compacted, then 300 mg of the osmotic layer granules are added to the punch, and the two layers of granules are pressed into a contact bi-layer tablet core by the tablet press.

Next, the bi-layer tablet core is coated with a coating membrane. The membrane-forming composition comprises, in weight percentage, 70 wt% of cellulose acetate with 39.8 wt% of acetyl content and 30 wt% of copovidone VA64. The membrane-forming composition is mixed with acetone to form a 4% of solid suspension. Using the process parameters listed in Embodiment 6, the membrane-forming composition is sprayed onto the bi-layer tablet cores in a Glatt GC 1 pan coater to form a coating membrane, and the membrane weight gain of the coated tablet is 6.5%. Finally, a 1.0 mm exit orifice is mechanically drilled on the drug-containing layer side of the dosage form. The immediate release overcoat comprises, in weight percentage, 62.15 wt% of LD, 26.85 wt% of CD, 10.0 wt% of hydroxypropyl cellulose, 0.9 wt% of aspartame and 0.1% wt% of mint flavor; the weight of the overcoat is 15.7 % of the weight of the tablet core (table core + first coating membrane cellulose acetate and copovidone VA64). As shown in FIG. 17, the release profile of the dosage form showed rapid release of LD/CD, followed by extended release with a duration of approximately 16 hours. The osmotic delivery system can be maintained in the oral cavity for 16 hours, maintained in the oral cavity during the entire release duration, or fastened on the corresponding teeth in the oral cavity after being combined with the retention enabling platform REP and maintained there for 16 hours.

### Embodiment 25 Preparation of extended release platform (Each tablet contains 37.5mgCD + 250mgLD)

Firstly, a drug layer composition comprising 45.0 wt% of levodopa, 31.0 wt% of hydroxypropyl cellulose, 22.0 wt% of mannitol, 0.9 wt% of aspartame, 0.1 wt% of mint flavor and 0.5 wt% of magnesium stearate is prepared, the components are each passed through a 40-mesh stainless steel sieve and granulated to obtain dry granules by a dry granulator, and then mixed with 0.5 wt% of magnesium stearate.

Next, a second composition, i.e. an osmotic layer, comprising 55.0 wt% of sodium carboxymethyl cellulose 7H4XF, 34.0 wt% of sorbitol, 10.0 wt% of hydroxypropyl cellulose and 0.5 wt% of iron oxide red is prepared; the components are respectively passed through a 40-mesh stainless steel sieve and then dried to obtain dry granules by a dry granulator, and then mixed with 0.5 wt% of magnesium stearate.

Next, the drug-containing layer and the osmotic layer granules are pressed into a bi-layer tablet core. First, 417 mg of the drug-containing layer granules are added to the 16* 7mm special-shaped punch of the tablet press and compacted, then 208 mg of the osmotic layer granules are added to the punch, and the two layers of granules are pressed into a contact bi-layer tablet core by the tablet press.

Next, the bi-layer tablet core is coated with a coating membrane. The membrane-forming composition comprises, in weight percentage, 70 wt% of cellulose acetate with 39.8 wt% of acetyl content and 30 wt% of copovidone VA64. The membrane-forming composition is mixed with acetone to form a 4% of solid suspension. Using the process parameters listed in Embodiment 6, the membrane-forming composition is sprayed onto the bi-layer tablet cores in a Glatt GC 1 pan coater to form a coating membrane, and the membrane weight gain of the coated tablet is 6.5%. Finally, a 1.0 mm exit orifice is laser-drilled on the drug-containing layer side of the dosage form.

Next, an immediate-release composition, comprising 54.0 wt% of levodopa, 35.0 wt% of carbidopa monohydrate, 10.0 wt% of hydroxypropyl cellulose, 0.9 wt% of aspartame and 0.1 wt% of mint flavor, is used to overcoat the dried dosage form, with the membrane weight gain of 6.5%. The immediate-release overcoat composition is mixed with absolute ethanol to make a 10.0 wt% of solid suspension. The final dosage form is composed of an immediate release overcoat of 62.5 mg levodopa and 37.5 mg carbidopa, and 187.5 mg levodopa is contained in an extended release drug-containing layer.

As shown in FIG. 18, the release profile of the dosage form showed rapid release of LD/CD, followed by extended release with a duration of approximately 8 hours. The osmotic delivery system can be kept in oral cavity for 4-5 hours, and then is be swallowed before meal time or kept in oral cavity for the whole release duration.

### Embodiment 26 Preparation of extended release platform (Each tablet contains 37.5mgCD + 150mgLD)

In this embodiment, the procedures of Embodiment 25 are repeated, and the dosage form comprises a drug-containing layer, an osmotic layer, a membrane-forming composition and an overcoat are identical to those provided in Embodiment 25.

Firstly, a drug layer composition comprising 45.0 wt% of levodopa, 31.0 wt% of hydroxypropyl cellulose, 22.0 wt% of mannitol, 0.9 wt% of aspartame, 0.1 wt% of mint flavor and 0.5 wt% of magnesium stearate is prepared, the components are each passed through a 40-mesh stainless steel sieve and granulated to obtain dry granules by a dry granulator, and then mixed with 0.5 wt% of magnesium stearate.

Next, a second composition, i.e. an osmotic layer, comprising 55.0 wt% of sodium carboxymethyl cellulose 7H4XF, 34.0 wt% of sorbitol, 10.0 wt% of hydroxypropyl cellulose and 0.5 wt% of iron oxide red is prepared; the components are respectively passed through a 40-mesh stainless steel sieve and then dried to obtain dry granules by a dry granulator, and then mixed with 0.5 wt% of magnesium stearate.

Next, the drug-containing layer and the osmotic layer granules are pressed into a bi-layer tablet core. First, 250 mg of drug-containing layer granules are added to a 9 mm round punch of a tablet press and tamped, then 125 mg of osmotic layer granules are added to the punch, and the granules of both layers are pressed with a tablet press into a contacting bi-layer tablet core.

Next, the bi-layer tablet core is coated with a coating membrane. The membrane-forming composition comprises, in weight percentage, 70 wt% of cellulose acetate with 39.8 wt% of acetyl content and 30 wt% of copovidone VA64. The membrane-forming composition is mixed with acetone to form a 4% of solid suspension. Using the process parameters listed in Embodiment 6, the membrane-forming composition is sprayed onto the bi-layer tablet cores in a Glatt GC 1 pan coater to form a coating membrane, and the membrane weight gain of the coated tablet is 7.0%. Finally, a 0.75 mm exit orifice is laser-drilled on the drug-containing layer side of the dosage form.

Next, an immediate-release composition, comprising 42.8 wt% of levodopa, 46.2 wt% of carbidopa monohydrate, 10.0 wt% of hydroxypropyl cellulose, 0.9 wt% of aspartame and 0.1 wt% of mint flavor, is used to overcoat the dried dosage form, with the membrane weight gain of 7.0%. The immediate-release overcoat composition is mixed with absolute ethanol to make a 10.0 wt% of solid suspension. The final dosage form is composed of an immediate release overcoat of 37.5 mg levodopa and 37.5 mg carbidopa, and 112.5 mg levodopa is contained in an extended release drug-containing layer.

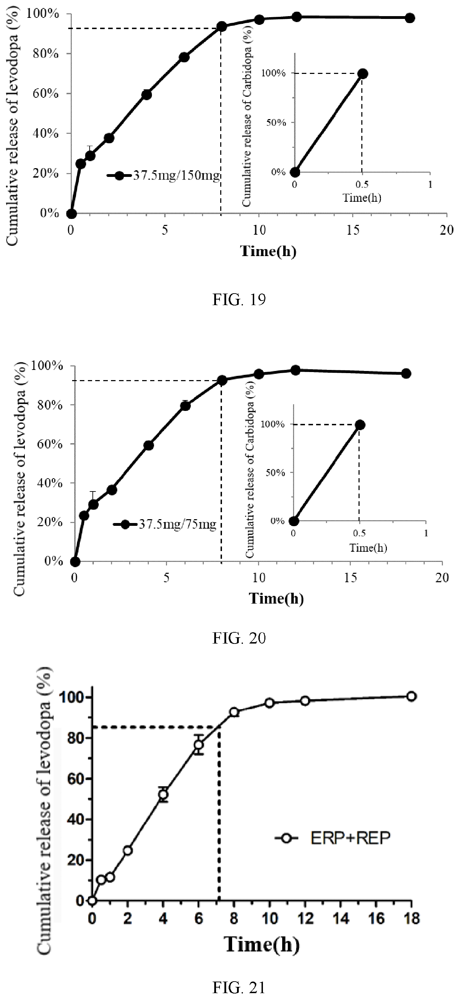

As shown in FIG. 19, the release profile of the dosage form shows rapid release of LD/CD, followed by extended release with a duration of approximately 8 hours. The osmotic delivery system can be kept in oral cavity for 4-5 hours, and then is be swallowed before meal time or kept in oral cavity for the whole release duration.

### Embodiment 27 Preparation of extended release platform (Each tablet contains 37.5mgCD + 75mgLD)

In this embodiment, the procedures of Embodiment 25 are repeated, and the dosage form comprises a drug-containing layer, an osmotic layer, a membrane-forming composition and an overcoat are identical to those provided in Embodiment 25.

First, a drug-containing layer composition comprising 45.0 wt% of levodopa, 31.0 wt% of hydroxypropyl cellulose, 22.0 wt% of mannitol, 0.9 wt% of aspartame, 0.1 wt% of mint flavor and 0.5 wt% of magnesium stearate is prepared, the components are each passed through a 40-mesh stainless steel sieve, and dried to obtain dry granules by a dry granulator, then mixed with 0.5 wt% of magnesium stearate.

Next, a second composition, i.e. an osmotic layer, comprising 55.0 wt% of sodium carboxymethyl cellulose 7H4XF, 34.0 wt% of sorbitol, 10.0 wt% of hydroxypropyl cellulose and 0.5 wt% of iron oxide red is prepared; the components are respectively passed through a 40-mesh stainless steel sieve and then dried to obtain dry granules by a dry granulator, and then mixed with 0.5 wt% of magnesium stearate.

Next, the drug-containing layer and the osmotic layer granules are pressed into a bi-layer tablet core. First, 125 mg of drug-containing layer granules are added to a 7 mm round punch of a tablet press and tamped, then 62.5 mg of osmotic layer granules are added to the punch, and the granules of both layers are pressed with a tablet press into a contacting bi-layer tablet core.

Next, the bi-layer tablet core is coated with a coating membrane. The membrane-forming composition comprises, in weight percentage, 70 wt% of cellulose acetate with 39.8 wt% of acetyl content and 30 wt% of copovidone VA64. The membrane-forming composition is mixed with acetone to form a 4% of solid suspension. Using the process parameters listed in Embodiment 6, the membrane-forming composition is sprayed onto the bi-layer tablet cores in a Glatt GC 1 pan coater to form a coating membrane, and the membrane weight gain of the coated tablet is 9.0%. Finally, a 0.5 mm exit orifice is laser-drilled on the drug-containing layer side of the dosage form.

Next, an immediate-release composition, comprising 28.2 wt% of levodopa, 60.8 wt% of carbidopa monohydrate, 10.0 wt% of hydroxypropyl cellulose, 0.9 wt% of aspartame and 0.1 wt% of mint flavor, is used to overcoat the dried dosage form, with the membrane weight gain of 9.0%. The immediate-release overcoat composition is mixed with absolute ethanol to make a 10.0 wt% of solid suspension. The final dosage form is composed of an immediate release overcoat of 18.75 mg levodopa and 37.5 mg carbidopa, and 56.25 mg levodopa is contained in an extended release drug-containing layer.

As shown in FIG. 20, the release profile of the dosage form shows rapid release of LD/CD, followed by extended release with a duration of approximately 8 hours. The osmotic delivery system can be kept in oral cavity for 4-5 hours, and then is be swallowed before meal time or kept in oral cavity for the whole release duration.

### Embodiment 28 Coordination of extended release platform and retention enabling platform

The extended release platform (ERP) of embodiments 6-27 is fastened to the personalized retention enabling platform (REP) of embodiments 1-5 to form the extended release dosage form (ERP + REP) of the present disclosure. Then, the extended release dosage form (ERP + REP) is fastened on the corresponding teeth in the oral cavity. The extended release dosage form (ERP + REP) can be maintained in the oral cavity until the push layer reaches the delivery orifice, or maintained there for 4-24 hours. The extended release dosage form (ERP + REP) is taken out, the extended release platform (ERP) is replaced, and the extended release dosage form (ERP + REP) is fastened on the corresponding teeth in the oral cavity again, thus releasing the drug continuously.

### Embodiment 29 Coordination of extended release platform and retention enabling platform (Each tablet contains 62.5mgCD + 250mgLD)

In this embodiment, the procedures of embodiment 9 are repeated to provide a dosage form. In this embodiment, an immediate release composition comprising 23.78 wt% of LD, 64.22 wt% of CD, 10 wt% of hydroxypropyl cellulose and 1 wt% of aspartame is used to overcoat the dried dosage form with 4.8% membrane weight gain (as shown in FIG. 3C). The immediate release overcoat composition is mixed with absolute ethanol to make a 6.7 wt% of solid suspension. The final extended release platform ERP dosage form comprises an immediate release coating layer of 62.5 mg CD and 25 mg LD, and an extended release drug-containing layer of 225 mg LD.

The extended release platform (ERP) is fastened to the personalized retention enabling platform (REP) to form the extended release dosage form (ERP + REP) of the present disclosure. Then, the extended release dosage form (ERP + REP) is fastened on the corresponding teeth in the oral cavity. The extended release dosage form (ERP + REP) can be maintained in the oral cavity until the push layer reaches the delivery orifice or maintained there for 8 hours. The extended release dosage form (ERP + REP) is taken out, the extended release platform (ERP) is replaced, and the extended release dosage form (ERP + REP) is fastened on the corresponding teeth in the oral cavity again, thus releasing the drug continuously.

The USP I basket method is used to determine the release profile of an extended release dosage form (ERP + REP) in a 0.1N hydrochloric acid aqueous solution. As shown in FIG. 21, an extended release dosage form (ERP + REP) with a membrane weight gain of 4.8% delivers LD at an average rate of 27.6 mg/hr, with 85% of LD delivered in 7.7 hours and 85% of CD delivered in 1 hour.

Although the specific embodiments of the present disclosure are described above, those skilled in the art should understand that these are merely embodiments, and that various changes or modifications may be made to these embodiments without departing from the principle and essence of the present disclosure. Therefore, the protection scope of the present disclosure is defined by the appended claims.

### Embodiment 30 Cobalt-chromium alloy back-insertion oral retention device

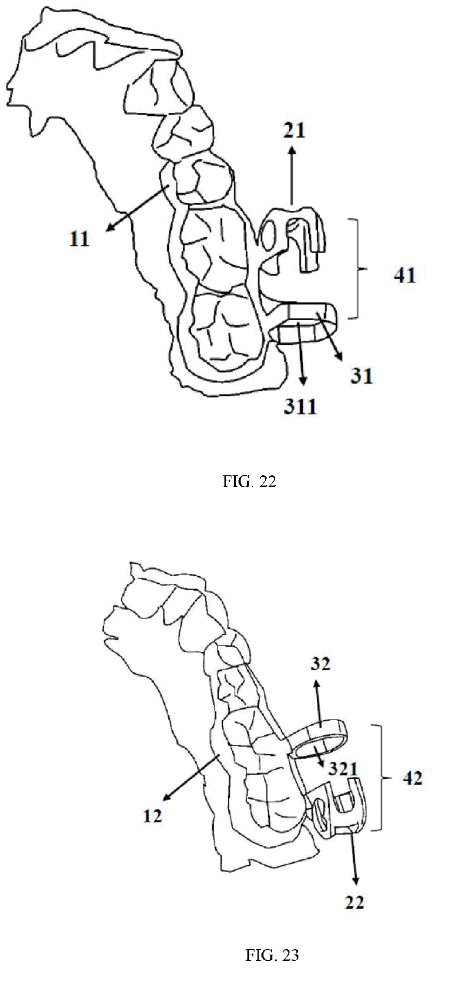

In the embodiment shown in FIG. 22, the oral retention device with a medicinal tablet being inserted from the back is composed of a tooth-matching component 11 and a drug-loaded component 41 connected to each other. The tooth-matching component 11 can be closely attached to the teeth in the oral cavity of the subject, and the drug-loaded component 41 is sized to hold at least one medicinal tablet and retain the medicinal tablet in the oral cavity. Both the tooth-matching component 11 and the drug-loaded component 41 are made of cobalt-chromium alloy, and the tooth-matching component 11 and the drug-loaded component 41 are connected on respective sides. The drug-loaded component comprises a ring body 31 and a retainer 21 that is located at the end. The ring body 31 is a circular closed ring body with an opening 311 for insertion of a medicinal tablet. The retainer and the ring body are arranged at an interval, and are respectively connected to the tooth-matching component 11. In another solution, the retainer may be integrally formed with the ring body and then fixed on the tooth-matching component, and may also be integrally formed with the ring body and the tooth-matching component. After the oral retention device matches with the teeth in the oral cavity of the subject via the tooth-matching component, the opening 311 opens toward the molars in the horizontal direction formed by the molars and the incisors (the ring body 31 is located on the molar side in the horizontal direction, that is, the position of the ring body 31 is closer to the molars than the position of the retainer 21), and the medicinal tablet is inserted from the molars toward the incisors in the horizontal direction. The retainer is of a circular arc hollow shape, and has a structure configured to limit the medicinal tablet in the drug-loaded component. The tooth-matching component 11 matches the first molar, the second molar and/or the third molar on the mandible in the oral cavity of the subject, and wraps the teeth by means of matching.

Wearing the oral retention device loaded with a medicinal tablet according to this embodiment in the oral cavity, even if the immediate release layer of the drug is quickly dissolved after intensive gargle, the medicinal tablet can be firmly secured and will not slip off the device.

### Embodiment 31 Cobalt-chromium alloy front-insertion oral retention device

In the embodiment shown in FIG. 23, the oral retention device with a medicinal tablet being inserted from the front is composed of a tooth-matching component 12 and a drug-loaded component 42 connected to each other. The tooth-matching component 12 can be closely attached to the teeth in the oral cavity of the subject, and the drug-loaded component 42 is sized to hold at least one medicinal tablet and retain the medicinal tablet in the oral cavity. Both the tooth-matching component 12 and the drug-loaded component 42 are made of cobalt-chromium alloy, and the tooth-matching component 12 and the drug-loaded component 42 are connected on respective sides. The drug-loaded component 42 comprises a ring body 32 and a retainer 22 that is located at the end. The ring body 32 is a circular closed ring body with an opening 321 for insertion of a medicinal tablet. The retainer and the ring body are arranged at an interval, and are respectively connected to the tooth-matching component 12. In another solution, the retainer may be integrally formed with the ring body and then fixed on the tooth-matching component, and may also be integrally formed with the ring body and the tooth-matching component. The opening 321 opens toward the incisors in the horizontal direction formed by the molars and the incisors (the ring body 32 is located on the incisor side in the horizontal direction formed by the molars and the incisors, that is, the position of the ring body 32 is closer to the incisors than the position of the retainer 22), and the medicinal tablet is inserted from the incisors toward the molars in the horizontal direction formed by the molars and the incisors. The retainer is of a circular arc hollow shape, and the retainer has a structure configured to limit the medicinal tablet in the drug-loaded component. The tooth-matching component 12 matches the first molar, the second molar and/or the third molar on the mandible in the oral cavity of the subject, and wraps the teeth by means of matching.

Wearing the oral retention device loaded with a medicinal tablet according to this embodiment in the oral cavity, the medicinal tablet may slip off the device during the rapid dissolution of the immediate release layer of the drug after intensive gargle, and may slip off the device during the wearing, but still has a certain use value.

### Embodiment 32 Preparation of cobalt-chromium alloy back-insertion oral retention device by means of 3D printing

The method for preparing the cobalt-chromium alloy back-insertion oral retention device according to this embodiment comprises the following steps:
step 1: obtaining the data of size of the medicinal tablet by scanning with a "3Shape Dental System" scanner; then, with the software "SolidWorks", designing a drug-loaded component capable of loading the drug part, and creating and saving a "standard attachment" file; every time an oral retention device is designed in the software "3Shape Dental System", adding the "standard attachment" and assembling same with the tooth-matching component to form an integrated oral retention device;
step 2: performing an intraoral scan:
   a. opening Trios intraoral scanning software;
   b. creating a new patient file, creating a new case file, and confirming;
   c. choosing a research model;
   d. performing scanning on the mandible with the Trios intraoral scanner;
   e. performing scanning on the maxillary;
   f. performing scanning for the occlusal relationship;
   g. post-processing the files to confirm that the scanning data of all molars and premolars are complete and free of defects, and the accurate scanning of occlusion of upper and lower teeth is implemented; and
   h. saving 3OXZ or STL format files;
step 3: designing the device
   a. opening the software 3Shape Dental System;
   b. creating a new order;
   c. selecting the tooth position;
   d. choosing a basal crown design;
   e. importing the oral scanning teeth data (a STL file);
   f. adding the "standard attachment" and assembling with the tooth-matching component to form an integrated oral retention device, in which the opening opens toward the molars in the horizontal direction formed by the molars and the incisors such that the medicinal tablet is located in the horizontal direction formed by the molars and the incisors and can be inserted from the molars toward the incisors; and
   g. after the design is completed, exporting a 3OXZ or STL format file; and
step 4: performing 3D printing/in-place polishing;
   a. inputting a CAM program;
   b. turning on an EOS laser accumulation apparatus;
   c. preparing cobalt-chromium alloy metal powder;
   d. downloading the processing program of the product to be produced;
   e. clicking to start processing;
   f. after the processing is completed, opening the compartment door and cleaning by dust collection; and
   g. taking out the device, performing in-place/grinding and polishing. The device is as shown in FIG. 1.

As shown in FIG. 22, the cobalt-chromium alloy back-insertion oral retention device prepared according to this embodiment comprises a tooth-matching component 11 and a drug-loaded component. The tooth-matching component 11 and the drug-loaded component are connected on respective sides, the tooth-matching component can be closely attached to a tooth in the oral cavity, and the drug-loaded component can hold at least one medicinal tablet and retain the medicinal tablet in the oral cavity. The medicinal tablet may be a controlled-release preparation, preferably an osmotic pump tablet. The osmotic pump tablet contains active pharmaceutical ingredient(s) and adjuvants, the active pharmaceutical ingredient(s) have an ingredient of one of levodopa or its ester, carbidopa, baclofen, acyclovir, valaciclovir, ganciclovir, metformin, and gabapentin, or one or two of levodopa or its ester and carbidopa. The drug-loaded component has a cross section in the shape of a circular closed ring. The drug-loaded component may have a reticular structure or a nonreticular structure. The drug-loaded component comprises at least one ring body 31 and at least one retainer 21 that is located at the end, and the ring body 31 forms an opening 311 for insertion of a medicinal tablet. The retainer is of a circular arc hollow shape. The opening faces the molars in the horizontal direction formed by the molars and the incisors, and the medicinal tablet is inserted from the molars toward the incisors in the horizontal direction.

### Embodiment 33 Preparation of cobalt-chromium alloy oral retention device by means of injection molding

At first, a tooth gypsum model of a patient/volunteer is prepared by means of the traditional model-taking technology; then, by means of a traditional manual process, with dental wax as the material, a dental wax model is manually prepared based on the gypsum model; and finally, with cobalt-chromium porcelain alloy as the material, the oral retention device is prepared by means of a traditional injection molding process.

### Embodiment 34 Cobalt-chromium alloy front-insertion oral retention device prepared by means of 3D printing

The oral retention device as shown in FIG. 23 is prepared by the same method as Embodiment 32, only different in the f in step 3, the positions of the ring body and the retainer of the drug-loaded component are opposite to those in Embodiment 32. The ring body 32 of the drug-loaded component 42 forms an opening 321 for insertion of a medicinal tablet, the opening opens toward the incisors in the horizontal direction formed by the molars and the incisors, and the medicinal tablet is inserted from the incisors to the molars in the horizontal direction. The medicinal tablet may be a controlled-release preparation, preferably an osmotic pump tablet.

### Embodiment 35

This embodiment is an oral drug delivery device that covers all mandibular teeth. As shown in FIG. 24, it comprises a core component 1 and a fixing component 2. The fixing component 2 is a fixator. The structural schematic diagram of the core component 1 is shown in FIG. 25, the structural schematic diagram of the fixing component 2 is shown in FIG. 26, and the cross-sectional view of the oral drug delivery device is shown in FIG. 27. The lingual side 201 of the fixing component, the buccal side 202 of the fixing component and the occlusal surface 203 of the fixing component correspondingly anastomose to the lingual side, buccal side and occlusal surface of the teeth and cover the outer peripheral surface of the teeth; the fixing component 2 covers molar-fitting functional area 11 at the same time. The core component 1 is composed of a molar-fitting functional area 11 and a drug-loaded functional area 12. The molar-fitting functional area 11 can be closely matched with the mandibular first and second premolars and the first and second molars in the subject's oral cavity, and the size of the drug-loaded functional area 12 allows it to accommodate at least one tablet and fix the tablet in the oral cavity. The fixing component 2 is tightly matched with all the mandibular teeth of the subject and with the molar-fitting functional area 11, so that the core component 1 and the tablet are fixed in the oral cavity. Both the molar-fitting functional area 11 and the drug-loaded functional area 12 are made of cobalt-chromium alloy, and the molar-fitting functional area 11 and the drug-loaded functional area 12 are connected on respective sides. The drug-loaded functional area 12 comprises a first ring 121 and a second ring 122. The first ring 121 and the second ring 122 are circular closed rings. The tablet is inserted from the second ring 122 and blocked by the first ring 121. The molar-fitting functional area 11 and the fixing component 2 are combined by embedding through mechanical assembly method. The fixing component 2 is provided with slotted holes at a location corresponding to the drug-loaded functional area 12 to achieve removable fixation between the fixing component 2 and the core component 1. The fixing component 2 is made of colorless and transparent polyethylene terephthalate and covers all the mandibular teeth, that is, covers 14 teeth. The core component 1 and the fixing component 2 are combined to match the teeth in the subject's oral cavity.

The oral drug delivery device of this embodiment can be worn firmly in the oral cavity after holding the tablets, the tablets are fixed in the oral cavity, and the fixator, the core component and the tablets will not fall off or shift with the actions of the oral cavity. The oral drug delivery device has high wearing comfort, and has little impact on facial appearance.

### Embodiment 36

This embodiment is an oral drug delivery device covering 10 mandibular teeth. As shown in FIG. 28, it comprises a core component 1 and a fixing component 2, the fixing component 2 is a fixator. The core component 1 is composed of a molar-fitting functional area 11 and a drug-loaded functional area 12. The molar-fitting functional area 11 can be closely matched with the mandibular first and second premolars and the first and second molars in the subject's oral cavity, and the size of the drug-loaded functional area 12 allows it to accommodate at least one tablet and fix the tablet in the oral cavity. The fixing component 2 is tightly matched with part of the subject's mandibular teeth (including the teeth from the left canine to the right second molar), and is tightly fitted with the molar-fitting functional area 11, so that the core component 1 and the tablet are fixed in the oral cavity. Both the molar-fitting functional area 11 and the drug-loaded functional area 12 are made of cobalt-chromium alloy, and the molar-fitting functional area 11 and the drug-loaded functional area 12 are connected on respective sides. The drug-loaded functional area 12 comprises a first ring 121 and a second ring 122. The first ring 121 and the second ring 122 are elliptical closed rings. A pair of clamping walls 123 are symmetrically provided on the first ring 121 in the direction of the open end of the molar-fitting functional area, which is suitable for inserting non-cylindrical shaped tablets. The tablets are inserted from the second ring 122 and blocked by the first ring 121. The molar-fitting functional area 11 and the fixing component 2 are combined by embedding through a mechanical assembly method. The fixing component 2 is provided with slotted holes at a location corresponding to the drug-loaded functional area 12 to achieve removable fixation between the fixing component 2 and the core component 1. The fixing component 2 is made of colorless and transparent polyethylene terephthalate and covers 10 teeth of the mandible from the left canine to the right second molar. The core component 1 and the fixing component 2 are combined to match with part of the mandibular teeth in the subject's oral cavity.

The oral drug delivery device of this embodiment can be worn firmly in the oral cavity after holding the tablets, the tablets are fixed in the oral cavity, and the fixator, the core component and the tablets will not fall off or shift with the actions of the oral cavity. The oral drug delivery device has high wearing comfort, and has little impact on facial appearance.

### Embodiment 37

This embodiment is an oral drug delivery device covering 7 mandibular teeth. As shown in FIG. 29, it comprises a core component 1 and a fixing component 2, the fixing component 2 is a fixator. The core component 1 is composed of a molar-fitting functional area 11 and a drug-loaded functional area 12. The molar-fitting functional area 11 can be closely matched with the mandibular first and second premolars and the first and second molars in the subject's oral cavity, and the size of the drug-loaded functional area 12 allows it to accommodate at least one tablet and fix the tablet in the oral cavity. The fixing component 2 is tightly matched with part of the mandibular teeth (including a total of 7 teeth from the right incisor to the right second molar), and is tightly fitted with the molar-fitting functional area 11, so that the core component 1 and the tablet are fixed in the oral cavity. Both the molar-fitting functional area 11 and the drug-loaded functional area 12 are made of cobalt-chromium alloy, and the molar-fitting functional area 11 and the drug-loaded functional area 12 are connected on respective sides. The drug-loaded functional area 12 comprises a first ring 121 and a second ring 122. The first ring 121 and the second ring 122 are circular closed rings and are suitable for inserting cylindrical-shaped tablets. The tablets are inserted from the second ring 122 and blocked by the first ring 121. The molar-fitting functional area 11 and the fixing component 2 are combined by embedding through a mechanical assembly method. The fixing component 2 is provided with slotted holes at a location corresponding to the drug-loaded functional area 12 to achieve removable fixation between the fixing component 2 and the core component 1. The fixing component 2 is made of colorless and transparent ethylene-vinyl acetate copolymer and covers a total of 7 teeth on the right side of the mandible from the incisor to the second molar. The core component 1 and the fixing component 2 are combined to match the subject's teeth in the subject's oral cavity.

Wearing the oral drug delivery device of this embodiment in the oral cavity, after being loaded with the tablets, the tablets can be fixed in the oral cavity and not easy to fall off. The wearing firmness is not significantly different from that of Embodiments 35 and 36. And at the same time, because the number of teeth covered is small, the wearing comfort and the degree of influence on facial appearance is better than that of Embodiments 35 and 36.

### Embodiment 38

The difference between this embodiment and Embodiment 35 is that the oral drug delivery device covers all of the maxillary teeth, and the molar-fitting functional area 11 can be closely matched with the maxillary second premolar and the first, second, and third molars in the subject's oral cavity. Others are the same as Embodiment 35.

The oral drug delivery device of this embodiment can be worn firmly in the oral cavity after holding the tablets, the tablets are fixed in the oral cavity, and the fixator, the core component and the tablets will not fall off or shift with the actions of the oral cavity. The oral drug delivery device has high wearing comfort, and has little impact on facial appearance.

### Embodiment 39

The difference between this embodiment and Embodiment 35 is that the length of the core component 1 corresponds to 3 teeth and can be designed for subjects with molar defects; the first ring 121 and the second ring 122 are polygonal closed rings, and the molar-fitting functional area 11 and the fixing component 2 are combined by bonding with a pressure-sensitive adhesive as the adhesive, and the bonding position is the entire outer surface of the molar-fitting functional area 11, and the thickness of the adhesive is 0.1 mm; the fixing component 2 is prepared from colorless and transparent ethylene-vinyl acetate copolymer. Others are the same as Embodiment 35.

The oral drug delivery device of this embodiment can be worn firmly in the oral cavity after holding the tablets, the tablets are fixed in the oral cavity, and the fixator, the core component and the tablets will not fall off or shift with the actions of the oral cavity. The oral drug delivery device has high wearing comfort, and has little impact on facial appearance.

### Embodiment 40

The structural schematic diagram of the oral drug delivery device of this embodiment is shown in FIG. 30. It comprises a core component 1 and a fixing component 2. The fixing component 2 is a fixing layer. The cross-sectional view of the oral drug delivery device is shown in FIG. 31. The core component 1 is composed of a molar-fitting functional area 11 and a drug-loaded functional area 12. The molar-fitting functional area 11 can be closely matched with the mandibular first and second premolars and the first and second molars in the subject's oral cavity. The drug holding functional area comprises a first ring 121 and a second ring 122. The first ring 121 and the second ring 122 are circular closed rings and are suitable for inserting cylindrical-shaped tablets. The tablets are inserted from the second ring 122 and blocked by the first ring 121. The size of drug-loaded functional area 12 allows it to accommodate at least one tablet and fix the tablet in the oral cavity. The fixing component 2 is attached to all outer surfaces of the first section of the U-shaped structure and the second section of the U-shaped structure; the molar-fitting functional area 11 and the drug-loaded functional area 12 are made of cobalt-chromium alloy, and the molar-fitting functional area 11 and the drug-loaded functional area 12 are connected at their respective sides. The fixing component 2 is made of colorless and transparent polyethylene terephthalate, which is softened by heating and then covered on the surface of the molar-fitting functional area 11, and is obtained by removing excess material after cooling. The thickness of the fixing component 2 is 0.3-0.5 mm, and the core component 1 and the fixing component 2 are combined to match the subject's teeth.

The oral drug delivery device of this embodiment can be worn firmly in the oral cavity after being loaded with the tablets, and the tablets are fixed in the oral cavity, and the fixing layer, the core component and the tablets will not fall off or shift with the actions of the oral cavity. The oral drug delivery device has high wearing comfort, and has little impact on facial appearance.

### Embodiment 41

The difference between this embodiment and Embodiment 40 is that the fixing component 2 is made of light blue polycaprolactone, and the thickness of the fixing component 2 is 0.8-1 mm. Others are the same as Embodiment 40.

The oral drug delivery device of this embodiment can be worn firmly in the oral cavity after being loaded with tablets, and the tablets can be fixed in the oral cavity. However, as the thickness of the fixing component is relatively thick, the degree of wearing firmness and comfort of the oral drug delivery device in this embodiment is not as good as that of Embodiment 40. Moreover, compared with Embodiment 40, this embodiment has a greater impact on facial appearance.

### Embodiment 42

The difference between this embodiment and Embodiment 40 is that the core component 1 and the fixing component 2 are bonded using pressure-sensitive adhesive. The bonding position is the part where the molar-fitting functional area is in contact with the teeth. The thickness of the adhesive is 0.1 mm; the fixing component 2 is a colorless and transparent polypropylene membrane with a thickness of 0.02-0.04 mm. Others are the same as Embodiment 40.

The oral drug delivery device in this embodiment can be worn firmly in the oral cavity after being loaded with tablets, and the tablets can be fixed in the oral cavity. However, as the thickness of the fixing component is relatively thin and the surface of the fixing layer material is smooth, the degree of wearing firmness and comfort of the oral drug delivery device in this embodiment is not as good as that of Embodiments 40 and 41.

### Embodiment 43

The oral drug delivery device of this embodiment is shown in FIG. 32, which comprises core components 1 which are symmetrical on both sides and a fixing component 2, the fixing component 2 is a fixator. Two core components 1 are composed of a molar-fitting functional area 11 and a drug-loaded functional area 12. The molar-fitting functional area 11 can be closely matched with the maxillary first and second premolars and the first and second molars in the subject's oral cavity, and the size of the drug-loaded functional area 12 allows it to accommodate at least one tablet and fix the tablet in the oral cavity. The fixing component 2 is tightly matched with all the maxillary teeth of the subject and with the molar-fitting functional area 11, so that the core components 1 and the tablet are fixed in the oral cavity. Both the molar-fitting functional area 11 and the drug-loaded functional area 12 are made of cobalt-chromium alloy, and the molar-fitting functional area 11 and the drug-loaded functional area 12 are connected on respective sides. The drug-loaded functional area 12 comprises a first ring 121 and a second ring 122. The first ring 121 and the second ring 122 are circular closed rings and are suitable for inserting cylindrical-shaped tablets. The tablets are inserted from the second ring 122 and blocked by the first ring 121. The molar-fitting functional area 11 and the fixing component 2 are combined by embedding through mechanical assembly method. The fixing component 2 is provided with slotted holes at a location corresponding to the drug-loaded functional area 12 to achieve removable fixation between the fixing component 2 and the core component 1. The fixing component 2 is made of colorless and transparent polyethylene terephthalate and covers all the maxillary teeth. The core components 1 and the fixing component 2 are combined to match the teeth in the subject's oral cavity.

The oral drug delivery device of this embodiment can be worn firmly in the oral cavity after holding the tablets, the tablets are fixed in the oral cavity, and the fixator, the core component and the tablets will not fall off or shift with the actions of the oral cavity. The oral drug delivery device has high wearing comfort, and has little impact on facial appearance.

### Embodiment 44

The laser casting method is used to prepare a cobalt-chromium alloy core component and the thermoforming method is used to prepare a fixator. The specific steps are as follows:
Step 1: Obtaining the oral data of the subject by scanning the subject's oral cavity, or taking an oral mold to make a plaster model and then scanning.
Step 2: Using 3D design software and dental design software to design the drug holding functional area and molar-fitting functional area of the core component respectively, and combining them in the dental design software to generate the core component design file.
Step 3: Preparing the cobalt-chromium alloy core component through laser casting, grinding, polishing, and cleaning.
Step 4: Wearing the core component on the subject's dental mold, heating and softing polyethylene terephthalate through a thermoforming machine and then vacuuming the dental mold, removing excess material and polishing, and removing it from the dental mold to obtain an oral drug delivery device. The oral drug delivery device matches the subject's teeth.

### Embodiment 45

The injection molding is used to prepare a cobalt-chromium alloy core component and the thermoforming method is used to prepare a drug delivery device. The specific steps are as follows:
Step 1: Taking an oral mold of the subject and making a plaster model.
Step 2: Preparing a dental wax model on a plaster model by using dental wax as the material.
Step 3: Preparing a core component through traditional injection molding process by using cobalt-chromium porcelain alloy as material.
Step 4: Wearing the core component on the plaster dental mold, heating and softing polyurethane through a thermoforming machine and then vacuuming the dental mold, removing excess material and polishing, and removing it from the dental mold to obtain the oral drug delivery device with the combination of the core components and the fixator.

### Embodiment 46

The injection molding is used to prepare a cobalt-chromium alloy core component and the impression method is used to prepare a drug delivery device. The specific steps are as follows:
Step 1: taking an oral mold of the subject and making a plaster model.
Step 2: preparing a dental wax model on a plaster model by using dental wax as the material.
Step 3: preparing the drug delivery device through traditional injection molding process by using cobalt-chromium porcelain alloy as material.
Step 4: wearing the core component on the subject's corresponding teeth, heating and softing polycaprolactone material, covering it on the subject's teeth and the molar-fitting functional area of the core component, and removing it from the teeth together with the core component after cooling to obtain the oral drug delivery device.

### Comparative Embodiment 1

The oral drug delivery device of this comparative embodiment only comprises a core component 1 in Embodiment 35, as shown in FIG. 25.

### Test Embodiment 1

The oral drug delivery devices of Embodiment 35, Embodiment 37, Embodiment 40 and Comparative Embodiment 1 are tested for wearing firmness, and the wearing firmness scores are shown in the following Table 2: wherein, 1 score is not firm, and means that it is very easy to fall off during basic oral actions such as opening the mouth and speaking after wearing; 2 score is less firm, and it sometimes loosens, buckles, or falls off under basic oral actions such as opening the mouth and speaking after wearing; 3 score is basically firm, and it is easy to fall off only during large oral actions; 4 score is firm, and it buckles or loosens only during large oral actions, but not fall off; 5 score is very firm, and it will not loosen, buckle or fall off during basic oral actions or large oral actions.

**Table 2**

| Subject | Embodiment 35 | Embodiment 37 | Embodiment 40 | Comparative Embodiment 1 |
|---|---|---|---|---|
| 1 | 5 | 5 | 4 | 3 |
| 2 | 5 | 5 | 5 | 5 |
| 3 | 5 | 5 | 5 | 4 |
| 4 | 5 | 5 | 5 | 3 |
| 5 | 5 | 5 | 4 | 2 |

As can be seen from Table 2, compared with Comparative Embodiment 1 which only uses the core component, Embodiment 35, Embodiment 37, and Embodiment 40 of the present disclosure which use a combination of the fixing component and the core component, have better wearing firmness, and the use of a fixator as the fixing component provides higher wearing firmness than the use of a fixing layer as the fixing component.

### Test Embodiment 2

The oral drug delivery devices of Embodiment 35, Embodiment 37, Embodiment 40, and Comparative Embodiment 1 are tested for wearing comfort, and the wearing comfort scores are shown in the following Table 3: wherein, 1 score is extremely uncomfortable, there is obvious foreign matter sensation or irritation that is unacceptable; 2 score is uncomfortable, there is relatively obvious foreign matter sensation or irritation that is less acceptable; 3 score, three is foreign matter sensation or irritation that has a slight impact on the oral function, but is acceptable; 4-score is relatively comfortable, there is no obvious foreign matter sensation or irritation that has essentially no impact on oral function; and 5 score is very comfortable, there is almost no foreign matter sensation or irritation that has no impact on the oral function.

**Table 3**

| Subject | Embodiment 35 | Embodiment 37 | Embodiment 40 | Comparative Embodiment 1 |
|---|---|---|---|---|
| 1 | 3 | 4 | 4 | 3 |
| 2 | 4 | 5 | 5 | 5 |
| 3 | 3 | 4 | 4 | 3 |
| 4 | 4 | 5 | 4 | 3 |
| 5 | 3 | 4 | 4 | 2 |

As can be seen from Table 3, compared with Comparative Embodiment 1 which only uses the core component, Embodiment 35, Embodiment 37, and Embodiment 40 of the present disclosure which use a combination of the fixing component and the core component have better wearing comfort.

### Test Embodiment 3

Embodiment 35, Embodiment 37, Embodiment 40 and Comparative Embodiment 1 are tested for the degree of influence on facial appearance, and the scores are shown in the following Table 4: wherein, 1 score has the greatest impact on facial appearance and is unacceptable; 2 score has relatively great impact on facial appearance and is less unacceptable; 3 score has an impact on facial appearance, but is acceptable; 4 score has little impact on facial appearance; 5 score almost has no difference on facial appearance before and after wearing.

**Table 4**

| Subject | Embodiment 35 | Embodiment 37 | Embodiment 40 | Comparative Embodiment 1 |
|---|---|---|---|---|
| 1 | 4 | 5 | 5 | 5 |
| 2 | 5 | 5 | 5 | 5 |
| 3 | 4 | 4 | 4 | 4 |
| 4 | 4 | 4 | 4 | 4 |
| 5 | 4 | 4 | 4 | 4 |

As can be seen from the above table 4, compared with Comparative Embodiment 1 which only uses the core component, Embodiment 35, Embodiment 37, and Embodiment 40 of the present disclosure which use a combination of the fixing component and the core component have similar impact on facial appearance when wearing an oral drug delivery device, that is, the oral drug delivery device of the present embodiment has a similar wearing aesthetics compared to the comparative embodiment.

The coating membrane involved in the following embodiments and comparative embodiments has a tensile strength of about 1-10 Mpa; a break elongation of about 1.1-2.0; and an average thickness of about 100±8 µm to 200±10 µm. Hydroxypropyl cellulose with a weight average molecular weight of 80,000 can be hydroxypropyl cellulose EXF, with a Brookfield viscosity of about 300-600 mPa.s and a concentration of about 10%.

### Embodiment 47 Preparation of an osmotic pump tablet

The osmotic pump tablet of this embodiment uses levodopa as the active pharmaceutical ingredient and is prepared according to the following steps:

### (1) Preparation of a drug-containing layer

Based on the total weight of the drug-containing layer, take 63.0 wt% of levodopa, 10.0 wt% of hydroxypropyl cellulose with a weight average molecular weight of 80,000, 5.0 wt% of povidone (K29/32), 10.0 wt% of poloxamer (P407), 10.0 wt% of sorbitol, 0.9 wt% of aspartame, 0.1 wt% of mint flavor and 0.5 wt% of magnesium stearate. The above ingredients are each passed through a 40-mesh stainless steel sieve, and then dry granulated; passed through a 1.2 mm stainless steel sieve to further granulate, and then mixed with 0.5 wt% of magnesium stearate to obtain drug-containing layer granules.

### (2) Preparation of a push layer

Based on the total weight of the push layer, take 49.0 wt% of sodium carboxymethyl cellulose (7H4XF), 30.0 wt% of sorbitol, 20.0 wt% of hydroxypropyl cellulose (EXF) and 0.5 wt% of iron oxide red; the above ingredients are each passed through a 40-mesh stainless steel sieve, and then dry granulated; passed through a 1.2 mm stainless steel sieve to further granulate, and then mixed with 0.5 wt% of magnesium stearate to obtain push layer granules.

### (3) Preparation of a bi-layer tablet core

The drug-containing layer granules and the push layer granules are pressed into a bi-layer tablet core, and the tablet pressing die is a 21*9.5 mm capsule-type punch. 1047.6 mg of the drug-containing layer granules are added to the punch of the tablet press and compacted, then 360 mg of the push layer granules are added to the punch, and the two layers of granules are pressed into a contacted bi-layer tablet core by the tablet press. The final bi-layer tablet core comprises about 660 mg of levodopa.

### (4) Preparation of an osmotic pump tablet

The bi-layer tablet core is coated with a coating membrane. The raw material composition of the coating membrane includes 55 wt% of cellulose acetate and 45 wt% of copolyvidone VA 64 by weight percentage, wherein the cellulose acetate is cellulose acetate with 39.8 wt% of acetyl. The composition is dissolved in acetone to obtain a 4 % of solution. Using a pot coater to spray the solution onto the bi-layer tablet core to form a coating membrane to obtain an osmotic pump tablet. Based on the obtained bi-layer osmotic pump tablet, the content of the coating membrane is 6.5 wt%. Using a laser or machine to drill a 1.0 mm drug-release orifice in the coating membrane.

The release profile of the final dosage form was measured using a USP I paddle method in simulated saliva. FIG. 33 shows the release profiles of LD. The osmotic pump tablet of this embodiment can be kept in the oral cavity, so that 85% of the active pharmaceutical ingredient(s) in the osmotic pump tablet is released for about 8 hours, and the drug released in the oral cavity can be continuously swallowed into the gastrointestinal tract, providing continuous and stable drug absorption and blood concentration. The retention amount of the active pharmaceutical ingredient(s) at the release site is less than 10%.

### Embodiment 48 Preparation of an osmotic pump tablet

The steps of Embodiment 47 are repeated in this embodiment, except that:
(1) The weight of the drug-containing layer is 1309.5 mg, the weight of the push layer is 451.6 mg, and the tablet pressing die is a 21*12 mm capsule-type punch.
(2) The coating membrane comprises 50 wt% of cellulose acetate membrane and 50 wt% of Copovidone VA64. Based on the obtained osmotic pump tablet, the content of the coating membrane is 6.0 wt%.

The release profile of the final dosage form was measured using a USP I paddle method in simulated saliva. FIG. 34 shows the release profiles of LD. The osmotic pump tablet of this embodiment can be kept in the oral cavity, so that 85% of the active pharmaceutical ingredient(s) in the osmotic pump tablet is released for about 8 hours, and the drug released in the oral cavity can be continuously swallowed into the gastrointestinal tract, providing continuous and stable drug absorption and blood concentration. The retention amount of the active pharmaceutical ingredient(s) at the release site is less than 10%.

### Embodiment 49 Preparation of an osmotic pump tablet;

The osmotic pump tablet of this embodiment uses levodopa as the active pharmaceutical ingredient(s). The operation of Embodiment 47 is repeated in this embodiment, and the differences are that:

### (1) Preparation of a drug-containing layer

Based on the total weight of the drug-containing layer, take 58.0 wt% of levodopa, 15.0 wt% of hydroxypropyl cellulose with a weight average molecular weight of 80,000, 5.0 wt% of povidone (K29/32), 5.0 wt% of poloxamer (P407), 15.0 wt% of sorbitol, 0.9 wt% of aspartame and 0.1 wt% of mint flavor. The above ingredients are each passed through a 40-mesh stainless steel sieve, and then wet granulated, and dried in a fluidized bed; passed through a 1.2 mm stainless steel sieve to further granulate, and then mixed with 1 wt% magnesium stearate to obtain drug-containing layer granules.

### (2) Preparation of a push layer

Based on the total weight of the push layer, take 68.5 wt% of sodium carboxymethyl cellulose (7H4XF), 10.0 wt% of sorbitol, 20.0 wt% of hydroxypropyl cellulose (EXF) and 0.5 wt% of iron oxide red; the above ingredients are each passed through a 40-mesh stainless steel sieve, and then dry granulated; pass through a 1.2 mm stainless steel sieve to further granulate, and then mixed with 0.5 wt% of colloidal silica and 0.5 wt% of magnesium stearate to obtain push layer granules.

### (3) Preparation of a bi-layer tablet core and an osmotic pump tablet

The weight of the drug-containing layer is 1484.1 mg, the weight of the push layer is 510 mg, and the tablet pressing die is a 21*12 mm capsule-type punch. The coating membrane comprises 60 wt% of cellulose acetate membrane and 40 wt% of Copovidone VA64. Based on the obtained bi-layer osmotic pump tablet, the content of the coating membrane is 6.5 wt%.

The release profile of the final dosage form was measured using a USP I paddle method in simulated saliva. The osmotic pump tablet is combined with the oral retention device of Embodiment 30 and then fixed on the matching teeth in the oral cavity. FIG. 35 shows the release profiles of LD. The release duration of 85% of the active pharmaceutical ingredient(s) in the osmotic pump tablet is approximately 16 hours. And the drug released in the oral cavity is continuously swallowed into the gastrointestinal tract to provide sustained and stable drug absorption and blood drug concentration. The retention amount of the active pharmaceutical ingredient(s) at the release site is less than 10%.

### Embodiment 50 Preparation of an osmotic pump tablet

This embodiment is prepared according to the following steps:

### (1) Preparation of a drug-containing layer

Based on the total weight of the drug-containing layer, take 58.0 wt% of levodopa, 15.0 wt% of hydroxypropyl cellulose with a weight average molecular weight of 80,000, 5.0 wt% of povidone (K29/32), 5.0 wt% of poloxamer (P407), 15.0 wt% of sorbitol, 0.9 wt% of aspartame and 0.1 wt% of mint flavor. The above ingredients are each passed through a 40-mesh stainless steel sieve, and then wet granulated, and dried in a fluidized bed; passed through a 1.2 mm stainless steel sieve to further granulate, and then mixed with 1 wt% of magnesium stearate to obtain drug-containing layer granules.

### (2) Preparation of a push layer

Based on the total weight of the push layer, take 68.5 wt% of sodium carboxymethyl cellulose (7H4XF), 10.0 wt% of sorbitol, 20.0 wt% of hydroxypropyl cellulose (EXF) and 0.5 wt% of iron oxide red; the above ingredients are each passed through a 40-mesh stainless steel sieve, and then dry granulated; passed through a 1.2 mm stainless steel sieve to further granulate, and then mixed with 0.5 wt% of colloidal silica and 0.5 wt% of magnesium stearate to obtain push layer granules.

### (3) Preparation of a bi-layer tablet core

The drug-containing layer and push layer granules are pressed into a bi-layer tablet core, and the tablet pressing die is a 7.5 mm round-type punch. 758.6 mg of the drug-containing layer granules are added to the punch of the tablet press and compacted, then 379.3 mg of the push layer granules are added to the punch, and the two layers of granules are pressed into a contacted bi-layer tablet core by the tablet press. The final bi-layer tablet core comprises 440 mg of levodopa.

### (4) Preparation of an osmotic pump tablet

The bi-layer tablet core is coated with a coating membrane. The raw material composition of the coating membrane comprises 50 wt% of cellulose acetate, 5 wt% of polyethylene glycol 400 and 45 wt% of copovidone VA 64 by weight percentage, wherein the cellulose acetate is a cellulose acetate membrane with 39.8 wt% of acetyl. The coating membrane raw material composition is dissolved in acetone to obtain a 4 % of solution. Using a pot coating machine to spray the solution onto the bi-layer tablet core to form a coating membrane to obtain an osmotic pump tablet. Based on the obtained osmotic pump tablet, the content of the coating membrane is 4.5 wt%. Using a laser or machine to drill a 1.0 mm drug-release orifice in the coating membrane.

The release profile of the final dosage form was measured using a USP I paddle method in simulated saliva. FIG. 36 shows the release profiles of LD. The osmotic pump tablet of this embodiment can be kept in the oral cavity, so that 85% of the active pharmaceutical ingredient(s) in the osmotic pump tablet is released for about 5 hours, and the drug released in the oral cavity can be continuously swallowed into the gastrointestinal tract, providing continuous and stable drug absorption and blood concentration. The retention amount of the active pharmaceutical ingredient(s) at the release site is less than 10%.

### Embodiment 51 Preparation of an osmotic pump tablet;

The steps of Embodiment 50 are repeated in this embodiment, except that: the weight of the drug-containing layer is 569.2 mg, the weight of the push layer is 284.6 mg, and the tablet pressing die is a 7 mm round-type punch. Based on the obtained bi-layer osmotic pump tablet, the content of the coating membrane is 6.0 wt%.

The release profile of the final dosage form was measured using a USP I paddle method in simulated saliva. The osmotic pump tablet is combined with the oral retention device of Embodiment 30 and then fixed on the matching teeth in the oral cavity. FIG. 37 shows the release profiles of LD. The release duration of 85% of the active pharmaceutical ingredient(s) in the osmotic pump tablet is approximately 5 hours. And the drug released in the oral cavity is continuously swallowed into the gastrointestinal tract to provide sustained and stable drug absorption and blood drug concentration. The retention amount of the active pharmaceutical ingredient(s) at the release site is less than 10%.

### Embodiment 52 Preparation of an osmotic pump tablet

The steps of Embodiment 50 are repeated in this embodiment, except that: the weight of the drug-containing layer is 379.3 mg, the weight of the push layer is 189.7 mg, and the tablet pressing die is a 6 mm round-type punch. Based on the obtained bi-layer osmotic pump tablet, the content of the coating membrane is 9.0 wt%.

The release profile of the final dosage form was measured using a USP I paddle method in simulated saliva. FIG. 38 shows the release profiles of LD. The osmotic pump tablet is combined with the oral retention device of Embodiment 30 and then fixed on the matching teeth in the oral cavity. The release duration of 85% of the active pharmaceutical ingredient(s) in the osmotic pump tablet is approximately 5 hours. And the drug released in the oral cavity is continuously swallowed into the gastrointestinal tract to provide sustained and stable drug absorption and blood drug concentration. The retention amount of the active pharmaceutical ingredient(s) at the release site is less than 10%.

### Embodiment 53 Preparation of an osmotic pump tablet;

The osmotic pump tablet of this embodiment is prepared according to the following steps:

### (1) Preparation of a drug-containing layer

Based on the total weight of the drug-containing layer, take 58.0 wt% of levodopa, 15.0 wt% of hydroxypropyl cellulose with a weight average molecular weight of 80,000, 5.0 wt% of povidone (K29/32), 5.0 wt% of poloxamer (P407), 15.0 wt% of sorbitol, 0.9 wt% of aspartame and 0.1 wt% of mint flavor. The above ingredients are each passed through a 40-mesh stainless steel sieve, and then wet granulated, and dried in a fluidized bed; passed through a 1.2 mm stainless steel sieve to further granulate, and then mixed with 1 wt% of magnesium stearate to obtain drug-containing layer granules.

### (2) Preparation of a push layer

Based on the total weight of the push layer, take 68.5 wt% of sodium carboxymethyl cellulose (7H4XF), 10.0 wt% of sorbitol, 20.0 wt% of hydroxypropyl cellulose (EXF) and 0.5 wt% of iron oxide red; the above ingredients are each passed through a 40-mesh stainless steel sieve, and then dry granulated; passed through a 1.2 mm stainless steel sieve to further granulate, and then mixed with 0.5 wt% of colloidal silica and 0.5 wt% of magnesium stearate to obtain push layer granules.

### (3) Preparation of a three-layer tablet core

The drug-containing layer, isolation layer material and push layer granules are pressed into a three-layer tablet core, and the tablet pressing die is a 7.5 mm round-type punch. 758.6 mg of the drug-containing layer granules are added to the punch of the tablet press and compacted, then 50 mg of isolation layer material of ethylcellulose N10 are added to the punch and compacted, and finally 379.3 mg of push layer granules are added to the punch, they are pressed into a contacted three-layer tablet core by the tablet press. The final three-layer tablet core comprises 440 mg of levodopa.

### (4) Preparation of an osmotic pump tablet

The three-layer tablet core is coated with a coating membrane. The raw material composition of the coating membrane comprises 50 wt% of cellulose acetate, 5 wt% of polyethylene glycol 400 and 45 wt% of Copovidone VA64 by weight percentage, wherein the cellulose acetate is cellulose acetate with 39.8 wt% of acetyl. The coating membrane raw material composition is dissolved in acetone to obtain a 4 % of solution. Using a pot coating machine to spray the solution onto the three-layer tablet core to form a coating membrane to obtain an osmotic pump tablet. Based on the obtained osmotic pump tablet, the content of the coating membrane is 4.5 wt%. Using a laser or machine to drill a 1.0 mm drug-release orifice in the coating membrane.

The release profile of the final dosage form was measured using a USP I paddle method in simulated saliva. The osmotic pump tablet of this embodiment can be kept in the oral cavity, so that 85% of the active pharmaceutical ingredient(s) in the osmotic pump tablet is released for about 5 hours, and the drug released in the oral cavity can be continuously swallowed into the gastrointestinal tract, providing continuous and stable drug absorption and blood concentration. The retention amount of the active pharmaceutical ingredient(s) at the release site is less than 10%.

### Embodiment 54 Preparation of an osmotic pump tablet

The osmotic pump tablet is designed, shaped and adapted for use with Embodiment 30, the osmotic pump tablet comprising levodopa and carbidopa monohydrate, is prepared according to the following steps:
(1) Preparation of a drug-containing layer which contains two parts. Based on the total weight of the drug-containing layer, the first part comprises 54.9 wt% of levodopa, 15.0 wt% of hydroxypropyl cellulose with a weight average molecular weight of 80,000, 5.0 wt% of povidone (K29/32), 3.07 wt% of sorbitol and 0.9 wt% of aspartame, each of which is passed through a 40-mesh stainless steel sieve, and then wet granulated, and dried in a fluidized bed; and passed through a 1.2 mm stainless steel sieve to further granulate. The second part comprises 3.16 wt% of carbidopa monohydrate, 11.8 wt% of sorbitol and 5.0 wt% of poloxamer P407, each of which is passed through a 40-mesh stainless steel, then hot-melt granulated, and passed through a 1.2 mm stainless steel sieve to further granulate. After mixing the first part and the second part of the granules, 0.1 wt% of mint flavor, 0.1 wt% of dibutylhydroxytoluene and 1.0 wt% of magnesium stearate are further added and mixed to obtain drug-containing layer granules.
(2) Preparation of a push layer
   Based on the total weight of the push layer, take 68.5 wt% of sodium carboxymethyl cellulose (7H4XF), 10.0 wt% of sorbitol, 20.0 wt% of hydroxypropyl cellulose (EXF) and 0.5 wt% of iron oxide red; the above ingredients are each passed through a 40-mesh stainless steel sieve, and then dry granulated; passed through a 1.2 mm stainless steel sieve to further granulate, and then mixed with 0.5 wt% of colloidal silica and 0.5 wt% of magnesium stearate to obtain push layer granules.
(3) Preparation of a bi-layer tablet core
   The drug-containing layer granules and push layer granules are pressed into a bi-layer tablet core, and the tablet pressing die is a 7.0 mm round-type punch. 563.9 mg of the drug-containing layer granules are added to the punch of the tablet press and compacted, then 282.0 mg of the push layer granules are added to the punch, and the two layers of granules are pressed into a contacted bi-layer tablet core by the tablet press. The final bi-layer tablet core comprises 309.4 mg of levodopa and 16.5 mg of carbidopa.
(4) Preparation of an osmotic pump tablet
   The bi-layer tablet core is coated with a coating membrane. The raw material composition of the coating membrane comprises 50 wt% cellulose acetate with acetyl group content of 39.8 wt%, 5 wt% of polyethylene glycol 400 and 45 wt% of Copovidone VA 64 by weight percentage. The raw material composition is dissolved in acetone to obtain a 4 % of solution. Using a pot coating machine to spray the solution onto the bi-layer tablet core to form a coating membrane to obtain an osmotic pump tablet. Based on the obtained osmotic pump tablet, the content of the coating membrane is 7.0 wt%. Using a laser or machine to drill a 1.0 mm drug-release orifice in the coating membrane.
(5) Preparation of an overcoat
   The raw material composition of the overcoat comprises 49.2 wt% of levodopa, 28.3 wt% of carbidopa, 20.0 wt% of hydroxypropyl cellulose, 1.47 wt% of dibutylhydroxytoluene and 1.0 % of triethyl citrate. These components are prepared as a 10 wt% of solution in ethanol, and the solution is sprayed onto the osmotic pump tablet by using a pan coater. The overcoat layer comprises 18.75 mg of levodopa and 10.8 mg of carbidopa.

The bi-layer osmotic pump tablet of this embodiment can be kept in the oral cavity, 85% of the active pharmaceutical ingredient(s) in the osmotic pump tablet is released for about 8 hours, and the drug released in the oral cavity can be continuously swallowed into the gastrointestinal tract, providing continuous and stable drug absorption and blood concentration. The retention amount of the active pharmaceutical ingredient(s) at the release site is less than 10%.

### Embodiment 55 Preparation of an osmotic pump tablet

The osmotic pump tablet is designed, shaped and adapted for use with Embodiment 30, the osmotic pump tablet comprising levodopa and carbidopa monohydrate, is prepared according to the following steps:
(1) Preparation of a drug-containing layer which contains two parts. Based on the total weight of the drug-containing layer, the first part comprises 54.9 wt% of levodopa, 15.0 wt% of hydroxypropyl cellulose with a weight average molecular weight of 80,000, 8.07 wt% of mannitol, and 0.9 wt% of aspartame, each of which is passed through a 40-mesh stainless steel sieve, and then wet granulated, and dried in a fluidized bed; and passed through a 1.2 mm stainless steel sieve to further granulate. The second part comprises 3.16 wt% of carbidopa monohydrate, 11.8 wt% of mannitol and 5.0 wt% of poloxamer (P407), each of which is passed through a 40-mesh stainless steel, then hot-melt granulated, and passed through a 1.2 mm stainless steel sieve to further granulate. After mixing the first part and the second part of the granules, 0.1 wt% of mint flavor, 0.1 wt% of dibutylhydroxytoluene and 1.0 wt% of magnesium stearate are further added and mixed to obtain drug-containing layer granules.
(2) Preparation of a push layer
   Based on the total weight of the push layer, take 68.5 wt% of sodium carboxymethyl cellulose (7H4XF), 10.0 wt% of sorbitol, 20.0 wt% of hydroxypropyl cellulose (EXF) and 0.5 wt% of iron oxide red; these ingredients are each passed through a 40-mesh stainless steel sieve, and then dry granulated; passed through a 1.2 mm stainless steel sieve to further granulate, and then mixed with 0.5 wt% of colloidal silica and 0.5 wt% of magnesium stearate to obtain push layer granules.
(3) Preparation of a bi-layer tablet core
   The drug-containing layer granules and push layer granules are pressed into a bi-layer tablet core, and the tablet pressing die is a 7.0 mm round-type punch. 563.9 mg of drug-containing layer granules were added to a punch of a tablet press and compacted, then 282.0 mg of osmotic layer granules were added to the punch, and the granules of both layers are pressed with a tablet press into a contacted bi-layer tablet core. The final bi-layer tablet core comprises 309.4 mg of levodopa and 16.5 mg of carbidopa.
(4) Preparation of an osmotic pump tablet
   The bi-layer tablet core is coated with a coating membrane. The raw material composition of the coating membrane comprises 50 wt% cellulose acetate with acetyl group content of 39.8 wt%, 5 wt% of polyethylene glycol 400 and 45 wt% of Copovidone VA 64 by weight percentage. The raw material composition is dissolved in acetone to obtain a 4 % of solution. Using a pan coater to spray the solution onto the bi-layer tablet core to form a coating membrane to obtain an osmotic pump tablet. Based on the obtained osmotic pump tablet, the content of the coating membrane is 7.0 wt%. Using a laser or machine to drill a 1.0 mm drug-release orifice in the coating membrane.
(5) Preparation of an overcoat
   The raw material composition of the overcoat comprises 55.4 wt% of levodopa, 31.9 wt% of carbidopa, 10.0 wt% of hydroxypropyl cellulose (EXF), 1.66 wt% of dibutylhydroxytoluene and 1.0 wt% triethyl citrate. These components are prepared as a 10 wt% of solution in ethanol, and the solution is sprayed onto the extended release tablet by using a pan coater. The overcoat layer comprises 18.75 mg of levodopa and 10.8 mg of carbidopa.

The bi-layer osmotic pump tablet of this embodiment can be kept in the oral cavity, 85% of the active pharmaceutical ingredient(s) in the osmotic pump tablet is released for about 8 hours, and the drug released in the oral cavity can be continuously swallowed into the gastrointestinal tract, providing continuous and stable drug absorption and blood concentration. The retention amount of the active pharmaceutical ingredient(s) at the release site is less than 10%.

### Embodiment 56 Preparation of an osmotic pump tablet

The osmotic pump tablet is designed, shaped and adapted for use with Embodiment 30, the osmotic pump tablet comprising levodopa and carbidopa monohydrate, is prepared according to the following steps:
(1) Preparation of a drug-containing layer which contains two parts. Based on the total weight of the drug-containing layer, the first part comprises 54.9 wt% of levodopa, 15.0 wt% of hydroxypropyl cellulose with a weight average molecular weight of 80,000, 8.07 wt% of mannitol, and 0.9 wt% of aspartame, each of which is passed through a 40-mesh stainless steel sieve, and then wet granulated, and dried in a fluidized bed; and passed through a 1.2 mm stainless steel sieve to further granulate. The second part comprises 3.16 wt% of carbidopa monohydrate, 11.8 wt% of mannitol and 5.0 wt% of poloxamer (P407), each of which is passed through a 40-mesh stainless steel, then hot-melt granulated, and passed through a 1.2 mm stainless steel sieve to further granulate. After mixing the first part and the second part of the granules, 0.1 wt% of mint flavor, 0.1 wt% of dibutylhydroxytoluene and 1.0 wt% of magnesium stearate are further added and mixed to obtain drug-containing layer granules.
(2) Preparation of a push layer
   Based on the total weight of the push layer, take 68.5 wt% of sodium carboxymethyl cellulose (7H4XF), 10.0 wt% of sorbitol, 20.0 wt% of hydroxypropyl cellulose (EXF) and 0.5 wt% of iron oxide red; the above ingredients are each passed through a 40-mesh stainless steel sieve, and then dry granulated; passed through a 1.2 mm stainless steel sieve to further granulate, and then mixed with 0.5 wt% of colloidal silica and 0.5 wt% of magnesium stearate to obtain push layer granules.
(3) Preparation of a bi-layer tablet core
   The drug-containing layer granules and push layer granules are pressed into a bi-layer tablet core, and the tablet pressing die is a 7.0 mm round-type punch. 563.9 mg of drug-containing layer granules were added to a punch of a tablet press and compacted, then 282.0 mg of osmotic layer granules were added to the punch, and the granules of both layers are pressed with a tablet press into a contacted bi-layer tablet core. The final bi-layer tablet core comprises 412.5 mg of levodopa and 23.8 mg of carbidopa.
(4) Preparation of an osmotic pump tablet
   The bi-layer tablet core is coated with a coating membrane. The raw material composition of the coating membrane comprises 50 wt% cellulose acetate with acetyl group content of 39.8 wt%, 5 wt% of polyethylene glycol 400 and 45 wt% of Copovidone VA 64 by weight percentage. The composition is dissolved in acetone to obtain a 4 % solution, and using a pan coater to spray the solution onto the bi-layer tablet core to form a coating membrane. Based on the obtained osmotic pump tablet, the content of the coating membrane is 5.5 wt%. Using a laser or machine to drill a 1.0 mm drug-release orifice in the coating membrane.

The bi-layer osmotic pump tablet of this embodiment can be kept in the oral cavity, 85% of the active pharmaceutical ingredient(s) in the osmotic pump tablet is released for about 8 hours, and the drug released in the oral cavity can be continuously swallowed into the gastrointestinal tract, providing continuous and stable drug absorption and blood concentration. The retention amount of the active pharmaceutical ingredient(s) at the release site is less than 10%.

### Embodiment 57 Capsule-type tablet with dose strength 25 mg/100 mg of Carbidopa/Levodopa

The prescription is shown in Table 5:

**Table 5**

| (1) | Drug-containing layer | mg/tablet | wt% |
|---|---|---|---|
| Drug-containing layer (Total weight 166.69 mg) | Levodopa | 75.0 | 45.0 |
| | Hydroxypropyl cellulose (EXF) | 51.7 | 31.0 |
| | Povidone (K29/32) | 8.33 | 5.00 |
| | Mannitol | 30.0 | 18.0 |
| | Magnesium stearate | 1.66 | 1.00 |

| (2) | Push layer | mg/tablet | wt% |
|---|---|---|---|
| Push layer (Total weight 83.27 mg) | Sodium carboxymethyl cellulose | 45.8 | 55.0 |
| | Sorbitol | 28.3 | 34.0 |
| | Hydroxypropyl cellulose (EXF) | 8.33 | 10.0 |
| | Iron oxide red | 0.42 | 0.50 |
| | Magnesium stearate | 0.42 | 0.50 |

| (3) | Coating membrane (solid content is 4.0%, solvent is acetone: dehydrated ethanol = 90%:10%, V:V) | mg/tablet | wt% |
|---|---|---|---|
| Coating membrane (Total weight 25.05 mg) | Cellulose acetate | 16.3 | 65.0 |
| | Copovidone VA64 | 8.75 | 35.0 |

| (4) | Drug-containing immediate release overcoat (solid content is 10.0%, solvent is dehydrated ethanol) | mg/tablet | wt% |
|---|---|---|---|
| Drug-containing immediate release overcoat (Total weight 57.78 mg) | Carbidopa monohydrate | 27.0 | 46.7 |
| | Levodopa | 25.0 | 43.3 |
| | Hydroxypropyl cellulose (EXF) | 5.78 | 10.0 |

### Methods of preparation:

### (1) Preparation of a drug-containing layer

Based on the total weight of the drug-containing layer, take 45.0 wt% of levodopa, 31.0 wt% of hydroxypropyl cellulose (EXF), 5.0 wt% of povidone (K29/32) and 18.0 wt% of mannitol. The above ingredients are dry granulated; passed through a 16-mesh stainless steel sieve to further granulate, and then mixed with 1.0 wt% of magnesium stearate to obtain drug-containing layer granules.

### (2) Preparation of a push layer

Based on the total weight of the push layer, take 55.0 wt% of sodium carboxymethyl cellulose, 34.0 wt% of sorbitol, 10.0 wt% of hydroxypropyl cellulose (EXF) and 0.5 wt% of iron oxide red; the above ingredients are dry granulated; passed through a 16-mesh stainless steel sieve to further granulate, and then mixed with 0.5 wt% of magnesium stearate to obtain push layer granules.

### (3) Preparation of a bi-layer tablet core

The drug-containing layer granules and the push layer granules are pressed into a bi-layer tablet core using a punch size of φ8 mm (shallow concave). Approximately 166.69 mg of the drug-containing layer granules obtained from step (1) are added to the punch of the tablet press and compacted, then approximately 83.27 mg of the push layer granules obtained from step (2) are added to the punch, and the two layers of granules are pressed into a contacted bi-layer tablet core (approximately 249.96 mg) by the tablet press.

### (4) Preparation of an osmotic pump tablet

The bi-layer tablet core is coated with a coating membrane. The raw material composition of the coating membrane includes 65.0 wt% of cellulose acetate and 35.0 wt% of copovidone VA 64 by weight percentage, wherein the cellulose acetate is cellulose acetate with 39.8 wt% of acetyl. The composition is dissolved in acetone/dehydrated ethanol (V:V=9:1), the solid content is 4.0%. Using a pot coater to spray the solution onto the bi-layer tablet core to form a coating membrane, which constitutes approximately 10.0% of the weight of the bi-layer tablet core.

Subsequently, the drug-containing immediate release overcoat is applied to further cover the coating membrane. The raw material composition of the drug-containing immediate release overcoat, in terms of weight percentage, comprises 46.7 wt% of carbidopa monohydrate, 43.3 wt% of levodopa, and 10.0 wt% of hydroxypropyl cellulose (EXF). This composition is dissolved in dehydrated ethanol to obtain the solution with a solid content of 10.0%. Using a pot coater to spray the solution onto the coating membrane to form a drug-containing immediate release overcoat to obtain an osmotic pump tablet. Using a laser or machine to drill a 1.0 mm drug-release orifice in the coating membrane. In this embodiment, the dose strength of the osmotic pump tablet is 25 mg/100 mg (carbidopa/levodopa) and it is a round, biconvex tablet.

Take the sample and perform the dissolution and release test according to the methods specified in General Rule of Chinese Pharmacopoeia <0931> (first method) and USP <711> (first method). Use 900 mL of 0.1 mol/L hydrochloric acid solution as the dissolution medium, with a rotation speed of 75 revolutions per minute, and proceed as specified. Samples are taken at 0.5, 1, 1.5, 2, 2.5, 3, 4, 5, 6, and 8 hours.

The active pharmaceutical ingredient levodopa in this osmotic pump tablet is released for about 6 hours (as shown in FIG. 39). The active pharmaceutical ingredient carbidopa in the drug-containing immediate release overcoat is released at 104% at 0.5 hours, with a standard deviation (SD) value of 10.5%.

### Embodiment 58 Capsule-type tablet with dose strength 25 mg/100 mg of Carbidopa/Levodopa

The prescription is shown in Table 6:

**Table 6**

| (1) | Drug-containing layer | mg/tablet | wt% |
|---|---|---|---|
| Drug-containing layer (Total weight 166.69 mg) | Levodopa | 75.0 | 45.0 |
| | Hydroxypropyl cellulose (EXF) | 51.7 | 31.0 |
| | Povidone (K29/32) | 8.33 | 5.00 |
| | Mannitol | 27.5 | 16.5 |
| | Magnesium stearate | 3.33 | 2.00 |
| | Colloidal silica | 0.83 | 0.50 |

| (2) | Push layer | mg/tablet | wt% |
|---|---|---|---|
| Push layer (Total weight 83.34 mg) | Sodium carboxymethyl cellulose | 40.8 | 49.0 |
| | Sorbitol | 25.0 | 30.0 |
| | Hydroxypropyl cellulose (EXF) | 16.7 | 20.0 |
| | Iron oxide red | 0.42 | 0.50 |
| | Magnesium stearate | 0.42 | 0.50 |

| (3) | Coating membrane (solid content is 4.0%, solvent is acetone: dehydrated ethanol = 90%:10%, V:V) | mg/tablet | wt% |
|---|---|---|---|
| Coating membrane (Total weight 25.05 mg) | Cellulose acetate | 16.3 | 65.0 |
| | Copovidone VA64 | 8.75 | 35.0 |

| (4) | Drug-containing immediate release overcoat (solid content is 10.0%, solvent is dehydrated ethanol) | mg/tablet | wt% |
|---|---|---|---|
| Drug-containing immediate release overcoat (Total weight 57.78 mg) | Carbidopa monohydrate | 27.0 | 46.7 |
| | Levodopa | 25.0 | 43.3 |
| | Hydroxypropyl cellulose (EXF) | 5.78 | 10.0 |

### Methods of preparation:

### (1) Preparation of a drug-containing layer

Based on the total weight of the drug-containing layer, take 45.0 wt% of levodopa, 31.0 wt% of hydroxypropyl cellulose (EXF), 5.0 wt% of povidone (K29/32), 16.5 wt% of mannitol and 0.5 wt% of colloidal silica. The above ingredients are dry granulated; passed through a 16-mesh stainless steel sieve to further granulate, and then mixed with 2.0 wt% of magnesium stearate to obtain drug-containing layer granules.

### (2) Preparation of a push layer

Based on the total weight of the push layer, take 49.0 wt% of sodium carboxymethyl cellulose, 30.0 wt% of sorbitol, 20.0 wt% of hydroxypropyl cellulose (EXF) and 0.5 wt% of iron oxide red; the above ingredients are dry granulated; passed through a 16-mesh stainless steel sieve to further granulate, and then mixed with 0.5 wt% of magnesium stearate to obtain push layer granules.

### (3) Preparation of a bi-layer tablet core

The drug-containing layer granules and the push layer granules are pressed into a bi-layer tablet core using a punch size of φ8 mm (shallow concave). Approximately 166.69 mg of the drug-containing layer granules obtained from step (1) are added to the punch of the tablet press and compacted, then approximately 83.34 mg of the push layer granules obtained from step (2) are added to the punch, and the two layers of granules are pressed into a contacted bi-layer tablet core (approximately 250.03 mg) by the tablet press.

### (4) Preparation of an osmotic pump tablet

The bi-layer tablet core is coated with a coating membrane. The raw material composition of the coating membrane includes 65.0 wt% of cellulose acetate and 35.0 wt% of copovidone VA 64 by weight percentage, wherein the cellulose acetate is cellulose acetate with 39.8 wt% of acetyl. The composition is dissolved in acetone/dehydrated ethanol (V:V=9:1), the solid content is 4.0%. Using a pot coater to spray the solution onto the bi-layer tablet core to form a coating membrane, which constitutes approximately 10.0% of the weight of the bi-layer tablet core.

Subsequently, the drug-containing immediate release overcoat is applied to further cover the coating membrane. The raw material composition of the drug-containing immediate release overcoat, in terms of weight percentage, comprises 46.7 wt% of carbidopa monohydrate, 43.3 wt% of levodopa, and 10.0 wt% of hydroxypropyl cellulose (EXF). This composition is dissolved in dehydrated ethanol to obtain the solution with a solid content of 10.0%. Using a pot coater to spray the solution onto the coating membrane to form a drug-containing immediate release overcoat to obtain an osmotic pump tablet. Using a laser or machine to drill a 1.0 mm drug-release orifice in the coating membrane. In this embodiment, the dose strength of the osmotic pump tablet is 25 mg/100 mg (carbidopa/levodopa) and it is a round, biconvex tablet.

Take the sample and perform the dissolution and release test according to the methods specified in General Rule of Chinese Pharmacopoeia <0931> (first method) and USP <711> (first method). Use 900 mL of 0.1 mol/L hydrochloric acid solution as the dissolution medium, with a rotation speed of 75 revolutions per minute, and proceed as specified. Samples are taken at 0.5, 1, 1.5, 2, 2.5, 3, 4, 5, 6, and 8 hours.

The active pharmaceutical ingredient levodopa in this osmotic pump tablet is released for about 6 hours (as shown in FIG. 40). The active pharmaceutical ingredient carbidopa in the drug-containing immediate release overcoat is released at 104% at 0.5 hours, with a standard deviation (SD) value of 10.5%.

### Embodiment 59 Capsule-type tablet with dose strength 25 mg/150 mg of Carbidopa/Levodopa

The prescription is shown in Table 7:

**Table 7**

| (1) | Drug-containing layer | mg/tablet | wt% |
|---|---|---|---|
| Drug-containing layer (Total weight 250 mg) | Levodopa | 112.5 | 45.0 |
| | Hydroxypropyl cellulose (EXF) | 77.5 | 31.0 |
| | Povidone (K29/32) | 12.5 | 5.00 |
| | Mannitol | 45.0 | 18.0 |
| | Magnesium stearate | 2.50 | 1.00 |

| (2) | Push layer | mg/tablet | wt% |
|---|---|---|---|
| Push layer (Total weight 125.06 mg) | Sodium carboxymethyl cellulose | 68.8 | 55.0 |
| | Sorbitol | 42.5 | 34.0 |
| | Hydroxypropyl cellulose (EXF) | 12.5 | 10.0 |
| | Iron oxide red | 0.63 | 0.50 |
| | Magnesium stearate | 0.63 | 0.50 |

| (3) | Coating membrane (solid content is 4.0%, solvent is acetone: dehydrated ethanol = 90%:10%, V:V) | mg/tablet | wt% |
|---|---|---|---|
| Coating membrane (Total weight 30 mg) | Cellulose acetate | 19.5 | 65.0 |
| | Copovidone VA64 | 10.5 | 35.0 |

| (4) | Drug-containing immediate release overcoat (solid content is 10.0%, solvent is dehydrated ethanol) | mg/tablet | wt% |
|---|---|---|---|
| Drug-containing immediate release overcoat (Total weight 71.67 mg) | Carbidopa monohydrate | 27.0 | 37.7 |
| | Levodopa | 37.5 | 52.3 |
| | Hydroxypropyl cellulose (EXF) | 7.17 | 10.0 |

### Methods of preparation:

### (1) Preparation of a drug-containing layer

Based on the total weight of the drug-containing layer, take 45.0 wt% of levodopa, 31.0 wt% of hydroxypropyl cellulose (EXF), 5.0 wt% of povidone (K29/32) and 18.0 wt% of mannitol. The above ingredients are dry granulated; passed through a 16-mesh stainless steel sieve to further granulate, and then mixed with 1.0 wt% of magnesium stearate to obtain drug-containing layer granules.

### (2) Preparation of a push layer

Based on the total weight of the push layer, take 55.0 wt% of sodium carboxymethyl cellulose, 34.0 wt% of sorbitol, 10.0 wt% of hydroxypropyl cellulose (EXF) and 0.5 wt% of iron oxide red; the above ingredients are dry granulated; passed through a 16-mesh stainless steel sieve to further granulate, and then mixed with 0.5 wt% of magnesium stearate to obtain push layer granules.

### (3) Preparation of a bi-layer tablet core

The drug-containing layer granules and the push layer granules are pressed into a bi-layer tablet core using a punch size of φ8 mm (shallow concave). Approximately 250 mg of the drug-containing layer granules obtained from step (1) are added to the punch of the tablet press and compacted, then approximately 125.06 mg of the push layer granules obtained from step (2) are added to the punch, and the two layers of granules are pressed into a contacted bi-layer tablet core (approximately 375.06 mg) by the tablet press.

### (4) Preparation of an osmotic pump tablet

The bi-layer tablet core is coated with a coating membrane. The raw material composition of the coating membrane includes 65.0 wt% of cellulose acetate and 35.0 wt% of copovidone VA 64 by weight percentage, wherein the cellulose acetate is cellulose acetate with 39.8 wt% of acetyl. The composition is dissolved in acetone/dehydrated ethanol (V:V=9:1), the solid content is 4.0%. Using a pot coater to spray the solution onto the bi-layer tablet core to form a coating membrane, which constitutes approximately 8.0% of the weight of the bi-layer tablet core.

Subsequently, the drug-containing immediate release overcoat is applied to further cover the coating membrane. The raw material composition of the drug-containing immediate release overcoat, in terms of weight percentage, comprises 37.7 wt% of carbidopa monohydrate, 52.3 wt% of levodopa, and 10.0 wt% of hydroxypropyl cellulose (EXF). This composition is dissolved in dehydrated ethanol to obtain the solution with a solid content of 10.0%. Using a pot coater to spray the solution onto the coating membrane to form a drug-containing immediate release overcoat to obtain an osmotic pump tablet. Using a laser or machine to drill a 1.0 mm drug-release orifice in the coating membrane. In this embodiment, the dose strength of the osmotic pump tablet is 25 mg/150 mg (carbidopa/levodopa) and it is a round, biconvex tablet.

Take the sample and perform the dissolution and release test according to the methods specified in General Rule of Chinese Pharmacopoeia <0931> (first method) and USP <711> (first method). Use 900 mL of 0.1 mol/L hydrochloric acid solution as the dissolution medium, with a rotation speed of 75 revolutions per minute, and proceed as specified. Samples are taken at 0.5, 1, 1.5, 2, 2.5, 3, 4, 5, 6, and 8 hours.

The active pharmaceutical ingredient levodopa in this osmotic pump tablet is released for about 6 hours (as shown in FIG. 41). The active pharmaceutical ingredient carbidopa in the drug-containing immediate release overcoat is released at 96% at 0.5 hours, with a standard deviation (SD) value of 12%.

### Embodiment 60 Capsule-type tablet with dose strength 25 mg/150 mg of Carbidopa/Levodopa

The prescription is shown in Table 8:

**Table 8**

| (1) | Drug-containing layer | mg/tablet | wt% |
|---|---|---|---|
| Drug-containing layer (Total weight 250.05 mg) | Levodopa | 112.5 | 45.0 |
| | Hydroxypropyl cellulose (EXF) | 77.5 | 31.0 |
| | Povidone (K29/32) | 12.5 | 5.00 |
| | Mannitol | 41.3 | 16.5 |
| | Magnesium stearate | 5.00 | 2.00 |
| | Colloidal silica | 1.25 | 0.50 |

| (2) | Push layer | mg/tablet | wt% |
|---|---|---|---|
| Push layer (Total weight 125.06 mg) | Sodium carboxymethyl cellulose | 61.3 | 49.0 |
| | Sorbitol | 37.5 | 30.0 |
| | Hydroxypropyl cellulose (EXF) | 25.0 | 20.0 |
| | Iron oxide red | 0.63 | 0.50 |
| | Magnesium stearate | 0.63 | 0.50 |

| (3) | Coating membrane (solid content is 4.0%, solvent is acetone: dehydrated ethanol = 90%:10%, V:V) | mg/tablet | wt% |
|---|---|---|---|
| Coating membrane (Total weight 30 mg) | Cellulose acetate | 19.5 | 65.0 |
| | Copovidone VA64 | 10.5 | 35.0 |

| (4) | Drug-containing immediate release overcoat (solid content is 10.0%, solvent is dehydrated ethanol) | mg/tablet | wt% |
|---|---|---|---|
| Drug-containing | Carbidopa monohydrate | 27.0 | 37.7 |
| immediate release overcoat (Total weight 71.67 mg) | Levodopa | 37.5 | 52.3 |
| | Hydroxypropyl cellulose (EXF) | 7.17 | 10.0 |

### Methods of preparation:

### (1) Preparation of a drug-containing layer

Based on the total weight of the drug-containing layer, take 45.0 wt% of levodopa, 31.0 wt% of hydroxypropyl cellulose (EXF), 5.0 wt% of povidone (K29/32), 16.5 wt% of mannitol and 0.5 wt% of colloidal silica. The above ingredients are dry granulated; passed through a 16-mesh stainless steel sieve to further granulate, and then mixed with 2.0 wt% of magnesium stearate to obtain drug-containing layer granules.

### (2) Preparation of a push layer

Based on the total weight of the push layer, take 49.0 wt% of sodium carboxymethyl cellulose, 30.0 wt% of sorbitol, 20.0 wt% of hydroxypropyl cellulose (EXF) and 0.5 wt% of iron oxide red; the above ingredients are dry granulated; passed through a 16-mesh stainless steel sieve to further granulate, and then mixed with 0.5 wt% of magnesium stearate to obtain push layer granules.

### (3) Preparation of a bi-layer tablet core

The drug-containing layer granules and the push layer granules are pressed into a bi-layer tablet core using a punch size of φ8 mm (shallow concave). Approximately 250.05 mg of the drug-containing layer granules obtained from step (1) are added to the punch of the tablet press and compacted, then approximately 125.06 mg of the push layer granules obtained from step (2) are added to the punch, and the two layers of granules are pressed into a contacted bi-layer tablet core (approximately 375.11 mg) by the tablet press.

### (4) Preparation of an osmotic pump tablet

The bi-layer tablet core is coated with a coating membrane. The raw material composition of the coating membrane includes 65.0 wt% of cellulose acetate and 35.0 wt% of copovidone VA 64 by weight percentage, wherein the cellulose acetate is cellulose acetate with 39.8 wt% of acetyl. The composition is dissolved in acetone/dehydrated ethanol (V:V=9:1), the solid content is 4.0%. Using a pot coater to spray the solution onto the bi-layer tablet core to form a coating membrane, which constitutes approximately 8.0% of the weight of the bi-layer tablet core.

Subsequently, the drug-containing immediate release overcoat is applied to further cover the coating membrane. The raw material composition of the drug-containing immediate release overcoat, in terms of weight percentage, comprises 37.7 wt% of carbidopa monohydrate, 52.3 wt% of levodopa, and 10.0 wt% of hydroxypropyl cellulose (EXF). This composition is dissolved in dehydrated ethanol to obtain the solution with a solid content of 10.0%. Using a pot coater to spray the solution onto the coating membrane to form a drug-containing immediate release overcoat to obtain an osmotic pump tablet. Using a laser or machine to drill a 1.0 mm drug-release orifice in the coating membrane. In this embodiment, the dose strength of the osmotic pump tablet is 25 mg/150 mg (carbidopa/levodopa) and it is a round, biconvex tablet.

Take the sample and perform the dissolution and release test according to the methods specified in General Rule of Chinese Pharmacopoeia <0931> (first method) and USP <711> (first method). Use 900 mL of 0.1 mol/L hydrochloric acid solution as the dissolution medium, with a rotation speed of 75 revolutions per minute, and proceed as specified. Samples are taken at 0.5, 1, 1.5, 2, 2.5, 3, 4, 5, 6, and 8 hours.

The active pharmaceutical ingredient levodopa in this osmotic pump tablet is released for about 6 hours (as shown in FIG. 42). The active pharmaceutical ingredient carbidopa in the drug-containing immediate release overcoat is released at 96% at 0.5 hours, with a standard deviation (SD) value of 12%.

### Embodiment 61 Capsule-type tablet with dose strength 25 mg/250 mg of Carbidopa/Levodopa

The prescription is shown in Table 9:

**Table 9**

| (1) | Drug-containing layer | mg/tablet | wt% |
|---|---|---|---|
| Drug-containing layer | Levodopa | 187.5 | 45.0 |
| (Total weight 416.66 mg) | Hydroxypropyl cellulose (EXF) | 129.2 | 31.0 |
| | Povidone (K29/32) | 20.8 | 5.00 |
| | Mannitol | 75.0 | 18.0 |
| | Magnesium stearate | 4.16 | 1.00 |

| (2) | Push layer | mg/tablet | wt% |
|---|---|---|---|
| Push layer (Total weight 208.28 mg) | Sodium carboxymethyl cellulose | 114.6 | 55.0 |
| | Sorbitol | 70.8 | 34.0 |
| | Hydroxypropyl cellulose (EXF) | 20.8 | 10.0 |
| | Iron oxide red | 1.04 | 0.50 |
| | Magnesium stearate | 1.04 | 0.50 |

| (3) | Coating membrane (solid content is 4.0%, solvent is acetone: dehydrated ethanol = 90%:10%, V:V) | mg/tablet | wt% |
|---|---|---|---|
| Coating membrane (Total weight 40.6 mg) | Cellulose acetate | 26.4 | 65.0 |
| | Copovidone VA64 | 14.2 | 35.0 |

| (4) | Drug-containing immediate release overcoat (solid content is 10.0%, solvent is dehydrated ethanol) | mg/tablet | wt% |
|---|---|---|---|
| Drug-containing immediate release overcoat (Total weight 99.44 mg) | Carbidopa monohydrate | 27.0 | 27.2 |
| | Levodopa | 62.5 | 62.9 |
| | Hydroxypropyl cellulose (EXF) | 9.94 | 10.0 |

### Methods of preparation:

### (1) Preparation of a drug-containing layer

Based on the total weight of the drug-containing layer, take 45.0 wt% of levodopa, 31.0 wt% of hydroxypropyl cellulose (EXF), 5.0 wt% of povidone (K29/32) and 18.0 wt% of mannitol. The above ingredients are dry granulated; passed through a 16-mesh stainless steel sieve to further granulate, and then mixed with 1.0 wt% of magnesium stearate to obtain drug-containing layer granules.

### (2) Preparation of a push layer

Based on the total weight of the push layer, take 55.0 wt% of sodium carboxymethyl cellulose, 34.0 wt% of sorbitol, 10.0 wt% of hydroxypropyl cellulose (EXF) and 0.5 wt% of iron oxide red; the above ingredients are dry granulated; passed through a 16-mesh stainless steel sieve to further granulate, and then mixed with 0.5 wt% of magnesium stearate to obtain push layer granules.

### (3) Preparation of a bi-layer tablet core

The drug-containing layer granules and the push layer granules are pressed into a bi-layer tablet core using a punch size of φ8 mm (shallow concave). Approximately 416.66 mg of the drug-containing layer granules obtained from step (1) are added to the punch of the tablet press and compacted, then approximately 208.28 mg of the push layer granules obtained from step (2) are added to the punch, and the two layers of granules are pressed into a contacted bi-layer tablet core (approximately 624.94 mg) by the tablet press.

### (4) Preparation of an osmotic pump tablet

The bi-layer tablet core is coated with a coating membrane. The raw material composition of the coating membrane includes 65.0 wt% of cellulose acetate and 35.0 wt% of copovidone VA 64 by weight percentage, wherein the cellulose acetate is cellulose acetate with 39.8 wt% of acetyl. The composition is dissolved in acetone/dehydrated ethanol (V:V=9:1), the solid content is 4.0%. Using a pot coater to spray the solution onto the bi-layer tablet core to form a coating membrane, which constitutes approximately 6.5% of the weight of the bi-layer tablet core.

Subsequently, the drug-containing immediate release overcoat is applied to further cover the coating membrane. The raw material composition of the drug-containing immediate release overcoat, in terms of weight percentage, comprises 27.2 wt% of carbidopa monohydrate, 62.9 wt% of levodopa, and 10.0 wt% of hydroxypropyl cellulose (EXF). This composition is dissolved in dehydrated ethanol to obtain the solution with a solid content of 10.0%. Using a pot coater to spray the solution onto the coating membrane to form a drug-containing immediate release overcoat to obtain an osmotic pump tablet. Using a laser or machine to drill a 1.0 mm drug-release orifice in the coating membrane. In this embodiment, the dose strength of the osmotic pump tablet is 25 mg/250 mg (carbidopa/levodopa) and it is a round, biconvex tablet.

Take the sample and perform the dissolution and release test according to the methods specified in General Rule of Chinese Pharmacopoeia <0931> (first method) and USP <711> (first method). Use 900 mL of 0.1 mol/L hydrochloric acid solution as the dissolution medium, with a rotation speed of 75 revolutions per minute, and proceed as specified. Samples are taken at 0.5, 1, 1.5, 2, 2.5, 3, 4, 5, 6, and 8 hours.

The active pharmaceutical ingredient levodopa in this osmotic pump tablet is released for about 6 hours (as shown in FIG. 43). The active pharmaceutical ingredient carbidopa in the drug-containing immediate release overcoat is released at 93% at 0.5 hours, with a standard deviation (SD) value of 4.6%.

### Embodiment 62 Capsule-type tablet with dose strength 25 mg/250 mg of Carbidopa/Levodopa

The prescription is shown in Table10:

**Table 10**

| (1) | Drug-containing layer | mg/tablet | wt% |
|---|---|---|---|
| Drug-containing layer (Total weight 416.71 mg) | Levodopa | 187.5 | 45.0 |
| | Hydroxypropyl cellulose (EXF) | 129.2 | 31.0 |
| | Povidone (K29/32) | 20.8 | 5.00 |
| | Mannitol | 68.8 | 16.5 |
| | Magnesium stearate | 8.33 | 2.00 |
| | Colloidal silica | 2.08 | 0.50 |

| (2) | Push layer | mg/tablet | wt% |
|---|---|---|---|
| Push layer (Total weight 208.28 mg) | Sodium carboxymethyl cellulose | 102.1 | 49.0 |
| | Sorbitol | 62.5 | 30.0 |
| | Hydroxypropyl cellulose (EXF) | 41.6 | 20.0 |
| | Iron oxide red | 1.04 | 0.50 |
| | Magnesium stearate | 1.04 | 0.50 |

| (3) | Coating membrane (solid content is 4.0%, solvent is acetone: dehydrated ethanol = 90%:10%, V:V) | mg/tablet | wt% |
|---|---|---|---|
| Coating membrane (Total weight 40.6 mg) | Cellulose acetate | 26.4 | 65.0 |
| | Copovidone VA64 | 14.2 | 35.0 |

| (4) | Drug-containing immediate release overcoat (solid content is 10.0%, solvent is dehydrated ethanol) | mg/tablet | wt% |
|---|---|---|---|
| Drug-containing immediate release overcoat (Total weight 99.44 mg) | Carbidopa monohydrate | 27.0 | 37.7 |
| | Levodopa | 62.5 | 52.3 |
| | Hydroxypropyl cellulose (EXF) | 9.94 | 10.0 |

### Methods of preparation:

### (1) Preparation of a drug-containing layer

Based on the total weight of the drug-containing layer, take 45.0 wt% of levodopa, 31.0 wt% of hydroxypropyl cellulose (EXF), 5.0 wt% of povidone (K29/32), 16.5 wt% of mannitol and 2.0 wt% of colloidal silica. The above ingredients are dry granulated; passed through a 16-mesh stainless steel sieve to further granulate, and then mixed with 2.0 wt% of magnesium stearate to obtain drug-containing layer granules.

### (2) Preparation of a push layer

Based on the total weight of the push layer, take 49.0 wt% of sodium carboxymethyl cellulose, 30.0 wt% of sorbitol, 20.0 wt% of hydroxypropyl cellulose (EXF) and 0.5 wt% of iron oxide red; the above ingredients are dry granulated; passed through a 16-mesh stainless steel sieve to further granulate, and then mixed with 0.5 wt% of magnesium stearate to obtain push layer granules.

### (3) Preparation of a bi-layer tablet core

The drug-containing layer granules and the push layer granules are pressed into a bi-layer tablet core using a punch size of φ8 mm (shallow concave). Approximately 416.71 mg of the drug-containing layer granules obtained from step (1) are added to the punch of the tablet press and compacted, then approximately 208.28 mg of the push layer granules obtained from step (2) are added to the punch, and the two layers of granules are pressed into a contacted bi-layer tablet core (approximately 624.99 mg) by the tablet press.

### (4) Preparation of an osmotic pump tablet

The bi-layer tablet core is coated with a coating membrane. The raw material composition of the coating membrane includes 65.0 wt% of cellulose acetate and 35.0 wt% of copovidone VA 64 by weight percentage, wherein the cellulose acetate is cellulose acetate with 39.8 wt% of acetyl. The composition is dissolved in acetone/dehydrated ethanol (V:V=9:1), the solid content is 4.0%. Using a pot coater to spray the solution onto the bi-layer tablet core to form a coating membrane, which constitutes approximately 6.5% of the weight of the bi-layer tablet core.

Subsequently, the drug-containing immediate release overcoat is applied to further cover the coating membrane. The raw material composition of the drug-containing immediate release overcoat, in terms of weight percentage, comprises 37.7 wt% of carbidopa monohydrate, 52.3 wt% of levodopa, and 10.0 wt% of hydroxypropyl cellulose (EXF). This composition is dissolved in dehydrated ethanol to obtain the solution with a solid content of 10.0%. Using a pot coater to spray the solution onto the coating membrane to form a drug-containing immediate release overcoat to obtain an osmotic pump tablet. Using a laser or machine to drill a 1.0 mm drug-release orifice in the coating membrane. In this embodiment, the dose strength of the osmotic pump tablet is 25 mg/250 mg (carbidopa/levodopa) and it is a round, biconvex tablet.

Take the sample and perform the dissolution and release test according to the methods specified in General Rule of Chinese Pharmacopoeia <0931> (first method) and USP <711> (first method). Use 900 mL of 0.1 mol/L hydrochloric acid solution as the dissolution medium, with a rotation speed of 75 revolutions per minute, and proceed as specified. Samples are taken at 0.5, 1, 1.5, 2, 2.5, 3, 4, 5, 6, and 8 hours.

The active pharmaceutical ingredient levodopa in this osmotic pump tablet is released for about 6 hours (as shown in FIG. 44). The active pharmaceutical ingredient carbidopa in the drug-containing immediate release overcoat is released at 93% at 0.5 hours, with a standard deviation (SD) value of 4.6%.

### Embodiment 63 Capsule-type tablet with dose strength 62.5 mg/250 mg of Carbidopa/Levodopa

The prescription is shown in Table 11:

**Table 11**

| (1) | Drug-containing layer | mg/tablet | wt% |
|---|---|---|---|
| Drug-containing layer (Total weight 396.76 mg) | Levodopa | 250.0 | 63.0 |
| | Hydroxypropyl cellulose (EXF) | 43.6 | 11.0 |
| | Povidone (K29/32) | 19.8 | 5.00 |
| | Sorbitol | 39.7 | 10.0 |
| | Poloxamer (P407) | 39.7 | 10.0 |
| | Magnesium stearate | 3.96 | 1.00 |

| (2) | Push layer | mg/tablet | wt% |
|---|---|---|---|
| Push layer (Total weight 198.38 mg) | Sodium carboxymethyl cellulose | 97.2 | 49.0 |
| | Sorbitol | 59.5 | 30.0 |
| | Hydroxypropyl cellulose (EXF) | 39.7 | 20.0 |
| | Iron oxide red | 0.99 | 0.50 |
| | Magnesium stearate | 0.99 | 0.50 |

| (3) | Coating membrane (solid content is 4.0%, solvent is acetone: dehydrated ethanol = 90%:10%, V:V) | mg/tablet | wt% |
|---|---|---|---|
| Coating membrane (Total weight 56.5 mg) | Cellulose acetate | 36.7 | 65.0 |
| | Copovidone VA64 | 19.8 | 35.0 |

| (4) | Drug-containing immediate release overcoat (solid content is 10.0%, solvent is dehydrated ethanol) | mg/tablet | wt% |
|---|---|---|---|
| Drug-containing | Carbidopa monohydrate | 67.5 | 90.0 |
| immediate release overcoat (Total weight 75 mg) | Hydroxypropyl cellulose (EXF) | 7.50 | 10.0 |

### Methods of preparation:

### (1) Preparation of a drug-containing layer

Based on the total weight of the drug-containing layer, take 63.0 wt% of levodopa, 11.0 wt% of hydroxypropyl cellulose (EXF), 5.0 wt% of povidone (K29/32), 10.0 wt% of sorbitol and 10.0 wt% of poloxamer (P407). The above ingredients are dry granulated; passed through a 16-mesh stainless steel sieve to further granulate, and then mixed with 1.0 wt% of magnesium stearate to obtain drug-containing layer granules.

### (2) Preparation of a push layer

Based on the total weight of the push layer, take 49.0 wt% of sodium carboxymethyl cellulose, 30.0 wt% of sorbitol, 20.0 wt% of hydroxypropyl cellulose (EXF) and 0.5 wt% of iron oxide red; the above ingredients are dry granulated; passed through a 16-mesh stainless steel sieve to further granulate, and then mixed with 0.5 wt% of magnesium stearate to obtain push layer granules.

### (3) Preparation of a bi-layer tablet core

The drug-containing layer granules and the push layer granules are pressed into a bi-layer tablet core using a punch size of φ8 mm (shallow concave). Approximately 396.76 mg of the drug-containing layer granules obtained from step (1) are added to the punch of the tablet press and compacted, then approximately 198.38 mg of the push layer granules obtained from step (2) are added to the punch, and the two layers of granules are pressed into a contacted bi-layer tablet core (approximately 595.14 mg) by the tablet press.

### (4) Preparation of an osmotic pump tablet

The bi-layer tablet core is coated with a coating membrane. The raw material composition of the coating membrane includes 65.0 wt% of cellulose acetate and 35.0 wt% of copovidone VA 64 by weight percentage, wherein the cellulose acetate is cellulose acetate with 39.8 wt% of acetyl. The composition is dissolved in acetone/dehydrated ethanol (V:V=9:1), the solid content is 4.0%. Using a pot coater to spray the solution onto the bi-layer tablet core to form a coating membrane, which constitutes approximately 9.5% of the weight of the bi-layer tablet core.

Subsequently, the drug-containing immediate release overcoat is applied to further cover the coating membrane. The raw material composition of the drug-containing immediate release overcoat, in terms of weight percentage, comprises 90.0 wt% of carbidopa monohydrate and 10.0wt% of hydroxypropyl cellulose (EXF). This composition is dissolved in dehydrated ethanol to obtain the solution with a solid content of 10.0%. Using a pot coater to spray the solution onto the coating membrane to form a drug-containing immediate release overcoat to obtain an osmotic pump tablet. Using a laser or machine to drill a 1.0 mm drug-release orifice in the coating membrane. In this embodiment, the dose strength of the osmotic pump tablet is 62.5 mg/250 mg (carbidopa/levodopa) and it is a round, biconvex tablet.

Take the sample and perform the dissolution and release test according to the methods specified in General Rule of Chinese Pharmacopoeia <0931> (first method) and USP <711> (first method). Use 900 mL of 0.1 mol/L hydrochloric acid solution as the dissolution medium, with a rotation speed of 75 revolutions per minute, and proceed as specified. Samples are taken at 1, 2, 3, 4, 6, 8, and 10 hours.

The active pharmaceutical ingredient levodopa in this osmotic pump tablet is released for about 10 hours (as shown in FIG. 45). The active pharmaceutical ingredient carbidopa in the drug-containing immediate release overcoat is released at 98% at 0.5 hours, with a standard deviation (SD) value of 6.4%.

### Embodiment 64 Capsule-type tablet with dose strength 62.5 mg/250 mg of Carbidopa/Levodopa

The prescription is shown in Table12:

**Table 12**

| (1) | Drug-containing layer | mg/tablet | wt% |
|---|---|---|---|
| Drug-containing layer (Total weight 396.72 mg) | Levodopa | 250.0 | 63.0 |
| | Hydroxypropyl cellulose (EXF) | 43.6 | 11.0 |
| | Povidone (K29/32) | 19.8 | 5.00 |
| | Sorbitol | 33.7 | 8.50 |
| | Poloxamer (P407) | 39.7 | 10.0 |
| | Magnesium stearate | 7.94 | 2.00 |
| | Colloidal silica | 1.98 | 0.50 |

| (2) | Push layer | mg/tablet | wt% |
|---|---|---|---|
| Push layer (Total weight 198.38 mg) | Sodium carboxymethyl cellulose | 97.2 | 49.0 |
| | Sorbitol | 59.5 | 30.0 |
| | Hydroxypropyl cellulose (EXF) | 39.7 | 20.0 |
| | Iron oxide red | 0.99 | 0.50 |
| | Magnesium stearate | 0.99 | 0.50 |

| (3) | Coating membrane (solid content is 4.0%, solvent is acetone: dehydrated ethanol = 90%:10%, V:V) | mg/tablet | wt% |
|---|---|---|---|
| Coating membrane (Total weight 56.5 mg) | Cellulose acetate | 36.7 | 65.0 |
| | Copovidone VA64 | 19.8 | 35.0 |

| (4) | Drug-containing immediate release overcoat (solid content is 10.0%, solvent is dehydrated ethanol) | mg/tablet | wt% |
|---|---|---|---|
| Drug-containing immediate release overcoat (Total weight 75 mg) | Carbidopa monohydrate | 67.5 | 90.0 |
| | Hydroxypropyl cellulose (EXF) | 7.50 | 10.0 |

### Methods of preparation:

### (1) Preparation of a drug-containing layer

Based on the total weight of the drug-containing layer, take 63.0 wt% of levodopa, 11.0 wt% of hydroxypropyl cellulose (EXF), 5.0 wt% of povidone (K29/32), 8.5 wt% of sorbitol, 10.0 wt% of poloxamer (P407) and 0.5 wt% of colloidal silica. The above ingredients are dry granulated; passed through a 16-mesh stainless steel sieve to further granulate, and then mixed with 2.0 wt% of magnesium stearate to obtain drug-containing layer granules.

### (2) Preparation of a push layer

Based on the total weight of the push layer, take 49.0 wt% of sodium carboxymethyl cellulose, 30.0 wt% of sorbitol, 20.0 wt% of hydroxypropyl cellulose (EXF) and 0.5 wt% of iron oxide red; the above ingredients are dry granulated; passed through a 16-mesh stainless steel sieve to further granulate, and then mixed with 0.5 wt% of magnesium stearate to obtain push layer granules.

### (3) Preparation of a bi-layer tablet core

The drug-containing layer granules and the push layer granules are pressed into a bi-layer tablet core using a punch size of φ8 mm (shallow concave). Approximately 396.72 mg of the drug-containing layer granules obtained from step (1) are added to the punch of the tablet press and compacted, then approximately 198.38 mg of the push layer granules obtained from step (2) are added to the punch, and the two layers of granules are pressed into a contacted bi-layer tablet core (approximately 595.1 mg) by the tablet press.

### (4) Preparation of an osmotic pump tablet

The bi-layer tablet core is coated with a coating membrane. The raw material composition of the coating membrane includes 65.0 wt% of cellulose acetate and 35.0 wt% of copovidone VA 64 by weight percentage, wherein the cellulose acetate is cellulose acetate with 39.8 wt% of acetyl. The composition is dissolved in acetone/dehydrated ethanol (V:V=9:1), the solid content is 4.0%. Using a pot coater to spray the solution onto the bi-layer tablet core to form a coating membrane, which constitutes approximately 9.5% of the weight of the bi-layer tablet core.

Subsequently, the drug-containing immediate release overcoat is applied to further cover the coating membrane. The raw material composition of the drug-containing immediate release overcoat, in terms of weight percentage, comprises 90.0 wt% of carbidopa monohydrate and 10.0 wt% of hydroxypropyl cellulose (EXF). This composition is dissolved in dehydrated ethanol to obtain the solution with a solid content of 10.0%. Using a pot coater to spray the solution onto the coating membrane to form a drug-containing immediate release overcoat to obtain an osmotic pump tablet. Using a laser or machine to drill a 1.0 mm drug-release orifice in the coating membrane. In this embodiment, the dose strength of the osmotic pump tablet is 62.5mg/250 mg (carbidopa/levodopa) and it is a round, biconvex tablet.

Take the sample and perform the dissolution and release test according to the methods specified in General Rule of Chinese Pharmacopoeia <0931> (first method) and USP <711> (first method). Use 900 mL of 0.1 mol/L hydrochloric acid solution as the dissolution medium, with a rotation speed of 75 revolutions per minute, and proceed as specified. Samples are taken at 1, 2, 3, 4, 5, 6, 8, and 10 hours.

The active pharmaceutical ingredient levodopa in this osmotic pump tablet is released for about 10 hours (as shown in FIG. 46). The active pharmaceutical ingredient carbidopa in the drug-containing immediate release overcoat is released at 98% at 0.5 hours, with a standard deviation (SD) value of 6.4%.

### Comparative Embodiment A

### Preparation of the osmotic pump tablet (Immersion Experiment)

The steps of Embodiment 47 are repeated in this comparative embodiment, except that: (1) The drug-containing layer comprises 63.0 wt% of levodopa, 5.0 wt% of hydroxypropyl cellulose with a weight average molecular weight of 80,000, 10.0 wt% of povidone, 15.0 wt% of mannitol, 0.9 wt% of aspartame, 0.1 wt% of mint flavor and 1.0 wt% of magnesium stearate.

The release profile of the final dosage form was measured using a USP I paddle method in simulated saliva. FIG. 48 shows the release profiles of LD, where the release amount of active pharmaceutical ingredient(s) is unable to reach 85 %. By observing, it is found that the controlled-release membrane ruptures during the dissolution process, as shown in FIG. 47.

### Comparative Embodiment B

### Preparation of the osmotic pump tablet (with many dissolution residues)

The steps of Embodiment 47 are repeated in this embodiment, except that: the drug-containing layer comprises 63.0 wt% of levodopa, 5.0 wt% of hydroxypropyl cellulose with a weight average molecular weight of 80,000, 10.0 wt% of povidone, 15.0 wt% of mannitol, 0.9 wt% of aspartame, 0.1 wt% of mint flavor and 1.0 wt% of magnesium stearate.

The release profile of the final dosage form is measured using a USP I paddle method in simulated saliva. FIG. 49 shows the release profile of LD. The release amount of active pharmaceutical ingredient(s) cannot reach 85%, the residual amount of active pharmaceutical ingredient(s) is large, and only 67.5% is released into the platform after 4 hours of release.

## Claims

1. An osmotic pump tablet, wherein the osmotic pump tablet is any one of the following schemes:
Scheme (1): the osmotic pump tablet comprises a tablet core and a coating membrane that coats the tablet core; the coating membrane has a drug-release orifice; the tablet core comprises a drug-containing layer, and the drug-containing layer comprises an active pharmaceutical ingredient, a hydrophilic polymer and a surfactant; the hydrophilic polymer comprises hydroxypropyl cellulose, and the surfactant comprises poloxamer;
Scheme (2): the osmotic pump tablet comprises a tablet core, a coating membrane that coats the tablet core, and a drug-containing immediate release overcoat; the coating membrane has a drug-release orifice; the tablet core comprises a drug-containing layer, and the drug-containing layer comprises an active pharmaceutical ingredient and a hydrophilic polymer, but does not comprise a surfactant; the hydrophilic polymer comprises povidone K29/32;
Scheme (3): the osmotic pump tablet comprises a tablet core, a coating membrane that coats the tablet core, and a drug-containing immediate release overcoat; the coating membrane has a drug-release orifice; the tablet core comprises a drug-containing layer, and the drug-containing layer comprises an active pharmaceutical ingredient and an excipient; the drug-containing immediate release overcoat comprises an active pharmaceutical ingredient and a pharmaceutical excipient, and the pharmaceutical excipient is a binding agent.

2. The osmotic pump tablet according to claim 1, wherein
Scheme (1) satisfies one or more of the following conditions (a) to (k):
(a) based on the total weight of the drug-containing layer, the content of the hydrophilic polymer is 5-25 wt%, such as 10-20 wt%, and such as 10-15 wt%;
(b) the hydroxypropyl cellulose is hydroxypropyl cellulose EXF;
(c) the hydrophilic polymer is a mixture of hydroxypropyl cellulose and one or more selected from hydroxypropyl methyl cellulose, carboxymethyl cellulose, polyvinylpyrrolidone, and hydroxyethyl cellulose;
(d) based on the total weight of the drug-containing layer, the content of the surfactant is 1-15 wt%, such as 2-10 wt%, and such as 5-10 wt%;
(e) the poloxamer is poloxamer 407;
(f) the surfactant is a mixture of poloxamer and one or more selected from polysorbates, fatty acid glycerides, sodium dodecyl benzene sulfonate, and sodium dodecyl sulfate;
(g) based on the total weight of the drug-containing layer, the content of the active pharmaceutical ingredient(s) is 50-75 wt%, such as 55-65 wt%, and such as 58-63 wt%;
(h) the active pharmaceutical ingredient(s) is active pharmaceutical ingredient(s) that are released in the oral cavity or drugs with an oral topical treatment or an absorption site in the upper gastrointestinal tract;
(i) the active pharmaceutical ingredient(s) is one or more selected from levodopa or its ester or its salt, carbidopa, baclofen, acyclovir, valacyclovir, ganciclovir, metformin, and gabapentin; preferably one or two selected from levodopa or its ester, and carbidopa;
(j) the active pharmaceutical ingredient(s) is selected from antifungal drugs and antitumor drugs; wherein the antifungal drug is, for example, one or more selected from nystatin, fluconazole, posaconazole, isavuconazole, voriconazole, anidulafungin, caspofungin, and micafungin;
(k) the drug-containing layer further comprises other excipients, and other excipients are one or more selected from an osmotic agent, a pharmaceutical carrier, a binding agent, a lubricant, an antioxidant, and a flavoring agent;
preferably, the osmotic agent is one or more selected from magnesium sulfate, magnesium chloride, sodium chloride, lithium chloride, potassium sulfate, sodium sulfate, mannitol, urea, sorbitol, inositol, sucrose, glucose, lactose, starch, pregelatinized starch, dextrin, and microcrystalline cellulose, such as sorbitol or mannitol; preferably, based on the total weight of the drug-containing layer, the content of the osmotic agent is 0-50 wt% but not 0 wt%, such as 5-20 wt%;
preferably, the pharmaceutical carrier is one or more selected from povidone (such as povidone K30 or povidone K29/32), copovidone, carbomer, hydroxypropyl methyl cellulose, hydroxyethyl cellulose, polyoxyethylene, and sodium alginate; preferably, based on the total weight of the drug-containing layer, the content of the pharmaceutical carrier is 0-50 wt% but not 0 wt%, such as 0.5-50 wt%, 5-50 wt%, and 5-10 wt%;
preferably, the binding agent is one or more selected from methyl cellulose, hydroxypropyl methyl cellulose, hydroxyethyl cellulose, hydroxypropyl cellulose, sodium carboxymethyl cellulose, povidone, copovidone, and gelatin; preferably, based on the total weight of the drug-containing layer, the content of the binding agent is 5-50 wt%, such as 5 wt%;
preferably, the lubricant is one or more selected from stearic acid, magnesium stearate, magnesium fumarate stearate, calcium stearate, sodium stearate fumarate, polyethylene glycol, talc, and colloidal silica, such as one or two selected from magnesium stearate and colloidal silica; preferably, based on the total weight of the drug-containing layer, the content of the lubricant is 0-3 wt% but not 0 wt%, such as 0.5-2.5 wt%, and such as 1-2 wt%;
preferably, the antioxidant is one or more selected from dibutylhydroxytoluene, butylhydroxyanisole, *tert*-butylhydroquinone, propyl gallate, vitamin C, and vitamin E, such as dibutylhydroxytoluene; preferably, based on the total weight of the drug-containing layer, the content of the antioxidant is 0-1 wt% but not 0 wt%, such as 0.01-0.1 wt%;
preferably, the flavoring agent is one or more selected from aspartame, apple flavor, orange flavor, banana flavor, mint flavor, saccharin sodium, and stevioside, such as one or two selected from aspartame and mint flavor; preferably, based on the total weight of the drug-containing layer, the content of the flavoring agent is 0-10 wt%, such as 0-2 wt% but not 0 wt%, for example, 1.0 wt%; when the flavoring agent is a combination of aspartame and mint flavor, the content of aspartame is 0.9 wt% and the content of mint flavor is 0.1 wt%;
Scheme (2) satisfies one or more of the following conditions (i) to (vii):
(i) based on the total weight of the drug-containing layer, the content of the active pharmaceutical ingredient(s) is 30-75 wt%, such as 38-65 wt%;
(ii) (h) in Scheme (1);
(iii) (i) in Scheme (1);
(iv) (j) in Scheme (1);
(v) based on the total weight of the drug-containing layer, the content of the hydrophilic polymer povidone K29/32 is 0.5-50 wt%, such as 0.5-20 wt%, and such as 5-10 wt%;
(vi) the hydrophilic polymer is a mixture of povidone K29/32 and one or more selected from hydroxypropyl cellulose, hydroxypropyl methyl cellulose, carboxymethyl cellulose, and hydroxyethyl cellulose;
(vii) the excipient is one or more selected from a filler, an osmotic agent, an acidifying agent, a lubricant, and a flavoring agent;
preferably, when the excipient comprises a filler, the filler is one or more selected from microcrystalline cellulose, hydroxypropyl cellulose, and mannitol; based on the total weight of the drug-containing layer, the content of the filler is 0-50 wt% but not 0 wt%, preferably 20-50 wt%; for example, 31 wt%;
preferably, when the excipient comprises an osmotic agent, the osmotic agent is one or more selected from magnesium sulfate, magnesium chloride, sodium chloride, lithium chloride, potassium sulfate, sodium sulfate, mannitol, urea, sorbitol, inositol, sucrose, and glucose; based on the total weight of the drug-containing layer, the content of the osmotic agent is 0-50 wt% but not 0 wt%; such as 4-50 wt%;
preferably, when the excipient comprises an acidifying agent, the acidifying agent is one or more selected from citric acid, sodium citrate, potassium citrate, malic acid, fumaric acid, lactic acid, phosphoric acid, and tartaric acid; based on the total weight of the drug-containing layer, the content of the acidifying agent is 0-10 wt% but not 0 wt%; such as 5-10 wt%;
preferably, when the excipient comprises a lubricant, the lubricant is one or more selected from magnesium stearate, magnesium stearate fumarate, talc, and colloidal silica, such as one or two selected from magnesium stearate and colloidal silica; based on the total weight of the drug-containing layer, the content of the lubricant is 0.5-3 wt%; such as 0.5-2.5 wt%, and such as 1-2 wt%;
preferably, when the excipient comprises a flavoring agent, the flavoring agent is one or more selected from aspartame, apple flavor, orange flavor, banana flavor, mint flavor, saccharin sodium, and stevioside, for example, one or two selected from aspartame and mint flavor (when both are selected, they may be present in any proportion); preferably, based on the total weight of the drug-containing layer, the content of the flavoring agent is 0-10 wt% but not 0 wt%, such as 1-6 wt%, and such as 2-5 wt%;
Scheme (3) satisfies one or more of the following conditions 1) to 10):
1) in the drug-containing immediate release overcoat, the pharmaceutical excipient is hydroxypropyl cellulose (EXF);
2) in the drug-containing immediate release overcoat, the content of the active pharmaceutical ingredient is 80-90 wt%; the content of the binding agent is 10-20.0 wt%;
3) in the drug-containing immediate release overcoat, the active pharmaceutical ingredient(s) comprise levodopa and/or carbidopa, and when the active pharmaceutical ingredient(s) comprise levodopa and carbidopa, preferably, the mass ratio of levodopa to carbidopa is 1:1-4:1;
4) in the drug-containing layer, based on the total weight of the drug-containing layer, the content of the active pharmaceutical ingredient(s) is 30-75 wt%, such as 38-65 wt%;
5) in the drug-containing layer, the active pharmaceutical ingredient is (h) in Scheme (1);
6) in the drug-containing layer, the active pharmaceutical ingredient is (i) in Scheme (1);
7) in the drug-containing layer, the active pharmaceutical ingredient is (j) in Scheme (1);
8) in the drug-containing layer, the excipient is one or more selected from a filler, an osmotic agent, an acidifying agent, a lubricant, a flavoring agent, an antioxidant, a hydrophilic polymer, a pharmaceutical carrier, a binding agent, and a surfactant;
preferably, when the excipient comprises a filler, the filler is one or more selected from microcrystalline cellulose, hydroxypropyl cellulose, and mannitol; based on the total weight of the drug-containing layer, the content of the filler is 0-50 wt% but not 0 wt%, preferably 20-50 wt%; for example, 31 wt%;
preferably, when the excipient comprises an osmotic agent, the osmotic agent is one or more selected from magnesium sulfate, magnesium chloride, sodium chloride, lithium chloride, potassium sulfate, sodium sulfate, mannitol, urea, sorbitol, inositol, sucrose, and glucose; based on the total weight of the drug-containing layer, the content of the osmotic agent is 0-50 wt% but not 0 wt%; such as 4-50 wt%;
preferably, when the excipient comprises an acidifying agent, the acidifying agent is one or more selected from citric acid, sodium citrate, potassium citrate, malic acid, fumaric acid, lactic acid, phosphoric acid, and tartaric acid; based on the total weight of the drug-containing layer, the content of the acidifying agent is 0-10 wt% but not 0 wt%; such as 5-10 wt%;
preferably, when the excipient comprises a lubricant, the lubricant is one or more selected from magnesium stearate, magnesium stearate fumarate, talc, and colloidal silica, such as one or two selected from magnesium stearate and colloidal silica; based on the total weight of the drug-containing layer, the content of the lubricant is 0.5-3 wt%; such as 0.5-2.5 wt%, and such as 1-2 wt%;
preferably, when the excipient comprises a flavoring agent, the flavoring agent is one or more selected from aspartame, apple flavor, orange flavor, banana flavor, mint flavor, saccharin sodium, and stevioside, for example, one or two selected from aspartame and mint flavor (when both are selected, they may be present in any proportion); preferably, based on the total weight of the drug-containing layer, the content of the flavoring agent is 0-10 wt% but not 0 wt%, such as 1-6 wt%, and such as 2-5 wt%;
preferably, when the excipient comprises a hydrophilic polymer, the hydrophilic polymer is one or more selected from hydroxypropyl cellulose, hydroxypropyl methyl cellulose, carboxymethyl cellulose, polyvinylpyrrolidone, and hydroxyethyl cellulose; preferably, based on the total weight of the drug-containing layer, the content of the hydrophilic polymer is 5-25 wt%, such as 10-20 wt%, and such as 10-15 wt%;
preferably, when the excipient comprises a pharmaceutical carrier, the pharmaceutical carrier is one or more selected from povidone (such as povidone K30 or povidone K29/32), copovidone, carbomer, hydroxypropyl methyl cellulose, hydroxyethyl cellulose, polyoxyethylene, and sodium alginate; preferably, based on the total weight of the drug-containing layer, the content of the pharmaceutical carrier is 0-50 wt% but not 0 wt%, such as 0.5-50 wt%, 5-50 wt%, and 5-10 wt%;
preferably, when the excipient comprises a binding agent, the binding agent is one or more selected from methyl cellulose, hydroxypropyl methyl cellulose, hydroxyethyl cellulose, hydroxypropyl cellulose, sodium carboxymethyl cellulose, povidone (such as povidone K30 or povidone K29/32), copovidone, and gelatin; preferably, based on the total weight of the drug-containing layer, the content of the binding agent is 0-50 wt% but not 0 wt%, such as 5-50 wt%, and such as 5-10 wt%;
preferably, when the excipient comprises a surfactant, the surfactant is one or more selected from poloxamer, polysorbates, fatty acid glycerides, sodium dodecyl benzene sulfonate, and sodium dodecyl sulfate; based on the total weight of the drug-containing layer, the content of the surfactant is 1-15 wt%, such as 2-10 wt%, and such as 5-10 wt%.

3. The osmotic pump tablet according to claim 2, wherein the drug-containing layer is any one of the following combinations:
(a) an active pharmaceutical ingredient, a hydrophilic polymer, a surfactant, a binding agent, an osmotic agent, a lubricant, and a flavoring agent;
(b) an active pharmaceutical ingredient, a hydrophilic polymer, a surfactant, a binding agent, an osmotic agent, a lubricant, an antioxidant, and a flavoring agent;
(c) an active pharmaceutical ingredient, a hydrophilic polymer, a surfactant, an osmotic agent, a lubricant, an antioxidant, and a flavoring agent;
(d) an active pharmaceutical ingredient, a hydrophilic polymer, a surfactant, a binding agent, an osmotic agent, and a lubricant;
(i) an active pharmaceutical ingredient, a filler, an osmotic agent, a hydrophilic polymer, an acidifying agent, and a lubricant;
(ii) an active pharmaceutical ingredient, a filler, a hydrophilic polymer, and a lubricant;
(iii) a filler, an osmotic agent, a hydrophilic polymer, a lubricant, and a flavoring agent;
(iv) an osmotic agent, a hydrophilic polymer, and a lubricant;
(v) a filler, a hydrophilic polymer, an osmotic agent, and a lubricant;
(vi) a filler, an osmotic agent, an acidifying agent, and a lubricant;
(vii) a filler, an osmotic agent, a flavoring agent, and a lubricant; or
(viii) a filler, an acidifying agent, and a lubricant;
in Scheme (1), the drug-containing layer is any combination of (a) to (d);
in Scheme (2), the drug-containing layer is any combination of (i) to (v);
in Scheme (3), the drug-containing layer is any combination of (a) to (d) and (i) to (viii).

4. The osmotic pump tablet according to claim 3, wherein the drug-containing layer is any one of the following combinations:
(a) 63.0 wt% of levodopa, 10.0 wt% of hydroxypropyl cellulose, 10.0 wt% of poloxamer, 5.0 wt% of povidone, 10.0 wt% of sorbitol, 0.9 wt% of aspartame, 0.1 wt% of mint flavor, and 1.0 wt% of magnesium stearate;
(b) 58.0 wt% of levodopa, 15.0 wt% of hydroxypropyl cellulose, 5.0 wt% of poloxamer, 5.0 wt% of povidone, 15.0 wt% of sorbitol, 0.9 wt% of aspartame, 0.1 wt% of mint flavor, and 1.0 wt% of magnesium stearate;
(c) 58.0 wt% of levodopa, 15.0 wt% of hydroxypropyl cellulose, 5.0 wt% of povidone, 5.0 wt% of poloxamer, 15.0 wt% of sorbitol, 0.9 wt% of aspartame, 0.1 wt% of mint flavor, and 1.0 wt% of magnesium stearate;
(d) 54.9 wt% of levodopa, 3.16 wt% of carbidopa, 15.0 wt% of hydroxypropyl cellulose, 5.0 wt% of povidone, 14.87 wt% of sorbitol, 5.0 wt% of poloxamer, 0.9 wt% of aspartame, 0.1 wt% of mint flavor, 0.1 wt% of dibutylhydroxytoluene, and 1.0 wt% of magnesium stearate;
(e) 54.9 wt% of levodopa, 3.16 wt% of carbidopa, 15.0 wt% of hydroxypropyl cellulose, 19.87 wt% of mannitol, 0.9 wt% of aspartame, 5.0 wt% of poloxamer, 0.1 wt% of mint flavor, 0.1 wt% of dibutylhydroxytoluene, and 1.0 wt% of magnesium stearate;
(f) 63.0 wt% of levodopa, 11.0 wt% of hydroxypropyl cellulose, 5.00 wt% of povidone, 10.0 wt% of sorbitol, 10.0 wt% of poloxamer, and 1.00 wt% of magnesium stearate;
(g) 63.0 wt% of levodopa, 11.0 wt% of hydroxypropyl cellulose, 5.00 wt% of povidone, 8.50 wt% of sorbitol, 10.0 wt% of poloxamer, 0.50 wt% of colloidal silica, and 2.00 wt% of magnesium stearate;
(i) 45.0 wt% of levodopa, 31.0 wt% of hydroxypropyl cellulose, 5.00 wt% of povidone K29/32, 18.0 wt% of mannitol, and 1.00 wt% of magnesium stearate;
(ii) 45.0 wt% of levodopa, 31.0 wt% of hydroxypropyl cellulose, 5.00 wt% of povidone K29/32, 16.5 wt% of mannitol, 0.50 wt% of colloidal silica, and 2.00 wt% of magnesium stearate;
1) 40.0 wt% of levodopa, 10.8 wt% of carbidopa monohydrate, 20.0 wt% of microcrystalline cellulose, 18.7 wt% of mannitol, 5.0 wt% of hydroxypropyl methyl cellulose, 5.0 wt% of citric acid, and 0.5 wt% of magnesium stearate;
2) 38.0 wt% of levodopa, 50.0 wt% of microcrystalline cellulose, 10.0 wt% of hydroxypropyl methyl cellulose, and 2.0 wt% of magnesium stearate;
3) 40.0 wt% of levodopa, 10.8 wt% of carbidopa monohydrate, 31.0 wt% of hydroxypropyl cellulose with an average molecular weight of 80,000, 12.7 wt% of mannitol, 5.0 wt% of citric acid, and 0.5 wt% of magnesium stearate;
4) 45.0 wt% of levodopa, 31.0 wt% of hydroxypropyl cellulose, 16.0 wt% of mannitol, 5.0 wt% of povidone K30, 1.0 wt% of aspartame, 1.0 wt% of mint flavor, and 1.0 wt% of magnesium stearate;
5) 45.0 wt% of levodopa, 31.0 wt% of hydroxypropyl cellulose, 17.0 wt% of mannitol, 5.0 wt% of povidone K30, 1.0 wt% of magnesium stearate, and 1.0 wt% of aspartame;
6) 45.0 wt% of levodopa, 31.0 wt% of hydroxypropyl cellulose, 17.0 wt% of mannitol, 5.0 wt% of povidone K30, 1.0 wt% of magnesium stearate, and 1.0 wt% of aspartame;
7) 70.0 wt% of levodopa, 9.0 wt% of mannitol, 20.0 wt% of povidone K30, and 1.0 wt% of magnesium stearate;
8) 20.0 wt% of levodopa, 20.0 wt% of carbidopa, 50.0 wt% of hydroxypropyl cellulose, 4.0 wt% of mannitol, 5.0 wt% of aspartame, and 1.0 wt% of magnesium stearate;
9) 45.0 wt% of levodopa, 31.0 wt% of hydroxypropyl cellulose, 22.0 wt% of mannitol, 0.9 wt% of aspartame, 0.1 wt% of mint flavor, and 1.0 wt% of magnesium stearate;
10) 19.5 wt% of levodopa, 20.0 wt% of carbidopa, 50.0 wt% of mannitol, 10.0 wt% of citric acid, and 0.5 wt% of magnesium stearate;
in Scheme (1), the drug-containing layer is any combination of (a) to (g);
in Scheme (2), the drug-containing layer is any combination of (i) to (ii);
in Scheme (3), the drug-containing layer is any combination of (a) to (f), (i) to (ii) and 1) to 10).

5. The osmotic pump tablet according to any one of claims 1-4, wherein
in Schemes (1), (2), and (3), the tablet core independently further comprises a push layer; the drug-containing layer and the push layer are sequentially laminated together to obtain a bi-layer tablet core, and the coating membrane is wrapped around the outside of the tablet core.

6. The osmotic pump tablet according to claim 5, wherein in Schemes (1), (2), and (3), the push layer independently satisfies one or more of the following conditions:
(1) the mass ratio of the drug-containing layer to the push layer is 0.5:1-4:1, such as 1.5:1-3.5:1;
(2) the push layer comprises one or more selected from a swelling agent, an osmotic agent, a binding agent, a lubricant, and a coloring agent, or a combination of a swelling agent, an osmotic agent, a binding agent, a lubricant, and a coloring agent.

7. The osmotic pump tablet according to claim 6, wherein in Schemes (1), (2), and (3), the push layer independently satisfies one or more of the following conditions:
(1) the swelling agent is one or more selected from sodium carboxymethyl starch, hydroxypropyl methyl cellulose, sodium carboxymethyl cellulose, hydroxyethyl cellulose, carbomer, sodium alginate, κ-carrageenan, sodium carboxymethyl cellulose, and polyethylene oxide; for example, sodium carboxymethyl cellulose (7H4XF); preferably, based on the total weight of the push layer, the content of the swelling agent is 30-95 wt%, such as 49.0-68.5 wt%;
(2) the osmotic agent is one or more selected from magnesium sulfate, magnesium chloride, sodium chloride, lithium chloride, potassium sulfate, sodium sulfate, mannitol, urea, sorbitol, inositol, sucrose, glucose, lactose, starch, pregelatinized starch, dextrin, and microcrystalline cellulose, such as sorbitol or mannitol; preferably, based on the total weight of the push layer, the content of the osmotic agent is 5-70 wt%, such as 10.0-30.0 wt%;
(3) the binding agent is one or more selected from methyl cellulose, hydroxypropyl methyl cellulose, hydroxyethyl cellulose, hydroxypropyl cellulose, sodium carboxymethyl cellulose, povidone, copovidone, and gelatin, such as hydroxypropyl cellulose (EXF); preferably, based on the total weight of the push layer, the content of the binding agent is 3-25 wt%, such as 20 wt%;
(4) the lubricant is one or more selected from stearic acid, magnesium stearate, magnesium fumarate stearate, calcium stearate, sodium stearyl fumarate, polyethylene glycol, talc, and colloidal silica, such as one or two selected from magnesium stearate and colloidal silica; preferably, based on the total weight of the push layer, the content of the lubricant is 0-7 wt% but not 0 wt%, such as 0-3 wt%, such as 0.5-1 wt%;
(5) the coloring agent is one or more selected from iron oxide red, iron oxide yellow, iron oxide violet, and iron oxide black, such as iron oxide red; preferably, based on the total weight of the push layer, the content of the coloring agent is 0-2 wt% but not 0 wt%, such as 0.5 wt%.

8. The osmotic pump tablet according to claim 7, wherein in Schemes (1), (2), and (3), the push layer independently satisfies any one of the following combinations:
(1) 49.0 wt% of sodium carboxymethyl cellulose, 30 wt% of sorbitol, 20 wt% of hydroxypropyl cellulose, 0.5 wt% of iron oxide red, and 0.5 wt% of magnesium stearate;
(2) 68.5 wt% of sodium carboxymethyl cellulose, 10.0 wt% of sorbitol, 20.0 wt% of hydroxypropyl cellulose, 0.5 wt% of iron oxide red, 0.5 wt% of colloidal silica, and 0.5 wt% of magnesium stearate.

9. The osmotic pump tablet according to any one of claims 1-8, wherein in Schemes (1), (2), and (3), the osmotic pump tablet independently further comprises an isolation layer; the drug-containing layer, the push layer, and the isolation layer are sequentially laminated together to obtain a three-layer tablet core; the coating membrane is wrapped around the outside of the tablet core.

10. The osmotic pump tablet according to claim 9, wherein in Schemes (1), (2), and (3), the isolation layer independently satisfies one or more of the following conditions:
(1) the isolation layer comprises one or more selected from ethyl cellulose, cellulose acetate, acrylic resin, and microcrystalline cellulose, such as ethyl cellulose;
(2) the mass ratio of the drug-containing layer to the isolation layer is (0.01-0.15):1, such as 0.05:1-0.1:1.

11. The osmotic pump tablet according to any one of claims 1-10, wherein in Schemes (1), (2), and (3), the coating membrane independently satisfies one or more of the following conditions:
(1) the coating membrane comprises a membrane-forming material and a porogen, or comprises a membrane-forming material, a porogen, and a plasticizer;
preferably, the membrane-forming material is one or more selected from cellulose acetate, ethyl cellulose, and acrylic resin, such as cellulose acetate, and such as cellulose acetate containing 39.8 wt% acetyl;
preferably, based on the total weight of the coating membrane, the content of the membrane-forming material is 50-70 wt%, such as 50wt%-55 wt%;
preferably, the porogen is copovidone, such as copovidone VA64;
preferably, based on the total weight of the coating membrane, the content of the porogen is 30-50 wt%, such as 45-50 wt%;
preferably, the plasticizer is one or more selected from polyethylene glycol, methyl phthalate, ethyl phthalate, dibutyl sebacate, triethyl citrate, tributyl citrate, tributyl acetyl citrate, glyceryl acetate, and castor oil, such as triethyl citrate or polyethylene glycol 400; preferably, based on the total weight of the coating membrane, the content of the plasticizer is 0-20 wt%, such as 0-5 wt% but not 0 wt%;
(2) the tensile strength of the coating membrane is 1-10 Mpa;
(3) the break elongation of the coating membrane is 1.1-2.0;
(4) the average thickness of the coating membrane is 100±8 µm to 200±10 µm;
(5) the pore size of the drug-release orifice is 0.3 mm-1.2 mm;
(6) based on the total weight of the osmotic pump tablet, the content of the coating membrane is 2-10 wt%.

12. The osmotic pump tablet according to any one of claims 1-11, wherein
in Schemes (1) and (2), the osmotic pump tablet further comprises a drug-containing immediate release overcoat;
preferably, the drug-containing immediate release overcoat comprises an active pharmaceutical ingredient and a pharmaceutical excipient, and the active pharmaceutical ingredient comprises one or more selected from levodopa, ester of levodopa, salt of levodopa, and carbidopa; the pharmaceutical excipient comprises one or more selected from a binding agent, an antioxidant, a plasticizer, and a flavoring agent; the content of the active pharmaceutical ingredient is preferably 70-90 wt%; the content of the binding agent is preferably 10-20 wt%; the content of the antioxidant is preferably 0.5-2.0 wt%; the content of the plasticizer is preferably 1-2 wt%;
further preferably, the active pharmaceutical ingredient is levodopa, carbidopa, or a combination thereof; the pharmaceutical excipient is a combination of a binding agent, an antioxidant and a plasticizer or only a binding agent.

13. The osmotic pump tablet according to claim 12, wherein
in the drug-containing immediate release overcoat, the active pharmaceutical ingredient comprises levodopa and/or carbidopa, wherein when the active pharmaceutical ingredient comprises levodopa and carbidopa, the mass ratio of levodopa to carbidopa is preferably from 1:1 to 4:1;
preferably, in the drug-containing immediate release overcoat, the mass of levodopa and carbidopa are 12.5 mg and 12.5 mg, 18.75 mg and 10.8 mg, 25 mg and 25 mg, 37.5 mg and 37.5 mg, 50 mg and 50 mg, 62.5 mg and 62.5 mg, 75 mg and 75 mg, 87.5 mg and 87.5 mg, 100 mg and 100 mg, 125 mg and 125 mg, 150 mg and 150 mg, 200 mg and 200 mg, 25 mg and 12.5 mg, 50 mg and 25 mg, 75 mg and 37.5 mg, 100 mg and 50 mg, 150 mg and 75 mg, 200 mg and 100 mg, 50 mg and 12.5 mg, 100 mg and 25 mg, 150 mg and 37.5 mg, or 200 mg and 50 mg, respectively.

14. A use of the osmotic pump tablet according to any one of claims 1-13 in the manufacture of a medicament for the treatment of fluctuations in motor symptoms in patients with advanced Parkinson's disease.

15. A preparation method for the osmotic pump tablet according to any one of claims 1-13, wherein in Schemes (1), (2), and (3), the preparation method for the osmotic pump tablet independently comprises the following steps:
S1, preparing drug-containing layer granules, comprising the following steps: mixing active pharmaceutical ingredient(s) and excipients and granulating to obtain drug-containing layer granules;
S2, preparing push layer granules, comprising the following steps: granulating at least one excipient in the push layer to obtain push layer granules;
S3, preparing a bi-layer tablet core: pressing drug-containing layer granules and push layer granules obtained from steps S1 and S2 to obtain a bi-layer tablet core;
S4, preparing an osmotic pump tablet: wrapping the tablet core with a coating membrane and perforating a drug-release orifice, thereby obtaining the osmotic pump tablet;
S5, optionally, after step S4 is completed, further comprising a step of wrapping a drug-containing immediate release overcoat around the osmotic pump tablet;
optionally, after step S3 and before step S4, a step of preparing an isolation layer is further comprised; the step of preparing the isolation layer comprises pressing the isolation layer material and the bi-layer tablet core;
step S5 is mandatory in Scheme (3).

16. The preparation method for the osmotic pump tablet according to claim 15, wherein in Schemes (1), (2), and (3), the preparation method for the osmotic pump tablet independently satisfies one or more of the following conditions:
(1) in steps S1 and S2, the preparation method comprises the step of screening each component prior to the mixing; the screening is preferably carried out through a 40 mesh sieve;
(2) in steps S1 and S2, granulating is each independently dry granulating, wet granulating, or fluidized bed granulating; when wet granulating is used, fluidized bed drying is preferred after the granulation is completed;
(3) in steps S1 and S2, after the granulation is completed, a step of further granulating is preferably comprised; the further granulating is preferably passed through a 1.2 mm sieve;
(4) in step S3, the tablet pressing die used in the pressing is preferably a 7.0 mm round punch;
(5) the drug-release orifice is obtained by laser perforation or mechanical perforation.

17. A method for using the osmotic pump tablet according to any one of claims 1-13, wherein the osmotic pump tablet is placed in an oral retention device or an oral drug delivery device.
